(19) Europäisches Patentamt
European Patent Office
Office européen des brevets

(11) Veröffentlichungsnummer: **0 352 682 B1**

(12) # EUROPÄISCHE PATENTSCHRIFT

(45) Veröffentlichungstag der Patentschrift: **02.02.94**

(51) Int. Cl.⁵: **C09B 62/503**, C09B 62/022, C07C 317/00, C07C 323/00

(21) Anmeldenummer: **89113540.2**

(22) Anmeldetag: **22.07.89**

Die Akte enthält technische Angaben, die nach dem Eingang der Anmeldung eingereicht wurden und die nicht in dieser Patentschrift enthalten sind.

(54) **Neue Doppelankerreaktivfarbstoffe und Benzylverbindungen als deren Zwischenprodukte.**

(30) Priorität: **28.07.88 DE 3825656**

(43) Veröffentlichungstag der Anmeldung:
**31.01.90 Patentblatt 90/05**

(45) Bekanntmachung des Hinweises auf die Patenterteilung:
**02.02.94 Patentblatt 94/05**

(84) Benannte Vertragsstaaten:
**CH DE FR GB IT LI**

(56) Entgegenhaltungen:
**EP-A- 0 181 585**
**EP-A- 0 197 418**
**EP-A- 0 307 817**
**US-A- 3 531 459**

(73) Patentinhaber: **BASF Aktiengesellschaft**
**Carl-Bosch-Strasse 38**
**D-67063 Ludwigshafen(DE)**

(72) Erfinder: **Patsch, Manfred, Dr.**
**Fritz-Wendel-Strasse 4**
**D-6706 Wachenheim(DE)**
Erfinder: **Nahr, Uwe, Dr.**
**In den Pfützen 19**
**D-6701 Dannstadt-Schauernheim(DE)**
Erfinder: **Wirsing, Friedrich, Dr.**
**Vergissmeinnichtweg 4**
**D-6720 Speyer(DE)**
Erfinder: **Jessen, Joerg L., Dr.**
**Maulbeerstück 20**
**D-6720 Speyer(DE)**
Erfinder: **Pandl, Klaus, Dr.**
**Schumannstrasse 18**
**D-6700 Ludwigshafen(DE)**
Erfinder: **Marschner, Claus, Dr.**
**Franz-Boegler-Weg 3**
**D-6720 Speyer(DE)**
Erfinder: **Dust, Matthias, Dr.**
**Teichgasse 7**
**D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 352 682 B1

**Beschreibung**

Die vorliegende Erfindung betrifft Doppelankerreaktivfarbstoffe der Formel I

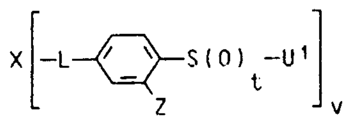 (I),

in der

$U^1$ $C_1$-$C_4$-Alkyl, Phenyl, $C_2$-$C_{10}$-Alkenyl oder den Rest

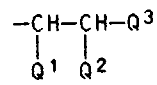,

wobei $Q^1$ und $Q^2$ gleich oder verschieden sind und unabhängig voneinander jeweils für Wasserstoff oder $C_1$-$C_4$-Alkyl und $Q^3$ für eine unter alkalischen Reaktionsbedingungen abspaltbare Gruppe stehen,

Z den Rest

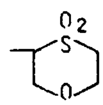

oder

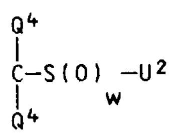,

wobei
$Q^4$ unabhängig voneinander für Wasserstoff oder $C_1$-$C_4$-Alkyl, das gegebenenfalls durch Cyano substituiert ist, $U^2$ für $C_1$-$C_4$-Alkyl, Phenyl, $C_2$-$C_{10}$-Alkenyl oder den Rest

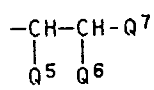,

in dem $Q^5$ und $Q^6$ gleich oder verschieden sind und unabhängig voneinander jeweils die Bedeutung von Wasserstoff oder $C_1$-$C_4$-Alkyl und $Q^7$ die Bedeutung einer unter alkalischen Reaktionsbedingungen abspaltbaren Gruppe besitzen und w für 0, 1 oder 2 stehen,

t 0, 1 oder 2,

v 0, 1 oder 2,

X a) den Rest eines Chromophors, der gegebenenfalls eine weitere reaktive Gruppe aufweist und der sich von einem Mono- oder Disazofarbstoff, einem Triphendioxazin, einem Anthrachinon, einem Kupfer-Formazan oder einem metallisierten Phthalocyanin ableitet, oder im Fall b) den Rest einer Kupplungskomponente, an den gegebenenfalls zusätzlich der Rest einer Diazokomponente über eine Azobrücke gebunden ist und der gegebenenfalls eine zusätzlich reaktive Gruppe aufweist, und

L im Fall a) ein Brückenglied der Formel

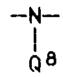

oder

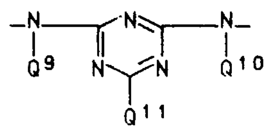 ,

wobei $Q^8$ für Wasserstoff oder $C_1$-$C_4$-Alkyl, $Q^9$ und $Q^{10}$ gleich oder verschieden sind und unabhängig voneinander jeweils für Wasserstoff oder $C_1$-$C_4$-Alkyl und $Q^{11}$ für Fluor, Chlor oder Brom stehen, oder im Fall b) eine Azobrücke bedeuten,

mit der Maßgabe, dass mindestens einer der beiden Reste S(O)t-$U^1$ und Z eine faserreaktive Gruppe darstellt.

Die vorliegende Erfindung betrifft weiterhin neue Benzylverbindungen, die als Zwischenprodukte dieser Farbstoffe dienen.

Aus der EP-A-48 355 und EP-A-65 732 sind Doppelankerreaktivfarbstoffe bekannt, die sich von Azofarbstoffen mit 1-Amino-8-hydroxynaphthalin-3,6- oder 4,6-disulfonsäure als Kupplungskomponente ableiten und die Ankerkomponenten auf Basis von Fluor-oder Chlortriazin und kernunsubstituiertem (Sulfonylmethyl)anilin aufweisen.

Gegenstand der älteren Patentanmeldung DE-A-3 731 202 sind Doppelankerreaktivfarbstoffe, die sich von gegebenenfalls metallisierten Azofarbstoffen ableiten und ein Doppelankersystem auf Basis eines Triazin/(Sulfonylmethyl)anilinderivats aufweisen.

Farbstoffe mit mehreren Reaktivresten vom Vinylsulfonyltyp oder auch mit einem Chlortriazinylreaktivrest im Kombination mit faserreaktiven Gruppen vom Vinylsulfonyltyp sind ferner beschrieben im EP-A-0 181 585 sowie EP-A-0 197 418 und US-A-4 812 558.

Aufgabe der vorliegenden Erfindung war es, neue Doppelankerreaktivfarbstoffe mit vorteilhaften anwendungstechnischen Eigenschaften bereitzustellen.

Demgemäß wurden nun die neuen Doppelankerreaktivfarbstoffe der obengenannten Formel I gefunden.

Alle in der obengenannten Formel I auftretenden Alkyl- und Alkenylreste können sowohl geradkettig als auch verzweigt sein.

Wenn in der obengenannten Formel I substituierte Phenylreste auftreten, kommen, sofern nicht anders vermerkt, als Substituenten z.B. $C_1$-$C_4$-Alkyl, $C_1$-$C_4$-Alkoxy oder Halogen in Betracht.

$Q^3$ und $Q^7$ in Formel I stehen jeweils für eine unter alkalischen Reaktionsbedingungen abspaltbare Gruppe. Solche Gruppen sind z.B. Chlor, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino,

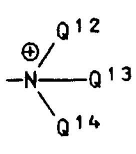 $An^{\ominus}$ , 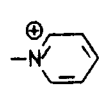 $An^{\ominus}$ , 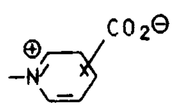 oder 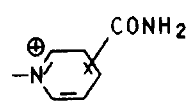 $An^{\ominus}$ ,

wobei $Q^{12}$, $Q^{13}$ und $Q^{14}$ gleich oder verschieden sind und unabhängig voneinander jeweils die Bedeutung von $C_1$-$C_4$-Alkyl oder Benzyl und $An^{\ominus}$ jeweils die Bedeutung eines Anions besitzen.

$U^1$, $U^2$, $Q^1$, $Q^2$, $Q^4$, $Q^5$, $Q^6$, $Q^8$, $Q^9$, $Q^{10}$, $Q^{12}$, $Q^{13}$ und $Q^{14}$ in Formel I stehen beispielsweise für Methyl, Ethyl, Propyl, Isopropyl, Butyl, Isobutyl oder sec-Butyl.

$Q^4$ steht weiterhin z.B. für 2-Cyanoethyl oder 2- oder 3-Cyanopropyl.

$U^1$ und $U^2$ in Formel I steht beispielsweise für 2-Chlorethyl, $C_2H_4OSO_3H$, $CH_2$-$CH(CH_3)OSO_3H$, $C_2H_4SSO_3H$, $C_2H_4OP(O)(OH)_2$, 2-(Methylsulfonyloxy)ethyl, 2-(Ethylsulfonyloxy)ethyl, 2-(Propylsulfonyloxy)-ethyl, 2-(Isopropylsulfonyloxyl)ethyl, 2-(Butylsulfonyloxyl)ethyl, 2-(Phenylsulfonyloxy)ethyl, dessen Phenylrest z.B. durch $C_1$-$C_4$-Alkyl, $C_1$-$C_4$-Alkoxy oder Halogen substituiert sein kann, 2-(Dimethylamino)ethyl, 2-(Diethylaminol)ethyl, 2-(Dipropylamino)ethyl, 2-(Diisopropylamino)ethyl, 2-(Dibutylamino)ethyl,2-(N-Methyl-N-ethylamino)ethyl, Vinyl, Prop-1-en-1-yl, Prop-1-en-3-yl oder But-2-en-2-yl.

$U^1$ und $U^2$ stehen weiterhin beispielsweise für 2-(Trimethylammonium)ethyl, 2-(Triethylammonium)ethyl, 2-(Methyl-diethylammonium)ethyl, 2-(Tripropylammonium)ethyl, 2-(Methyl-dipropylammonium)ethyl, 2-(Tributylammonium)ethyl, 2-(Benzyl-dimethylammonium)ethyl, 2-(Benzyl-diethylammonium)ethyl, 2-Pyridiniumethyl oder 2-(2-, 3- oder 4-Carboxylatopyridinium)ethyl, wobei (mit Ausnahme des 2-(2-, 3- oder 4-Carboxylatopyridinium)ethyl-Rests, der als Betain vorliegt) als Anion $An^{\ominus}$ z. B. Fluorid, Chlorid, Bromid, Iodid, Mono-, Di- oder Trichloracetat, Methylsulfonat, Phenylsulfonat oder 2-oder 4-Methylphenylsulfonat in Betracht kommt.

Der faserreaktive Doppelankerrest der Formel II

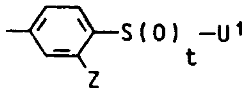

(II),

in der $U^1$, Z und t jeweils die obengenannte Bedeutung besitzen, wird im folgenden als "E" bezeichnet.

Bevorzugt sind Doppelankerreaktivfarbstoffe der Formel Ia,

$X[-L-E^1]_v$ (Ia)

in der X, L und v jeweils die obengenannte Bedeutung besitzen und $E^1$ für einen Rest der Formel IIa

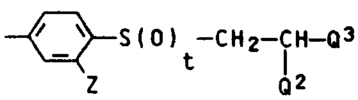

(IIa)

steht, in der

$Q^2$ Wasserstoff oder Methyl,

$Q^3$ $OSO_3H$,

Z den Rest

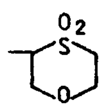

oder

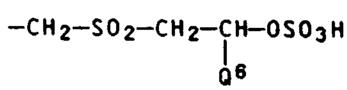

,

wobei $Q^6$ für Wasserstoff oder Methyl stehen, und

t 0 oder 2 bedeuten.

Besonders bevorzugt sind Doppelankerreaktivfarbstoffe der Formel Ib

$X[-L-E^2]_v$ (Ib),

in der X, L und v jeweils die obengenannte Bedeutung besitzen und $E^2$ für einen Rest der Formel IIb

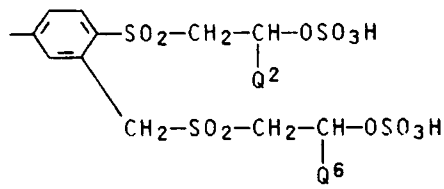

(IIb)

steht, in der $Q^2$ und $Q^6$ unabhängig voneinander Wasserstoff oder Methyl bedeuten.

Ganz besonders bevorzugt sind Doppelankerreaktivfarbstoffe der Formel Ic

$X[-L-E^3]_v$ (Ic)

in der X, L und v jeweils die obengenannte Bedeutung besitzen und $E^3$ für den Rest der Formel IIc

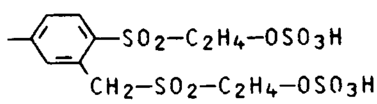

(IIc)

steht.

Neben dem Doppelankersystem E kann der Rest X noch weitere faserreaktive Reste tragen. Solche Reste leiten sich z.B. von Triazin-, Pyrimidin- oder Vinylsulfonylverbindungen ab.

X in Formel I stellt z.B. den Rest einer Kupplungskomponente dar, an den gegebenenfalls zusätzlich der Rest einer Diazokomponente über eine Azobrücke gebunden ist und der gegebenenfalls eine zusätzliche reaktive Gruppe aufweist. In diesem Fall ist der Doppelankerrest E über eine Azobrücke (-N = N-) an den Rest X geknüpft.

Farbstoffe dieser Klasse gehorchen der Formel IVa oder IVb

$(E-N=N-)_aK$ (IVa)

E-N = N-K-N = N-D (IVb),

wobei K den Rest einer Kupplungskomponente, D den Rest einer Diazokomponente und a 1 oder 2 bedeuten und E die obengenannte Bedeutung besitzt.

Wertvolle Farbstoffe dieser Klasse sind z.B. wasserlösliche Azofarbstoffe, insbesondere Monoazofarbstoffe der Formel IVa (a = 1) oder Disazofarbstoffe der Formel IVb, die Hydroxysulfonyl- und/oder Carboxylgruppen aufweisen.

Wichtige Kupplungskomponenten HK leiten sich z.B. von Verbindungen aus der Benzol-, Naphthalin-, Pyrazolon-, Pyridon- oder Hydroxypyrimidinreihe ab.

Wichtige Diazokomponenten $D-NH_2$ leiten sich z.B. von Verbindungen aus der Anilin- oder Aminonaphthalinreihe ab.

Besonders bevorzugt sind Farbstoffe der Formel V

$E^1-N=N-K^1$ (V),

in der $E^1$ die obengenannte Bedeutung besitzt und $K^1$ für den Rest einer Kupplungskomponente der Naphthalin-, Pyrazol-, Pyridon- oder Hydroxypyrimidinreihe, der gegebenenfalls eine weitere faserreaktive Gruppe aufweist, stehen.

Weiterhin besonders bevorzugt sind Farbstoffe der Formel VI

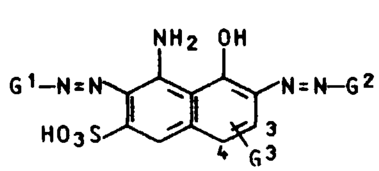

(VI),

in der einer der beiden Reste $G^1$ und $G^2$ für den Rest $E^1$, wobei dieser die obengenannte Bedeutung besitzt, und der andere für den Rest $D^1$, wobei dieser die Bedeutung eines Rest einer Diazokomponente der Anilin- oder Naphthalinreihe, der gegebenenfalls eine weitere faserreaktive Gruppe aufweist, besitzt, und $G^3$ für Hydroxysulfonyl in Ringposition 3 oder 4 stehen.

X in Formel I stellt weiterhin z.B. einen gegebenenfalls metallisierten Rest eines Azofarbstoffs dar. Geeignete Azofarbstoffreste sind an sich bekannt und in großer Zahl beschrieben, z. B. in K. Venkataraman "The Chemistry of Synthetic Dyes", Vol. VI, Academic Press, New York, London, 1972. Die Azofarbstoffe gehorchen der Formel VII

$$D\text{-}N = N\text{-}K(\text{-}N{=}N\text{-}D)_1 \qquad \text{(VII)},$$

in der D den Rest einer Diazokomponente, K den Rest einer Kupplungskomponente und 1 O oder 1 bedeuten.

Wertvolle Farbstoffe sind z.B. wasserlösliche Azofarbstoffe, insbesondere Monoazofarbstoffe, der Formel VII (1 = 0), die Hydroxysulfonyl- und/oder Carboxylgruppen aufweisen können.

Bevorzugt sind nichtmetallisierte Azofarbstoffe, insbesondere solche, die Sulfonsäure- und/oder Carboxylgruppen enthalten, wobei jene, die 1 bis 6 Sulfonsäuregruppen aufweisen, besonders hervorzuheben sind.

Wichtige Azofarbstoffe sind beispielsweise solche der Benzol-azo-naphthalin-, Benzol-azo-1-phenylpyrazol-5-on-, Benzol-azo-benzol-, Naphthalin-azo-benzol-, Benzol-azo-aminonaphthalin-, Naphthalin-azo-naphthalin-, Naphthalin-azo-1-phenylpyrazol-5-on-, Benzol-azo-pyridon, Benzol-azo-aminopyridin, Naphthalin-azo-pyridon-, Naphthalin-azo-aminopyridin oder Stilben-azo-benzolreihe.

Besonders bevorzugt sind Doppelankerreaktivfarbstoffe der Formel VIII

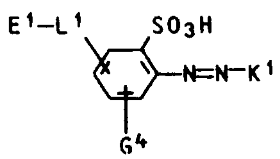

(VIII),

in der $E^1$ die obengenannte Bedeutung besitzt,

$L^1$ für den Rest

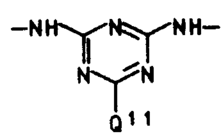

wobei $Q^{11}$ die obengenannte Bedeutung besitzt, $G^4$ für Wasserstoff, $C_1$-$C_4$-Alkyl, Chlor, oder Hydroxysulfonyl, und $K^1$ für den Rest einer Kupplungskomponente der Naphthalin-, Pyrazolon-, Pyridon- oder Hydroxypyrimidinreihe, der gegebenenfalls eine weitere faserreaktive Gruppe aufweist, stehen.

Weiterhin besonders bevorzugt sind Doppelankerreaktivfarbstoffe der Formel IX

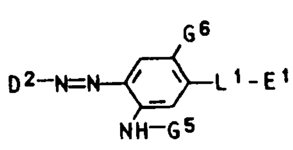

(IX),

in der $E^1$ und $L^1$ jeweils die obengenannte Bedeutung besitzen, $G^5$ für $C_1$-$C_4$-Alkanoyl, Carbamoyl, $C_1$-$C_4$-Mono- oder Dialkylcarbamoyl, Phenylcarbamoyl oder Cyclohexylcarbamoyl, $G^8$ für Wasserstoff, $C_1$-$C_4$-Alkyl, $C_1$-$C_4$-Alkoxy, Hydroxysulfonyl oder Chlor und $D^2$ für den Rest einer Diazokomponente der Anilin- oder Naphthalinreihe, die keine weitere faserreaktive Gruppe trägt, stehen.

Weiterhin besonders bevorzugt sind Doppelankerreaktivfarbstoffe der Formel X

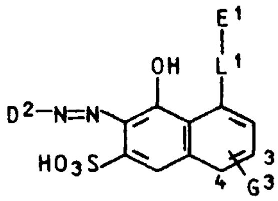

(X),

in der $D^2$, $E^1$ und $L^1$ jeweils die obengenannte Bedeutung besitzen und $G^3$ für Hydroxysulfonyl in Ringposition 3 oder 4 steht.

Weiterhin besonders bevorzugt sind Doppelankerreaktivfarbstoffe der Formel XI

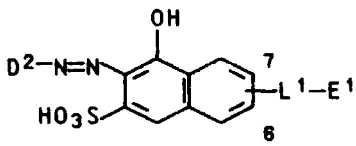

(XI),

in der $D^2$, $E^1$ und $L^1$ jeweils die obengenannte Bedeutung besitzen und wobei die Gruppe -L-$E^1$ in Ringposition 6 oder 7 steht.

Weiterhin sind wertvolle Verbindungen solche der Formel XII

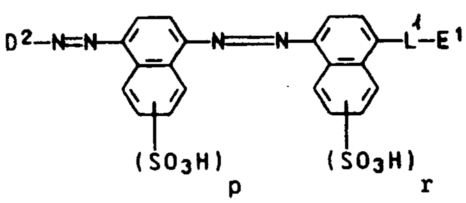

(XII),

in der $D^2$, $E^1$ und $L^1$ jeweils die obengenannte Bedeutung besitzen und p und r für 0,1 oder 2 stehen.

Weiterhin sind wertvolle Verbindungen solche der Formel XIII

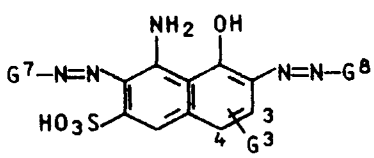

(XIII),

in der $G^3$ die obengenannte Bedeutung besitzt und einer der beiden Reste $G^7$ und $G^8$ für den Rest $D^2$, wobei dieser die obengenannte Bedeutung besitzt,
und der andere für den Rest

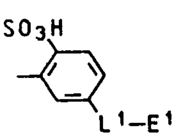

,

in dem $L^1$ und $E^1$ jeweils die obengenannte Bedeutung besitzen, stehen.

Solche aromatischen Reste D der Diazokomponenten der Anilin- und Aminonaphthalinreihe, die keine faserreaktiven Gruppen tragen, leiten sich beispielsweise von Aminen der Formel XIV a-f

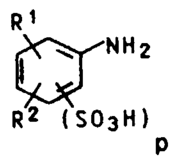
,
(XIVa)

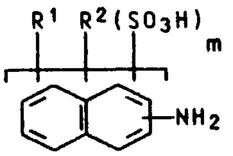
,
(XIVb)

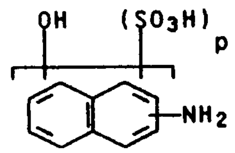
,
(XIVc)

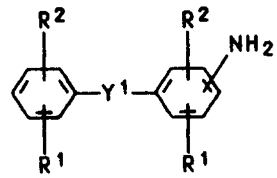
,
(XIVd)

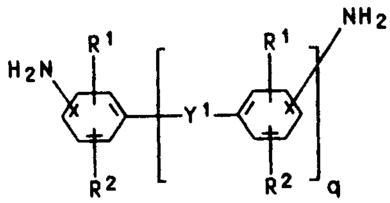
,
(XIVe)

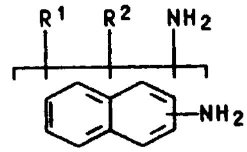
(XIVf)

ab, wobei

- m 0, 1, 2 oder 3,
- P 0, 1 oder 2
- q 0 oder 1,
- $R^1$ Wasserstoff, Methyl, Ethyl, Methoxy, Ethoxy, Acetyl, Cyano, Carboxyl, Hydroxysulfonyl, $C_1$-$C_4$-Alkoxycarbonyl, Hydroxy, Carbamoyl, $C_1$-$C_4$-Mono-oder Dialkylcarbamoyl, Fluor, Chlor, Brom oder Trifluormethyl,
- $R^2$ Wasserstoff, Methyl, Ethyl, Methoxy, Ethoxy, Cyano, Carboxyl, Hydroxysulfonyl, Acetylamino, $C_1$-$C_4$-Alkoxycarbonyl, Carbamoyl, $C_1$-$C_4$-Mono- oder Dialkylcarbamoyl, Fluor, Chlor, Nitro, Sulfamoyl, $C_1$-$C_4$-Mono- oder Dialkylsulfamoyl, $C_1$-$C_4$-Alkylsulfonyl, Phenylsulfonyl oder Phenoxy und
- $Y^1$ eine direkte Bindung, Sauerstoff, Schwefel oder die Gruppe -NHCO-, -CONH-, -CO-, $-NHSO_2-$, $-SO_2NH-$, $-SO_2-$, -CH = CH-, $-CH_2-CH_2-$, $-CH_2-$, -NH-, oder -N = N- bedeuten.

Bevorzugt sind solche Komponenten, in denen $R^1$ Wasserstoff, Methyl, Methoxy, Carboxyl, Hydroxysulfonyl, Hydroxy oder Chlor, $R^2$ Wasserstoff, Methyl, Methoxy, Carboxyl, Hydroxysulfonyl, Acetylamino oder Chlor und $Y^1$ die Gruppe -CO-, $-SO_2-$,-CH = CH-, $-CH_2-CH_2-$, $-CH_2-$oder -N = N- bedeuten.

Aromatische Amine die sich als Diazokomponenten eignen und die der Formel XIVa, XIVb, XIVc oder XIVd entsprechen, sind beispielsweise Anilin, 2-Methoxyanilin, 2-Methylanilin, 4-Chlor-2-aminoanisol, 4-Methylanilin, 4-Methoxyanilin, 2-Methoxy-5-methylanilin, 2,5-Dimethoxyanilin, 2,5-Dimethylanilin, 2,4-Dimethylanilin, 4-Butylanilin, 2,5-Diethoxyanilin, 2-Chloranilin, 3-Chloranilin, 4-Chloranilin, 2,5-Dichloranilin, 4-Chlor-2-nitroanilin, 4-Chlor-2-methylanilin, 3-Chlor-2-methylanilin, 4-Chlor-2-aminotoluol, 4-(p-Tolylsulfonyl)-anilin, 2-Ethoxy-1-naphthylamin, 1-Naphthylamin, 2-Naphthylamin, 4-Benzoylamino-2-ethoxyanilin, 4-Methylsulfonylanilin, 2,4-Dichloranilin-5-carbonsäure, 2-Aminobenzoesäure, 4-Aminobenzoesäure, 3-Aminobenzoesäure, 3-Chloranilin-6-carbonsäure, Anilin-2-oder -3- oder -4-sulfonsäure, Anilin-2,5-disulfonsäure, Anilin-2,4-disulfonsäure, Anilin-3,5-disulfonsäure, 2-Aminotoluol-4-sulfonsäure, 2-Aminoanisol-4-sulfonsäure, 2-Aminoanisol-5-sulfonsäure, 2-Ethoxyanilin-5-sulfonsäure, 2-Ethoxyanilin-4-sulfonsäure, 4-Hydroxysulfonyl-2-aminobenzoesäure, 2,5-Dimethoxyanilin-4-sulfonsäure, 2,4-Dimethoxyanilin-5-sulfonsäure, 2-Methoxy-5-methylanilin-4-sulfonsäure, 4-Aminoanisol-3-sulfonsäure, 4-Aminotoluol-3-sulfonsäure, 2-Aminotoluol-5-sulfonsäure, 2-Chloranilin-4-sulfonsäure, 2-Chloranilin-5-sulfonsäure, 2-Bromanilin-4-sulfonsäure, 2,6-Dichloranilin-4-sulfonsäure, 2,6-Dimethyl-anilin-3-, oder -4-sulfonsäure, 3-Acetylamino-6-sulfonsäure, 4-Acetyl-amino-2-hydroxysulfonyl-anilin, 1-Aminonaphthalin-4-sulfonsäure, 1-Aminonaphthalin-3-sulfonsäure, 1-Aminonaphthalin-5-sulfonsäure, 1-Aminonaphthalin-6-sulfonsäure, 1-Aminonaphthalin-7-sulfonsäure, 1-Aminonaphthalin-3,7-disulfonsäure, 1-Aminonaphthalin-3,6,8-trisulfon-säure, 1-Aminonaphthalin-4,6,8-trisulfonsäure, 2-Napththylamin-5-sulfonsäure oder -6- oder -8-sulfonsäure, 2-Aminonaphthalin-3,6,8-trisulfonsäure, 2-Aminonaphthalin-6,8-disulfonsäure, 2-Aminonaphthalin-1,6-disulfonsäure, 2-Aminonaphthalin-1-sulfonsäure, 2-Aminonaphthalin-1,5-disulfonsäure, 2-Aminonapthalin-3,6-disulfonsäure, 2-Aminonaphthalin-4,8-disulfonsäure, 2-Aminophenol-4-sulfonsäure, 2-Aminophenol-5-sulfonsäure, 3-Aminophenol-6-sulfonsäure, 1-Hydroxy-2-aminonaphthalin-5,8-oder -4,6-disulfonsäure, 4-Aminodiphenylamin, 4-Amino-4′-methoxydiphenylamin, 4-Amino-4′-methoxy-diphenylamin-3-sulfonsäure, 4-(2′-Methylphenylazo)-2-methylanilin, 4-Aminoazobenzol, 4′-Nitrophenylazo-1-aminonaphthalin, 4-(6′-Hydroxy-sulfonylnaphthylazo)-1-aminonaphthalin, 4-(2′,5′-Dihydroxy-sulfonylphenylazo)-1-aminonaphthalin, 4′-Amino-3′-methyl-3-nitrobenzophenon, 4-Aminobenzophenon, 4-(4′-Aminophenylazo)benzolsulfon-säure, 4-(4′-Amino-3′-methoxyphenylazo)benzolsulfonsäure oder 2-Ethoxy-1-naphthylamin-6-sulfonsäure.

Aromatische Diamine, die sich als Tetrazokomponenten eignen und die der Formel XIVe oder XIVf entsprechen, sind beispielsweise 1,3-Diaminobenzol, 1,3-Diaminobenzol-4-sulfonsäure, 1,4-Diaminobenzol, 1,4-Diaminobenzol-2-sulfonsäure, 1,4-Diamino-2-methylbenzol, 1,4-Diamino-2-methoxybenzol, 1,3-Diamino-4-methylbenzol, 1,3-Diaminobenzol-5-sulfon-säure, 1,3-Diamino-5-methylbenzol, 1,6-Diaminonaphthalin-4-sulfonsäure, 2,6-Diaminonaphthalin-4,8-disulfonsäure, 3,3′-Diaminodiphenylsulfon, 4,4′-Diaminodiphenylsulfon, 4,4′-Diaminostilben-2,2′-disulfonsäure, 2,7′Diaminodiphenylsulfon, 2,7′-Diamino-diphenylsulfon-4,5-disulfonsäure, 4,4′-Diaminobenzophenon, 4,4′-Diamino-3,3′-dinitrobenzophenon, 3,3′-Diamino-4,4′-dichlorbenzophenon, 4,4′- oder 3,3′-Diaminodiphenyl, 4,4′-Diamino-3,3′-dichlordiphenyl, 4,4′-Diamino-3,3′-dimethoxy- oder -3,3′-dimethyl- oder -2,2′-dimethyl- oder -2,2′-dichlor- oder -3,3′-diethoxydiphenyl, 4,4′-Diamino-3,3′-dimethyl-6,6′-dinitrodiphenyl, 4,4′-Diaminodiphenyl-2,2′- oder -3,3′-disulfonsäure, 4,4′-Diamino-3,3′-dimethyl- oder -3,3′-dimethoxy oder -2,2′-dimethoxydiphenyl-6,6′-disulfonsäure, 4,4′-Diamino-2,2′, 5,5′-tetrachlordiphenyl, 4,4′-Diamino-3,3′-dinitrodiphenyl, 4,4′-Diamino-2,2′-dichlor-5,5′-dimethoxydiphenyl, 4,4′-Diaminodiphenyl-2,2′- oder -3,3′-dicarbonsäure, 4,4′-Diamino-3,3′-dimethyldiphenyl-5,5′-disulfonsäure, 4,4′-Diamino-2-nitrodiphenyl, 4,4′-Diamino-3-ethoxy-oder -3-hydroxysulfondiphenyl, 4,4′-Diamino-3,3′-dimethyldiphenyl-5-sulfonsäure, 4-4′-Diaminodiphenylmethan, 4,4′-Diamino-3,3′-dimethyldiphenylmethan, 4,4′-Diamino-2,2′, 3,3′-tetramethyldiphenylmethan, 4,4′-Diaminodi-phenylethan, 4,4′-Diaminostilben oder 4,4′-Diaminodiphenylmethan-3,3′-dicarbonsäure.

Aromatische Reste D der Diazokomponeten der Anilin- oder Aminonaphthalinreihe, die einen faserreaktiven Rest E tragen können, leiten sich beispielsweise von Aminen der Formel XVa-c

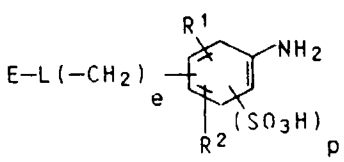
(XVa)
,
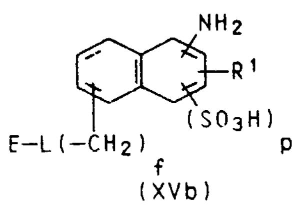
(XVb)
,
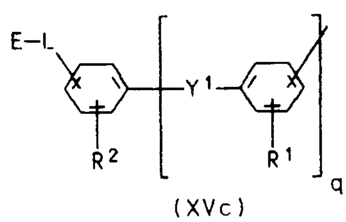
(XVc)

ab, in denen L, $R^1$, $R^2$, p, q, $Y^1$ und E jeweils die obengenannte Bedeutung besitzen und e und f gleich oder verschieden sind und abhängig voneinander jeweils 0 oder 1 bedeuten.

Aromatische Amine, die der Formel XVa, XVb oder XVc entsprechen, sind beispielsweise 1,3-Diaminobenzol, 1,3-Diaminobenzol-4-sulfonsäure, 1,3-Diaminobenzol-4,6-disulfonsäure, 1,4-Diaminobenzol, 1,4-Diaminobenzol-2-sulfonsäure, 1,4-Diaminobenzol-2,5-disulfonsäure, 1,4-Diamino-2-methylbenzol , 1,4-Diamino-2-methoxybenzol, 1,3-Diamino-4-methylbenzol, 1,4-Diaminobenzol-2,6-disulfonsäure, 1,5-Diamino-4-methylbenzol-2-sulfonsäure, 1,5-Diamino-4-methoxybenzol-2-sulfonsäure, 1,6-Diaminonapht-2-ol-4-sulfonsäure, 1,6-Diaminonaphthalin-4-sulfonsäure, 2,6-diaminonaphthalin-4,8-disulfonsäure, 2,6-Diaminonapht-1-ol-4,8-disulfonsäure, 1,3-Diaminobenzol-5-sulfonsäure, 1,3-Diamino-5-methylbenzol, 2,6-Diaminophenol-4-sulfonsäure, 5-(Aminomethyl)-2-aminonaphthalin-1-sulfonsäure, 5-(N-Methylaminomethyl)-2-aminonaphthalin-1-sulfonsäure, 4,4′-Diaminostilben-3,3-dicarbonsäure, 4-(N-Methylaminomethyl)anilin-2-sulfonsäure oder 3-(N-Methylaminomethyl)anilin-6-sulfonsäure.

Die Reste K der Kupplungskomponente entstammen vorzugsweise der Anilin-, Naphthalin-, Pyrazol-, Pyridin, Pyrimidin-, Indol- oder Acylacetarylidreihe und können auch faserreaktive Gruppen tragen.

Faserreaktivegruppenfreie Kupplungskomponenten der Anilin- und Naphthalinreihe entsprechen beispielsweise den Verbindungen der Formel XVIa-g

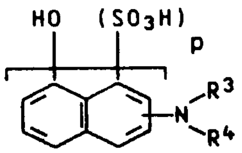 , 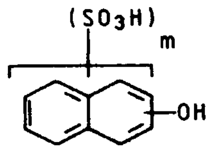 , 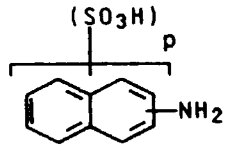 , 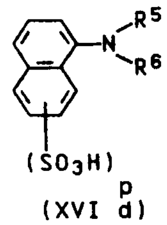 ,

(XVI a) (XVI b) (XVI c) (XVI d)

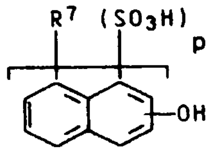 , 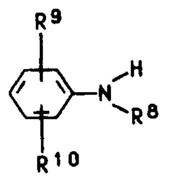 , 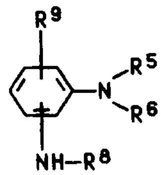 ,

(XVI e) (XVI f) (XVI g)

wobei

- $R^3$ Wasserstoff oder $C_1$-$C_4$-Alkyl,
- $R^4$ Wasserstoff, $C_1$-$C_4$-Alkyl oder Phenyl, das durch $C_1$-$C_4$-Alkyl, $C_1$-$C_4$-Alkoxy, Chlor, Brom oder Hydroxysulfonyl ein- oder zweifach substituiert sein kann,
- $R^5$ Wasserstoff oder $C_1$-$C_4$-Alkyl, das durch Hydroxy, Cyano, Carboxyl, Hydroxysulfonyl, Hydroxysulfonyloxy, Methoxycarbonyl, Ethoxycarbonyl oder Acetoxy substituiert sein kann,
- $R^6$ Wasserstoff, $C_1$-$C_4$-Alkyl, das durch Hydroxy, Cyano, Carboxyl, Hydroxysulfonyl, Hydroxysulfonyloxy, Methoxycarbonyl, Ethoxycarbonyl oder Acetoxy substituiert sein kann, Benzyl oder Phenyl, das durch $C_1$-$C_4$-Alkyl, $C_1$-$C_4$-Alkoxy, Chlor oder Hydroxysulfonyl substituiert sein kann,
- $R^7$ $C_1$-$C_6$-Alkylureido, Phenylureido, das durch Chlor, Methyl, Methoxy, Nitro, Hydroxysulfonyl oder Carboxyl substituiert sein kann, $C_1$-$C_6$-Alkanoylamino, Cyclohexanoylamino, Benzoylamino, das durch Chlor, Methyl, Methoxy, Nitro, Hydroxysulfonyl oder Carboxyl substituiert sein kann, oder Hydroxy,
- $R^8$ Wasserstoff, $C_1$-$C_6$-Alkyl, insbesondere $C_1$-$C_4$-Alkyl, das jeweils durch Phenyl, $C_1$-$C_4$-Alkoxy, Hydroxy, Phenoxy oder $C_1$-$C_4$-Alkanoyloxy substituiert sein kann, $C_5$-$C_7$-Cycloalkyl, Hydroxysulfonylphenyl, $C_1$-$C_4$-Alkanoyl, Carbamoyl, $C_1$-$C_4$-Mono- oder Dialkylcarbamoyl, Phenylcarbamoyl oder Cyclohexylcarbamoyl,
- $R^9$ Methoxy, Ethoxy, Chlor, Brom, Acetylamino, Amino, Ureido, Methylsulfonylamino, Ethylsulfonylamino, Dimethylaminosulfonylamino, Methylamino, Ethylamino, Dimethylamino oder Diethylamino und
- $R^{10}$ Wasserstoff, Methyl, Ethyl, Methoxy, Ethoxy, Chlor oder Brom bedeuten und
- p und m jeweils die obengenannte Bedeutung besitzen.

Im einzelnen sind beispielsweise Anilin-N-methansulfonat, o- oder m-Toluidin, o- oder m-Anisidin, Kresidin, 2,5-Dimethylanilin, 2,5-Dimethoxyanilin, m-Aminoacetanilid, 3-Amino-4-methoxyacetanilid, 3-Amino-4-methylacetanilid, m-Aminophenylharnstoff, N-Methylanilin, N-Methyl-m-toluidin, N-Ethylanilin, N-Ethyl-m-toluidin, N-(2-Hydroxyethyl)anilin oder N-(2-Hydroxyethyl)-m-toluidin zu nennen.

Naphtholsulfonsäuren sind beispielsweise 1-Naphthol-3-sulfonsäure, 1-Naphthol-4-sulfonsäure, 1-Naphthol-5-sulfonsäure, 1-Naphthol-8-sulfonsäure, 1-Naphthol-3,6-disulfonsäure, 1-Naphthol-3,8-disulfonsäure, 2-Naphthol-5-sulfonsäure, 2-Naphthol-6-sulfonsäure, 2-Naphthol-7-sulfonsäure, 2-Naphthol-8-sulfonsäure, 2-Naphthol-3,6-disulfonsäure, 2-Naphthol-6,8-disulfonsäure, 2-Naphthol-3,6,8-trisulfonsäure, 1,8-Dihydroxynaphthalin-3,6-disulfonsäure, 2,6-Dihydroxynapthalin-8-sulfonsäure oder 2,8-Dihydroxynaphthalin-6-sulfonsäure.

Weiterhin sind beispielsweise 1-Naphthylamin, N-Phenyl-1-napthylamin, N-Ethyl-1-naphthylamin, N-Phenyl-2-naphthylamin, 1,5-Naphthylendiamin, 1,8-Naphthylendiamin, 1-Naphthol, 2-Naphthol, 1,5-Dihydroxynaphthalin, 1,6-Dihydroxynaphthalin, 1,7-Dihydroxynaphthalin, 2,7-Dihydroxynaphthalin, 2-Hydroxynaphthalin-3-carbonsäure-N-phenylamid, 2-Hydroxynaphthalin-3-carbonsäure-N-(2′-methoxyphenyl)amid oder 2-Hydroxynaphthalin-3-carbonsäure-N-(2′-5′-dimethoxyphenyl)amid zu nennen.

Aminonaphthalinsulfonsäuren sind beispielsweise 1-Naphthylamin-6-sulfonsäure, 1-Naphthylamin-7-sulfonsäure, 1-Naphthylamin-8-sulfonsäure, 2-Naphthylamin-3,6-disulfonsäure, 2-Naphthylamin-5,7-disulfonsäure oder 2-Naphthylamin-6,8-disulfonsäure.

Als Aminonaphtholsulfonsäuren sind z. B. 1-Amino-5-hydroxynaphthalin-7-sulfonsäure, 1-Amino-8-hydroxynaphthalin-4-sulfonsäure, 1-Amino-8-hydroxynaphthalin-2,4-disulfonsäure, 1-Amino-8-hydroxynaphthalin-3,6-disulfonsäure, 1-Amino-8-hydroxynaphthalin-4,6-disulfonsäure, 2-Amino-5-hydroxynaphthalin-7-sulfonsäure, 2-Amino-8-hydroxynaphthalin-6-sulfonsäure, 2-Amino-8-hydroxynaphthalin-3,6-disulfonsäure, 2-Amino-5-hydroxynaphthalin-1,7-disulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure, 1-Benzoylamino-8-hydroxynaphthalin-4,6-disulfonsäure, 1-Acetylamino-5-hydroxynaphthalin-7-sulfonsäure, 2-Methylamino-8-hydroxynaphthalin-6-sulfonsäure, 2-Methylamino-8-hydroxynaphthalin-6-sulfonsäure oder 2-(3'-oder 4'-Hydroxysulfonylphenyl)amino-8-hydroxynaphthalin-6-sulfonsäure zu nennen.

Von besonderer Bedeutung sind Kupplungskomponenten, die Sulfonsäuren- und/oder Carboxylgruppen aufweisen, und die in ortho- oder para-Stellung zu einer Hydroxy- und/oder Aminogruppe kuppeln.

Als Biespiele für solche Kupplungskomponenten seien 2-Acetylamino-5-hydroxynaphthalin-7-sulfonsäure, 2-Acetylamino-8-hydroxynaphthalin-6-sulfonsäure, 1-Acetylamino-8-hydroxynaphthalin-3,6-disulfonsäure, 1-Benzoylamino-8-hydroxynaphthalin-3,6-disulfonsäure, 1-Acetylamino-8-hydroxynaphthalin-4,6-disulfonsäure oder 1-Benzoylamino-8-hydroxynaphthalin-4,6-disulfonsäure genannt.

Kupplungskomponenten der weiteren Reihen sind beispielsweise: Pyrazolone Aminopyrazole, 2,6-Diaminopyridine, Pyridone, Hydroxy- oder Aminopyrimidine, Indole oder Acetoacetarylide.

Faserreaktivgruppenfreie Kupplungskomponenten dieser Reihe entsprechen dabei beispielsweise der Formel XVIIa-f.

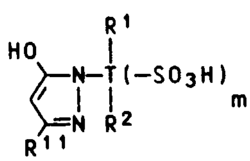
,
(XVII a)

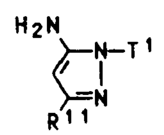
,
(XVII b)

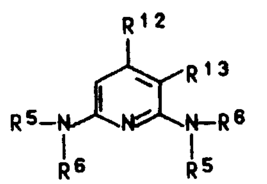
,
(XVII c)

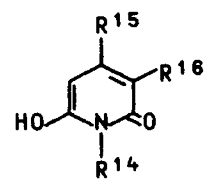
,
(XVII d)

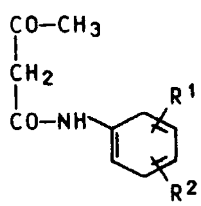
,
(XVII e)

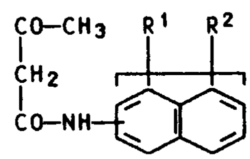
,
(XVII f)

wobei

- T für einen Benzol- oder Naphthalinkern,
- $T^1$ für $C_1$-$C_4$-Alkyl, Cyclohexyl, Benzyl oder Phenyl, das ein- oder mehrfach durch Fluor, Chlor, Brom, Methyl, Methoxy, Nitro, Hydroxysulfonyl, Carboxyl, Acetyl, Acetylamino, Methylsulfonyl, Sulfamoyl oder Carbamoyl substituiert ist,
- $R^{11}$ für Methyl, Carboxyl, Methoxycarbonyl, Ethoxycarbonyl oder Phenyl,
- $R^{12}$ für Wasserstoff oder $C_1$-$C_4$-Alkyl, das durch Methoxy, Ethoxy oder Cyano substituiert sein kann,
- $R^{13}$ für Wasserstoff, Methyl, Hydroxysulfonylmethyl, Hydroxysulfonyl, Cyano oder Carbamoyl,
- $R^{14}$ für Wasserstoff, $C_1$-$C_4$-Alkyl, das durch Phenyl, Hydroxysulfonylphenyl, Hydroxy, Amino, Methoxy, Ethoxy, Carboxyl, Hydroxysulfonyl, Acetylamino, Benzoylamino oder Cyano substituiert sein kann, Cyclohexyl, Phenyl, das gegebenenfalls durch Carboxyl, Hydroxysulfonyl, Benzoylamino, Acetylamino, Methyl, Methoxy, Cyano oder Chlor substituiert ist, oder Amino, das durch Phenyl, $C_1$-$C_4$-Alkyl, Acetyl oder Benzoyl substituiert ist,
- $R^{15}$ für $C_1$-$C_4$-Alkyl, Phenyl, Hydroxy, Cyano, Acetyl, Benzoyl, Carboxyl, Methoxycarbonyl, Carbamoyl oder Hydroxysulfonylmethyl und
- $R^{16}$ für Wasserstoff, Chlor, Brom, Acetylamino, Amino, Nitro, Hydroxysulfonyl, Sulfamoyl, Methylsulfonyl, Phenylsulfonyl, Carboxyl, Methoxycarbonyl, Acetyl, Benzoyl, Carbamoyl, Cyano oder

Hydroxysulfonylmethyl stehen und $R^1$, $R^2$, $R^5$, $R^6$ und m jeweils die obengenannte Bedeutung besitzen.

Als Pyrazolon-Kupplungskomponenten sind beispielsweise 3-Methyl-, 3-Carboxy- oder 3-($C_1$-$C_4$-Alkoxycarbonyl)pyrazol-5-one zu nennen, die in 1-Stellung Wasserstoff, gegebenenfalls durch Methyl, Ethyl, Fluor, Chlor, Brom, Trifluormethyl, Methoxy, Ethoxy, Cyano, Phenoxy, Phenylsulfonyl, Methylsulfonyl, Hydroxysulfonyl, Benzoyl, Acetyl, Acetylamino, Nitro, Hydroxyl, Carboxyl, Carbamoyl oder Sulfamoyl substituiertes Phenyl oder durch Hydroxysulfonyl substituiertes 1-, oder 2-Naphthyl tragen können. Beispielsweise sind 1-Phenyl-, 1-(2′-Chlorphenyl)-, 1-(2′-Methoxyphenyl)-, 1-(2′Methylphenyl)-, 1-(1′,5′-Dichlorphenyl)-, 1-(2′,6′-Dichlorphenyl)-, 1-(2′-Methyl-6′-chlorphenyl)-, 1-(2′-Methoxy-5′-methylphenyl)-, 1-(2′-Chlor-5′-hydroxysulfonylphenyl)-, 1-(2′-Methoxy-5′-hydroxysulfonylphenyl)-, 1-(2,5′-Dichlor-4′-hydroxysulfonylphenyl)-, 1-(2′,5′-Dihydroxysulfonylphenyl)-, 1-(2′-Carboxyphenyl)-, 1-(3′-Hydroxysulfonylphenyl)-, 1-(4′-Hydroxysulfonylphenyl)- oder 1-(3′-Sulfamoylphenyl)-3-carboxylpyrazol-5-on, 1-(3′- oder 4′-Hydroxysulfonylphenyl)-, 1-(2′-Chlorphenyl)-, 1-(2′-Chlor-4′- oder -5′-hydroxysulfonylphenyl)-, 1-(2′-Methyl-4′-hydroxysulfonylphenyl)-,1-(2′,5′-Dichlorphenyl)-, 1-(4′,8′-Dihydroxysulfonyl-8-naphthyl)-, 1-(6′-Hydroxysulfonyl-1-naphthyl)-3-methylpyrazol-5-on, 1-Phenylpyrazol-5-on-3-carbonsäureethylester, Pyrazol-5-on-3-carbonsäureethylester oder Pyrazol-5-on-3-carbonsäure zu nennen.

Andere aus der Pyrazolreihe stammende Kupplungskomponenten sind beispielsweise 1-Methyl-, 1-Ethyl-, 1-Propyl-, 1-Butyl-, 1-Cyclohexyl-, 1-Benzyl- oder 1-Phenyl-5-aminopyrazol, 1-(4′-Chlorphenyl)-, 1-(4′-Methylphenyl)-5-aminopyrazol oder 1-Phenyl-3-methyl-5-aminopyrazol.

Acetoacetanilide sind vor allem Acetessiganilid und dessen im Phenylkern durch Chlor, Methyl, Ethyl, Methoxy, Ethoxy, Acetylamino, Hydroxysulfonyl, Carboxyl, Carbamoyl oder Sulfamoyl ein- oder mehrfach substituierte Derivate.

Vom Pyridin abgeleitete Kupplungskomponenten sind beispielsweise die in der DE-A-2 260 827 beschriebenen Derivate.

Als Pyrimidinkupplungskomponenten sind z. B. die in der DE-A-2 202 820, DE-A-2 308 663 oder DE-A-3 119 349 aufgeführten Verbindungen geeignet. Weiterhin sind Barbitursäure und deren N-Substitutionsprodukte zu nennen. Als N-Substituenten kommen dabei insbesondere $C_1$-$C_4$-Alkyl oder gegebenenfalls substituiertes Phenyl in Betracht.

Als Indolkupplungskomponenten sind beispielsweise 2-Methylindol, 2-Phenylindol, 2-Phenylindol-5-sulfonsäure, 1-Methyl-2-phenylindol, 1-(2′-Hydroxyethyl)-, 1-(2′-Carboxyethyl)-, 1-(2′Carbamoylethyl)-2-methylindol oder -2-phenylindol zu nennen.

Als Pyridonkupplungskomponenten sind beispielsweise 1-Ethyl-2-hydroxy-4-methyl-5-carbamoylpyrid-6-on, 1-(2′Hydroxyethyl)-2-hydroxy-4-methyl-5-carbamoylpyrid-6-on, 1-Phenyl-2-hydroxy-4-methyl-5-carbamoylpyrid-6-on, 1-Ethyl-2-hydroxy-4-methyl-5-cyanopyrid-6-on, 1-Ethyl-2-hydroxy-4-hydroxysulfonylmethyl-5-carbamoylpyrid-6-on, 1-Ethyl-2-hydroxy-4-methyl-5-hydroxysulfonylmethylpyrid-6-on, 1-Methyl-2-hydroxy-4-methyl-5-cyanopyrid-6-on, 1-Methyl-2-hydroxy-5-acetylpyrid-6-on, 1,4-Dimethyl-2-hydroxy-5-cyanopyrid-6-on, 1,4-Dimethyl-5-carbamoylpyrid-6-on, 2,6-Dihydroxy-4-ethyl-5-cyanopyridin, 2,6-Dihydroxy-4-ethyl-5-carbamoylpyridin, 1-Ethyl-2-hydroxy-4-methyl-5-hydroxysulfonylmethylpyrid-6-on, 1-Methyl-2-hydroxy-4-methyl-5-methylsulfonylpyrid-6-on oder 1-Carboxymethyl-2-hydroxy-4-ethyl-5-phenylsulfonylpyrid-6-on zu nennen.

Faserreaktivgruppenhaltige Kupplungskomponenten K der Anilin sind Naphthalinreihe sind beispielsweise Verbindungen der Formel XVIII a - e.

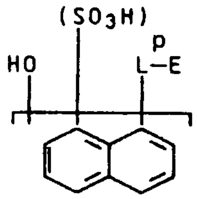
,
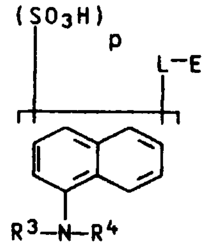
,
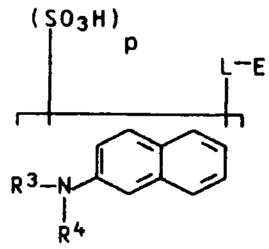
,
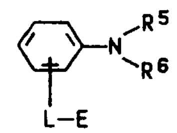
,

(XVIII a) (XVIII b) (XVIII c) (XVIII d)

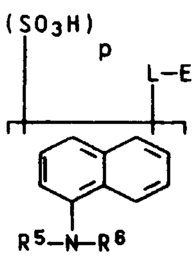
,

(XVIII e)

wobei L, $R^3$, $R^4$, $R^5$, $R^6$, E und p jeweils die obengenannte Bedeutung besitzen.

Faserreaktivgruppenhaltige Kupplungskomponenten der Pyrazolon-, Aminopyrazol-, 2,6-Diaminopyridin-, Pyridon-, Hydroxy- oder Aminopyrimidin-, Indol-, oder Acetoacetarylidreihe entsprechen beispielsweise der Formel XIX a - f.

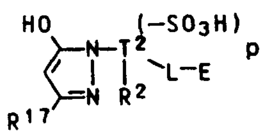
,
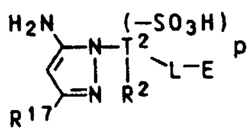
,
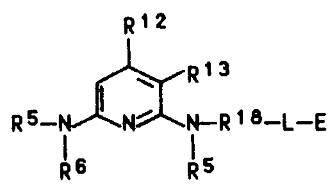
,

(XIX a) (XIX b) (XIX c)

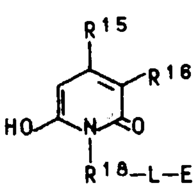
,
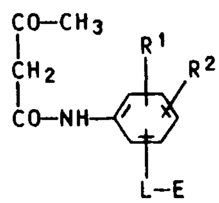
,
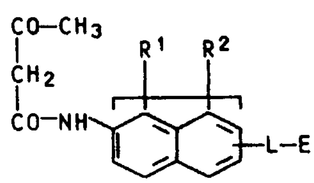
,

(XIX d) (XIX e) (XIX f)

wobei

- $T^2$ für einen Benzol- oder Naphthalinkern
- $R^{17}$ für Methyl, Carboxyl, Methoxycarbonyl, Ethoxycarbonyl oder Phenyl,
- $R^{18}$ für $C_1$-$C_4$-Alkyl, Benzyl, Phenylethyl oder Phenyl, wobei die Phenylkerne jeweils noch durch Fluor, Chlor, Brom, Methyl, Methoxy, Cyano, Hydroxysulfonyl, Carboxyl, Acetyl, Nitro, Carbamoyl oder Sulfamoyl substituiert sein können, stehen und
- L $R^1$, $R^2$, $R^5$, $R^6$, $R^{12}$, $R^{13}$, $R^{15}$, $R^{16}$, p und E jeweils die obengenannte Bedeutung besitzen.

Faserreaktive Reste E tragende Pyrazolonkupplungskomponenten leiten sich beispielsweise von folgenden Pyrazolonen ab: 1-(3'- oder 4'-Aminophenyl)-, 1-(2'-Hydroxysulfonyl-5'-aminophenyl)-, 1-(2'-Methoxy-5'-aminophenyl)-3-carboxylpyrazol-5-on, 1-(3'- oder 4'-Aminophenyl)-, 1-(3'- oder 4'-Nitrophenyl)-3-methylpyrazol-5-on, 1-(3′- oder 4′-Nitrophenyl)-, 1-(6′-Nitro-4′, 8′-dihydroxysulfonylnaphth-2′-yl)-oder 1-(6′-Amino-4′,8′-dihydroxysulfonylnaphth-2′-yl)-3-carboxylpyrazol-5-on.

Anstelle der Azofarbstoffreste können die Farbstoffe der Formel I auch entsprechende Metallkomplexfarbstoffreste enthalten. Als zu komplexierende Metalle kommen dabei insbesondere Kupfer, Kobalt, Chrom, Nickel oder Eisen in Betracht, wobei Kupfer, Kobalt oder Chrom bevorzugt sind.

Dabei befinden sich die metallisierten Gruppen vorzugsweise jeweils in ortho-Stellung zur Azogruppe, z. B. in Form von o,o′-Dihydroxy-, o-Hydroxy-o′-carboxy-, o-Carboxy-o′-amino oder o-Hydroxy-o′-amino-azogruppierungen.

X in Formel I stellt weiterhin z.B. den Rest eines Kupfer-Formazanfarbstoffs dar. Kupfer-Formazane sind an sich bekannt und beispielsweise in K. Venkataraman "The Chemistry of Synthetic Dyes", Vol. III, Academic Press, New York, London, 1970, beschrieben.

Besonders bevorzugt sind Kupfer-Formazanfarbstoffe der Formel XX

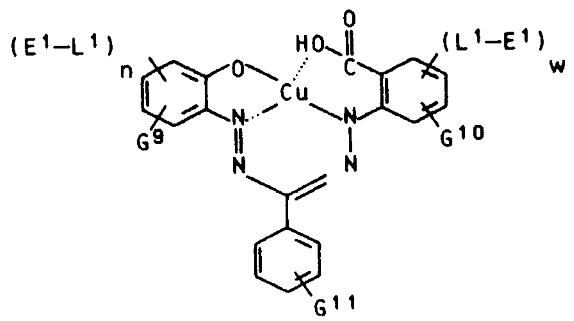

(XX),

in der

| | |
|---|---|
| $G^9$, $G^{10}$ und $G^{11}$ | gleich oder verschieden sind und unabhängig voneinander jeweils Wasserstoff oder Hydroxysulfonyl, |
| n | 0 oder 1 und |
| w | 0 oder 1 bedeuten und |
| $E^1$ und $L^1$ | jeweils die obengenannte Bedeutung besitzen, mit der Maßgabe, daß n und w nicht gleichzeitig 0 bedeuten. |

Eine Methode zur Herstellung der diesen Farbstoffen zugrundeliegenden Formazane ist beispielsweise in der älteren Patentanmeldung DE-A-3 737 536 beschrieben.

X in Formel I stellt weiterhin z.B. den Rest eines Anthrachinonfarbstoffs dar. Anthrachinone sind an sich bekannt und beispielsweise in K. Venkataraman "The Chemistry of Synthetic Dyes", Vol. II, Academic Press, New York, 1952, beschrieben.

Besonders bevorzugt sind Anthrachinonfarbstoffe der Formel XXI

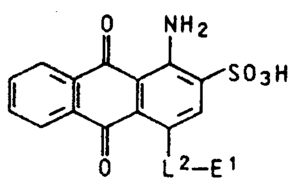

(XXI),

in der

$L^2$ für den Rest

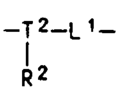

,

worin $L^1$, $R^2$ und $T^2$ jeweils die obengenannte Bedeutung besitzen, oder insbesondere für den Rest -NH- steht und $E^1$ die obengenannte Bedeutung besitzt.

X in Formel I stellt weiterhin z.B. den Rest eines Triphendioxazinfarbstoffs dar. Triphendioxazine sind in sich bekannt und beispielsweise in der EP-A-141 359 oder der älteren Patentanmeldung DE-A-3 735 057

beschrieben.

Besonders bevorzugt sind Triphendioxazinfarbstoffe der Formel XXII

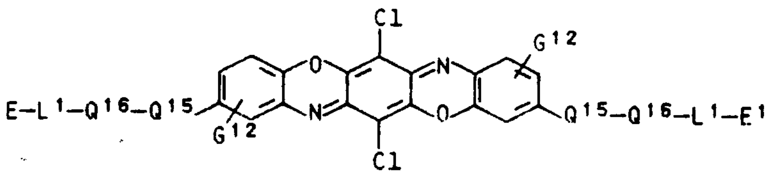

(XXII),

in der

- $E^1$ und $L^1$ jeweils die obengenannte Bedeutung besitzen und
- $G^{12}$ für Hydroxysulfonyl oder den Rest $SO_2$-$C_2H_4$-$OSO_3H$,
- $Q^{15}$ für Sauerstoff, Imino oder $C_1$-$C_4$-Alkylimino und
- $Q^{16}$ für geradkettige oder verzweigtes $C_2$-$C_4$-Alkylen oder Phenylen stehen.

X in der Formel I stellt weiterhin z.B. den Rest eines metallisierten Phthalocyaninfarbstoffs dar. Phthalocyanine sind an sich bekannt und beispielsweise in F.H. Moser, D.L. Thomas "The Phthalocyanines", Vol. II, CRC Press, Boca Raton, Florida, 1983, beschrieben.

Besonders bevorzugt sind Phthalocyaninfarbstoffe der Formel XXIII

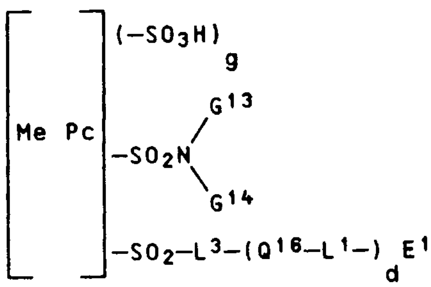

(XXIII),

in der

- $G^{13}$ und $G^{14}$ gleich oder verschieden sind und unabhängig voneinander jeweils Wasserstoff oder $C_1$-$C_4$-Alkyl,
- $L^3$ Imino oder $C_1$-$C_4$-Alkylimino,
- g 0, 1, 2 oder 3 und
- d 0 oder 1 bedeuten und $L^1$, E und $Q^{16}$ jeweils die obengenannte Bedeutung besitzen.

Die Erfindung betrifft weiterhin neue Benzylverbindungen der Formel III

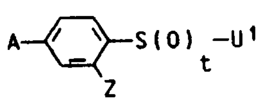

(III),

in der

- A Nitro oder Amino,
- $U^1$ $C_1$-$C_4$-Alkyl, Phenyl, $C_2$-$C_{10}$-Alkenyl oder den Rest

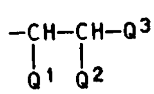

,

wobei $Q^1$ und $Q^2$ gleich oder verschieden sind und unabhängig voneinander jeweils für Wasserstoff oder $C_1$-$C_4$-Alkyl und $Q^3$ für Hydroxy oder eine unter alkalischen Reaktionsbedingungen abspaltbare Gruppe stehen,

- Z den Rest

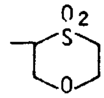

oder

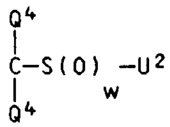,

wobei
$Q^4$ unabhängig voneinander für Wasserstoff oder $C_1$-$C_4$-Alkyl, das gegebenenfalls durch Cyano substituiert ist, $U^2$ für $C_1$-$C_4$-Alkyl, Phenyl, $C_2$-$C_{10}$-Alkenyl oder den Rest

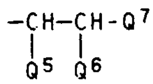,

in dem $Q^5$ und $Q^6$ gleich oder verschieden sind und unabhängig voneinander jeweils die Bedeutung von Wasserstoff oder $C_1$-$C_4$-Alkyl und $Q^7$ die Bedeutung von Hydroxy oder einer unter alkalischen Reaktionsbedingungen abspaltbaren Gruppe besitzen und w für 0, 1 oder 2 stehen, und t 0, 1 oder 2 bedeuten, mit der Maßgabe, dass mindestens einer der beiden Reste $S(O)_t$-$U^1$ und Z eine faserreaktive Gruppe darstellt.

Beispielshafte Reste $Q^1$, $Q^2$, $Q^3$, $Q^4$, $Q^5$, $Q^6$ und $Q^7$ sind bereits oben genannt.

Die neuen Benzylverbindungen, die wertvolle Zwischenprodukte für die Synthese der neuen Doppelankerreaktivfarbstoffe darstellen, können nach an sich bekannten Methoden erhalten werden, wie sie beispielsweise in der älteren Patentanmeldung DE-A-3 731 202 beschrieben sind.

Beispielsweise kann man eine Verbindung der Formel XXIV

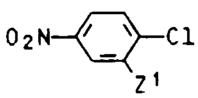 (XXIV),

in der $Z^1$, mit der Ausnahme von $C_2$-$C_{10}$-Alkenyl, die für Z genannte Bedeutung besitzt, mit einem Thioalkanol, z.B. 2-Thioethanol, 2-Methyl-2-thioethanol oder 1-Methyl-2-thioethanol, umsetzen.

Durch Reduktion oder Nitro- zur Aminogruppe und gegebenenfalls Oxidation des Schwefelatoms zur Sulfoxid- oder Sulfongruppe, wobei diese Schritte in beliebiger Reihenfolge durchgeführt werden können, und anschließender Veresterung, z.B. mit Chlorsulfonsäure, gelangt man zum Benzylsulfon der Formel XXV

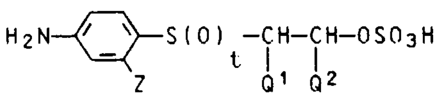 (XXV),

in der $Q^1$, $Q^2$, Z und t jeweils die obengenannte Bedeutung besitzen.

Verbindungen der Formel XXIV können beispielsweise erhalten werden, wenn man ein Benzylchlorid der Formel XXVI

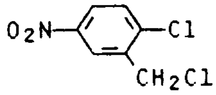 (XXVI)

mit einem Thioalkanol umsetzt, den resultierenden Thioether gegebenenfalls oxidiert und anschließend z.B. mit Chlorsulfonsäure verestert.

Ausgehend vom Benzylchlorid XXVI ist es beispielsweise auch möglich, nach den obengenannten Methoden in einem Schritt sowohl das Chloratom im aromatischen Ring durch den Rest $-S(O)_t-U^1$ und das Chloratom der Benzylgruppe durch den Rest $-S(O)_w-U^2$ zu ersetzen, wobei die Reste $-S(O)_t-U^1$ und $S(O)_w-U^2$ in diesem Fall identisch sind und $U^1$, $U^2$, t und w jeweils die obengenannte Bedeutung besitzen.

Anstelle des Schwefelsäureesters können auf an sich bekanntem Wege auch die anderen unter alkalischen Reaktionsbedingungen abspaltbaren Reste eingeführt werden.

Die Herstellung der Reaktivfarbstoffe der Formel I erfolgt z.B. dadurch, daß man einen geeigneten organischen Farbstoff oder ein geeignetes Farbstoffvorprodukt und die faseraktive Verbindung der Formel XXVII

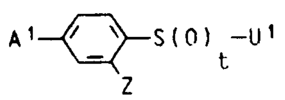 (XXVII),

in der Z, $U^1$ und t jeweils die obengenannte Bedeutung besitzen und $A^1$ den Rest

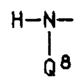

oder

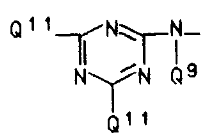 ,

wobei $Q^8$, $Q^9$ und $Q^{11}$ jeweils die obengenannte Bedeutung besitzen, umsetzt, und daß man im Falle der Verwendung von Farbstoffvorprodukten die erhaltenen Zwischenverbindungen nach an sich bekannten Methoden in die gewünschten Farbstoffe umwandelt.

Wenn die Verbindung X-H eine Kupplungskomponente darstellt, gelangt man z.B. zu den erfindungsgemäßen Farbstoffen, wenn man den faserreaktiven Doppelanker der Formel XXVIII

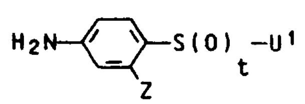 (XXVIII),

in der Z, $U^1$ und t jeweils die obengenannte Bedeutung besitzen, nach an sich bekannter Weise diazotiert und mit der Kupplungskomponente X-H kuppelt.

Die erfindungsgemäßen Doppelankerreaktivfarbstoffe eignen sich vorteilhaft zum Färben von hydroxygruppenhaltigen Fasern, insbesondere von Baumwolle, sowie auch von Wolle.

Als Färbeverfahren eignen sich die bekannten Reaktivfärbeverfahren, insbesondere Ausziehverfahren bei 40 bis 80 °C und Kaltverweilverfahren. Die neuen Farbstoffe zeichnen sich durch hohe Ausgiebigkeit und hohe Naßechtheit aus.

Die folgenden Beispiele, in denen sich Angaben über Prozente sofern nicht anders vermerkt, auf das Gewicht beziehen, sollen die Erfindung näher erläutern.

In den Tabellenbeispielen besitzen die Abkürzungen E-1 bis E-8 die folgende Bedeutung:

E-1:

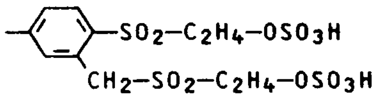

E-2:

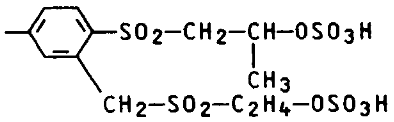

E-3:

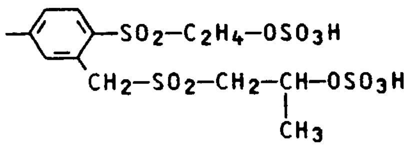

E-4:

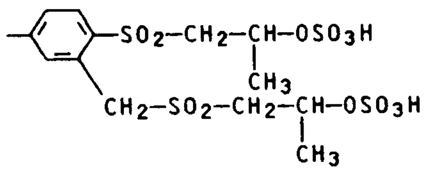

E-5:

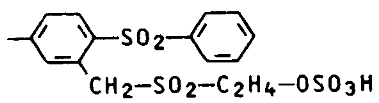

E-6:

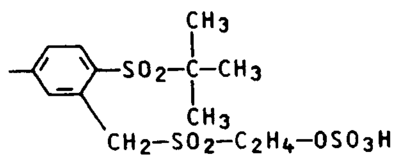

E-7:

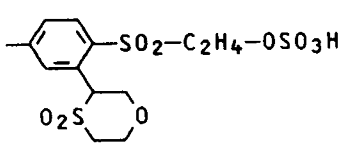

E-8:

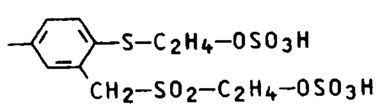

Beispiel 1

Eine Lösung von 24,2 g 4- (2'-Sulfatoethyl)-3-(2'-sulfatoethylsulfonylmethyl)-anilin in 270 ml Wasser wurde mit 30 ml 5 N-Salzsäure und 15 ml 3,33 N $NaNO_2$-Lösung bei 0 bis 5 °C diazotiert und mit einer neutralen wäßrigen Lösung von 14,2 g 1-(4-Sulfophenyl)-3-carboxypyrazolon-5 versetzt. Durch Einstreuen

von Natriumbicarbonat wurde ein pH-Wert von 5 bis 6 eingestellt. Nach beendeter Kupplung wurde der Farbstoff mit Kaliumchlorid ausgesalzen und schonend unter vermindertem Druck getrocknet. Er färbt Baumwolle in lichtechten gelben Tönen und entspricht der Formel

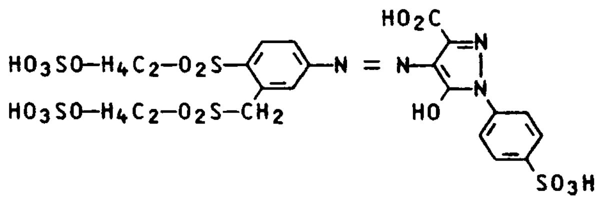

Weitere erfindungsgemäße Farbstoffe, die auf ähnlichem Weg erhalten wurden, sind in Tabelle 1 aufgeführt:

Tabelle 1

E–N = N–K

| Bsp.-Nr. | E | K | Farbton auf Baumwolle |
|---|---|---|---|
| 2 | E-1 | 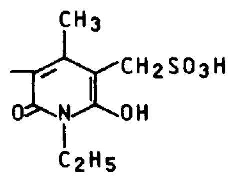 | grünstichig gelb |
| 3 | E-1 | 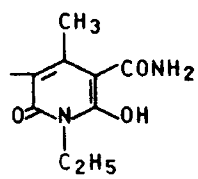 | grünstichig gelb |
| 4 | E-1 | 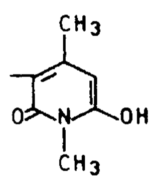 | grünstichig gelb |

Fortsetzung - Tabelle 1

E—N = N—K

| Bsp.-Nr. | E | K | Farbton auf Baumwolle |
|---|---|---|---|
| 5 | E-5 | 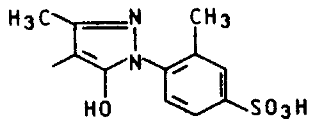 | gelb |
| 6 | E-7 | 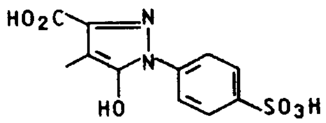 | gelb |
| 7 | E-1 | 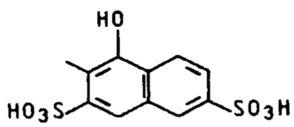 | orange |
| 8 | E-2 | 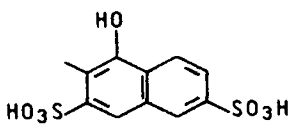 | orange |
| 9 | E-3 | 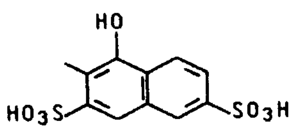 | orange |
| 10 | E-4 | 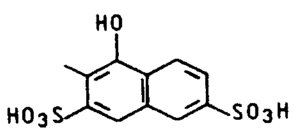 | orange |
| 11 | E-1 | 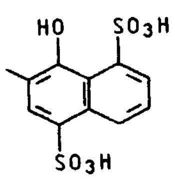 | orange |

Fortsetzung - Tabelle 1

E—N = N—K

| Bsp.-Nr. | E | K | Farbton auf Baumwolle |
|---|---|---|---|
| 12 | E-2 | 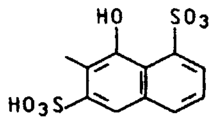 | orange |
| 13 | E-1 | 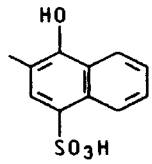 | orangerot |
| 14 | E-2 | 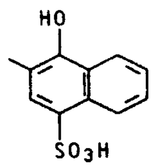 | orangerot |
| 15 | E-1 | 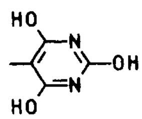 | grünstichig gelb |
| 16 | E-1 | 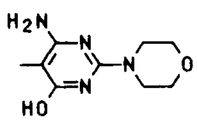 | gelb |
| 17 | E-1 | 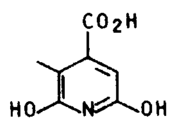 | grünstichig gelb |
| 18 | E-1 | 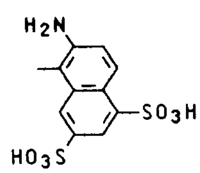 | orangerot |

Beispiel 19

13 g 4-(2'-Hydroxyethylsulfonyl)-3-(2'-hydroxyethylsulfonylmethyl)-anilin wurden in 200 ml Wasser bei 60 °C gelöst, mit 13,5 ml 3,33 N $NaNO_2$-Lösung versetzt und innerhalb 30 min. auf ein Gemisch aus 400 g

Eis und 30 ml 5 N HCl gegeben. Nach beendeter Diazotierung wurde die erhaltene Lösung innerhalb einer Stunde zur Lösung von 15,5 g 1-Hydroxy-7-(3′-sulfophenylamino)-naphthalin-3-sulfonsäure in 200 ml wäßriger Natronlauge gegeben, wobei durch gleichzeitige Zugabe von 50 ml Sodalösung ein pH-Wert von 8 bis 8,5 eingehalten wurde. Die erhaltene Lösung wurde eingedampft und der Rückstand unter vermindertem Druck bei 60 °C getrocknet. Der Rückstand wurde in 200 ml wasserfreiem N-Methylpyrrolidon suspendiert und tropfenweise mit 19 g Chlorsulfonsäure versetzt. Nach 11-stündigem Rühren bei 50 °C wurde auf Eis gegeben, mit Natriumbicarbonat auf pH 6 gestellt und ausgeschiedenes Natriumsulfat bei 0 bis 5 °C abgesaugt. Die erhaltene Lösung wurde eingedampft, mit 1-Propanol versetzt, abgesaugt und der Rückstand schonend unter vermindertem Druck getrocknet. Der erhaltene Farbstoff entspricht der Formel

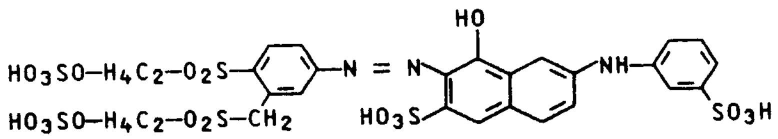

und färbt Baumwolle in echten braunen Tönen.

Beispiel 20

24,9 g 4-(Sulfatoethylsulfonyl)-3-(2″-sulfatopropylsulfonylmethyl)-anilin in 480 ml Wasser wurden bei 0 bis 5 °C salzsauer diazotiert und mit einer neutralen wäßrigen Lösung von 21,2 g 1-Benzoylamino-8-hydroxynaphthalin-4,6-disulfonsäure versetzt. Die Kupplung wurde bei pH 5 bis 6 durch Einstreuen von Natriumcarbonat zu Ende geführt, der gebildete Farbstoff mit Kaliumchlorid ausgesalzen und schonend unter vermindertem Druck getrocknet. Er färbt Baumwolle in brillanten roten Tönen mit guten Echtheiten und entspricht der Formel

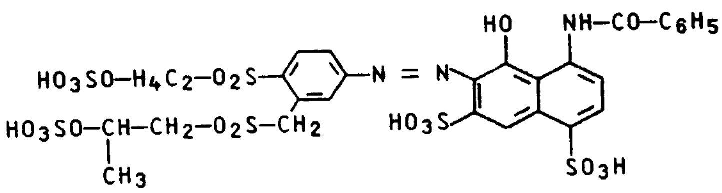

Weitere erfindungsgemäße Farbstoffe sind in Tabelle 2 enthalten.

Tabelle 2

E—N = N—K

| Bsp.-Nr. | E | K | Farbton auf Baumwolle |
|---|---|---|---|
| 21 | E-1 | 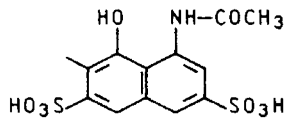 | rot |
| 22 | E-2 | 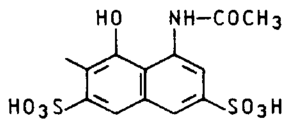 | rot |
| 23 | E-3 | 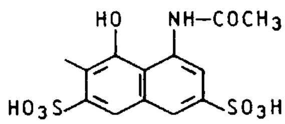 | rot |
| 24 | E-5 | 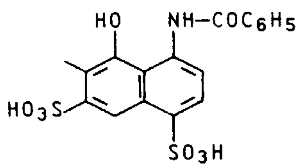 | rot |
| 25 | E-8 | 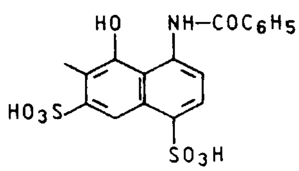 | blaustichig rot |
| 26 | E-1 | 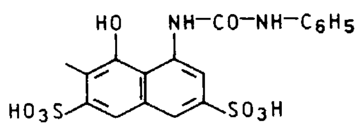 | blaustichig rot |
| 27 | E-1 | 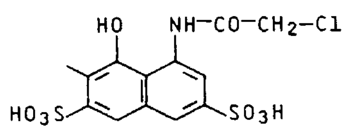 | rot |

Fortsetzung - Tabelle 2

E—N = N—K

| Bsp.-Nr. | E | K | Farbton auf Baumwolle |
|---|---|---|---|
| 28 | E-1 | 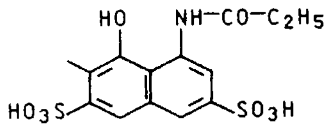 | rot |
| 29 | E-1 | 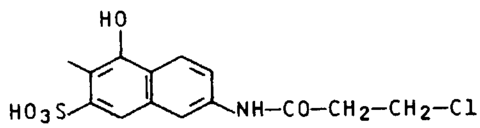 | orange |
| 30 | E-2 | 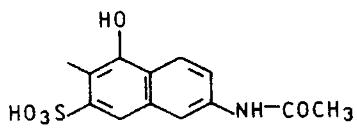 | orange |
| 31 | E-1 | 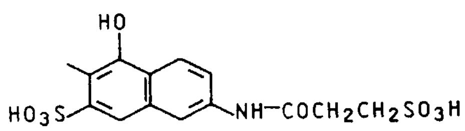 | orange |
| 32 | E-1 | 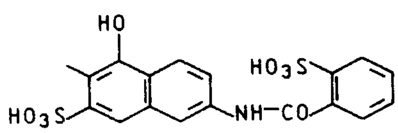 | orange |
| 33 | E-1 | 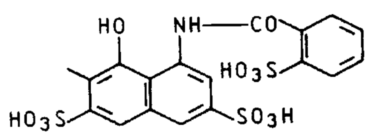 | rot |

Beispiel 34

8,4 g 4-(2'-Sulfatoethylsulfonyl)-3-(2'-sulfatoethylsulfonylmethyl)-anilin wurden in 200 ml $H_2O$ bei pH = 6 mit $NaHCO_3$ gelöst und bei 0 °C mit 3,2 ml 5 N $NaNO_2$ Lösung, 2 ml Ameisensäure und 3 ml 10 N HCl diazotiert. Zu dieser Lösung tropfte man eine Suspension von 5,6 g 1-Amino-8-hydroxynaphthalin-3,6-disulfonsäure in 100 ml $H_2O$ bei einem pH-Wert von 1. Man ließ 4 Stunden rühren, kühlte auf 10 °C ab und tropfte 8,4 g 4-(2'-Sulfatoethylsulfonyl)-3-(2'-sulfatoethylsulfonylmethyl)-anilin - wie vorher beschrieben diazotiert - hinzu und kuppelte mit Natriumbicarbonat bei pH = 4 bis 4,5. Man ließ über Nacht rühren und salzte mit KCl aus. Nach schonender Trocknung erhielt man einen Farbstoff der Formel

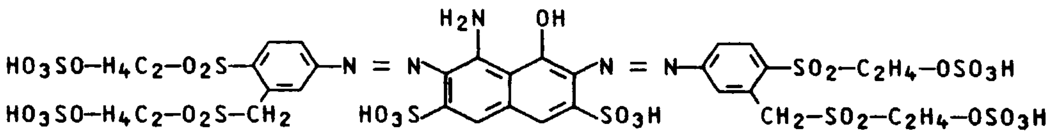

Er färbt Baumwolle in marineblauen Tönen mit guten Echtheiten.

Beispiel 35

7 g Sulfanilamid wurden in 200 ml $H_2O$ gelöst und bei 0 °C mit 8 ml 5 N Natriumnitritlösung und 10 ml 10 N Salzsäure in 1 Stunde diazotiert. Hierzu tropfte man eine Suspension von 13,7 g 1-Amino-8-hydroxynaphthalin-3,6-disulfonsäure (Na-Salz) in 100 ml Wasser suspendiert. Der pH-Wert lag bei 2,5. Es wurde über Nacht bei Raumtemperatur gerührt.

Man tropfte dann bei 10 °C eine Diazoniumsalzlösung hinzu, die man folgendermaßen herstellte: 21 g 4-(2′-Sulfatoethylsulfonyl)-3-(2′-sulfatoethylsulfonylmethyl)-anilin (Na-Salz) in 200 ml $H_2O$ mit Natriumbicarbonat bei pH = 6 kalt gelöst, wurden mit 8 ml 5 N Natriumnitritlösung, 5 ml Ameisensäure und 7 ml 10 N Salzsäure bei 0 bis 5 °C 1 Stunde diazotiert.

Den pH-Wert hielt man 2 Stunden mit Natriumbicarbonat auf 4,5 bis 5 und salzte mit Kaliumchlorid aus. Man saugte ab, trocknete und erhielt ein schwarzes Pulver, das Baumwolle in marineblauen Tönen von guter Lichtechtheit färbt. Der Farbstoff entspricht der Formel

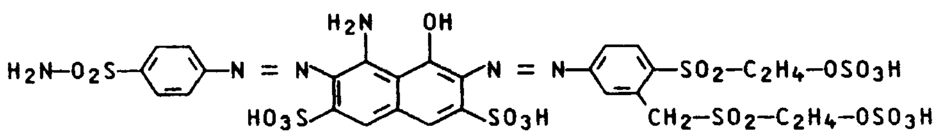

Weitere erfindungsgemäße Farbstoffe enthält Tabelle 3.

Tabelle 3

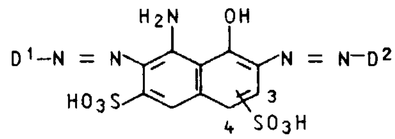

| Bsp.-Nr. | $D^1$ | $D^2$ | Stellung 3,4 | Farbton auf Baumwolle |
|---|---|---|---|---|
| 36 | 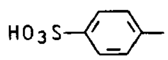 | E-2 | 3 | marineblau |
| 37 | 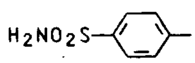 | E-2 | 3 | marineblau |
| 38 | 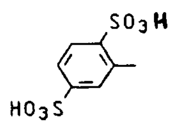 | E-5 | 3 | marineblau |
| 39 | 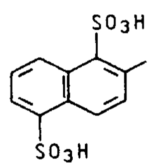 | E-1 | 3 | marineblau |
| 40 | E-2 | E-2 | 3 | marineblau |
| 41 | E-3 | E-3 | 3 | marineblau |
| 42 | E-1 | 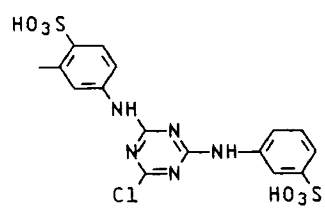 | 3 | marineblau |
| 43 | E-1 | 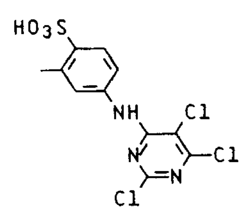 | 3 | marineblau |

Fortsetzung - Tabelle 3

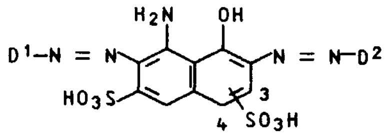

| Bsp.-Nr. | $D^1$ | $D^2$ | Stellung 3,4 | Farbton auf Baumwolle |
|---|---|---|---|---|
| 44 | E-2 | 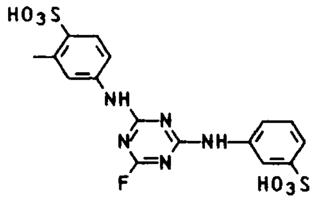 | 3 | marineblau |
| 45 | E-1 | 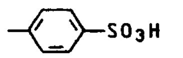 | 3 | marineblau |
| 46 | E-1 | 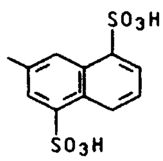 | 4 | marineblau |
| 47 | E-1 | 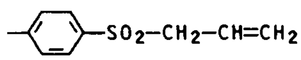 | 3 | marineblau |
| 48 | E-2 | 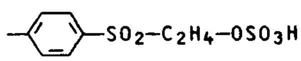 | 3 | marineblau |
| 49 | E-1 | 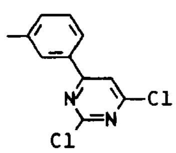 | 3 | marineblau |
| 50 | E-2 | 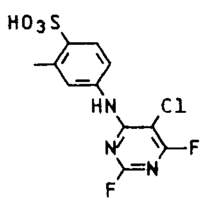 | 3 | marineblau |

Fortsetzung - Tabelle 3

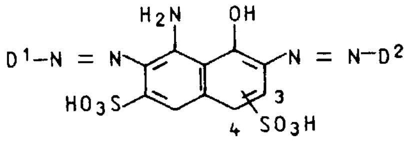

| Bsp.-Nr. | $D^1$ | $D^2$ | Stellung 3,4 | Farbton auf Baumwolle |
|---|---|---|---|---|
| 51 | E-1 | 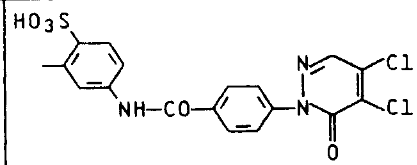 | 3 | marineblau |
| 52 | 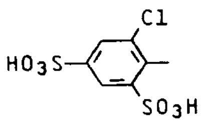 | E-5 | 3 | rotstichig marineblau |
| 53 | E-1 | E-1 | 4 | marineblau |
| 54 | E-2 | E-2 | 4 | marineblau |
| 55 | 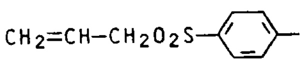 | E-1 | 3 | marineblau |
| 56 | 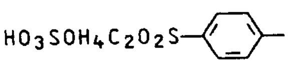 | E-1 | 3 | marineblau |
| 57 | 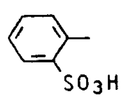 | E-1 | 3 | marineblau |
| 58 | 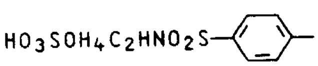 | E-1 | 3 | marineblau |
| 59 | E-1 | 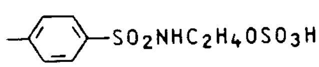 | 3 | marineblau |

Fortsetzung - Tabelle 3

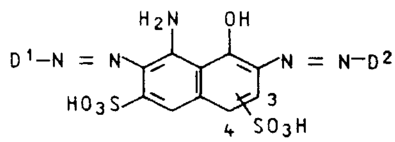

| Bsp.-Nr. | $D^1$ | $D^2$ | Stellung 3,4 | Farbton auf Baumwolle |
|---|---|---|---|---|
| 60 | E-5 | E-1 | 3 | marineblau |
| 61 | E-2 | 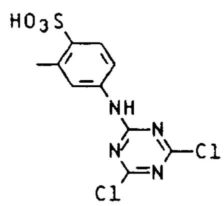 | 3 | marineblau |
| 62 | 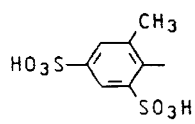 | E-6 | 3 | marineblau |
| 63 | E-6 | 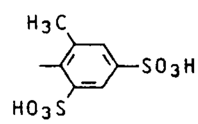 | 3 | marineblau |
| 64 | 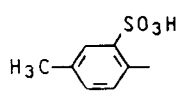 | E-1 | 3 | marineblau |
| 65 | E-1 | 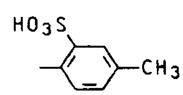 | 3 | marineblau |
| 66 | 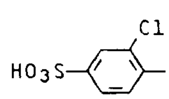 | E-1 | 3 | marineblau |
| 67 | E-3 | 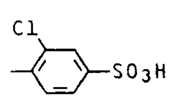 | 3 | marineblau |

Fortsetzung - Tabelle 3

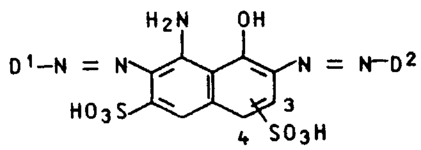

| Bsp.-Nr. | $D^1$ | $D^2$ | Stellung 3,4 | Farbton auf Baumwolle |
|---|---|---|---|---|
| 68 | 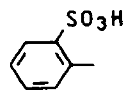 | E-1 | 4 | marineblau |
| 69 | 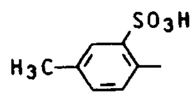 | E-1 | 4 | marineblau |
| 70 | 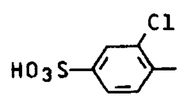 | E-1 | 4 | marineblau |

Beispiel 71

Zu 25,4 g des in 800 ml Wasser neutral gelösten sekundären Kondensationsproduktes aus Anilin-3-sulfonsäure, 2,4,6-Trifluor-1,3,5-triazin und 2-Amino-5-hydroxynaphthalin-7-sulfonsäure wurden bei 0 bis 5 °C 24,2 g salzsauer diazotiertes 4-(2'-Sulfatoethylsulfonyl)-5-(2'-sulfatoethylsulfonylmethyl)-anilin in 300 ml Wasser gegeben. Durch Zusatz von Natriumbicarbonat wurde ein pH-Wert von 5 bis 6 eingestellt und der erhaltene Farbstoff mit Natriumchlorid ausgesalzen. Nach schonendem Trocknen erhielt man ein orangerotes Pulver, das Baumwolle in brillanten orangefarbenen Tönen färbt. Der Farbstoff entspricht der Formel

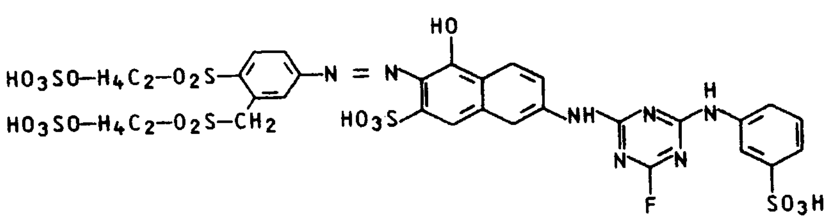

Beispiel 72

In eine neutrale Lösung von 30,2 g des sekundären Kondensationsproduktes aus 1-Amino-8-hydroxynaphthalin-3,6-disulfonsäure, Cyanurchlorid und Anilin-4-sulfonsäure in 700 ml Wasser gab man 24,9 Teile salzsauer diazotiertes 4(2'-Sulfatoethylsulfonyl)-3-(2''-sulfatopropylsulfonylmethyl)-anilin in 300 ml Wasser. Durch Zugabe von Natriumbicarbonat wurde die Kupplung bei pH 5 bis 6 zu Ende geführt. Der gebildete Farbstoff wurde mit Natriumchlorid ausgesalzen und schonend getrocknet. Er entspricht der Formel

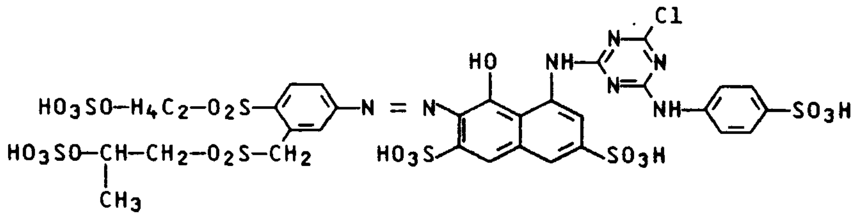

und färbt Baumwolle in brillanten roten Tönen mit guten Echtheiten.

Ähnlich wie in den Beispielen 71 und 72 wurden die in Tabelle 4 enthaltenen Farbstoffe hergestellt.

Tabelle 4

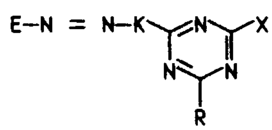

| Bsp.-Nr. | E | K | X | R | Farbton auf Baumwolle |
|---|---|---|---|---|---|
| 73 | E-2 | 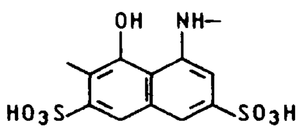 | Cl | $OCH_3$ | rot |
| 74 | E-2 | 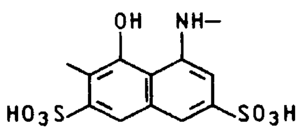 | F | 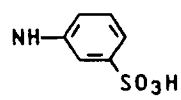 | rot |
| 75 | E-1 | 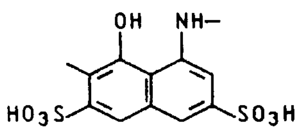 | Cl | $OCH_2CH_2OCH_3$ | rot |

Tabelle 4

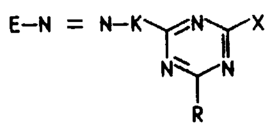

| Bsp.-Nr. | E | K | X | R | Farbton auf Baumwolle |
|---|---|---|---|---|---|
| 76 | E-1 | 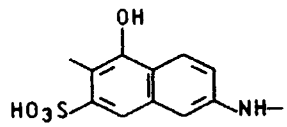 | Cl | 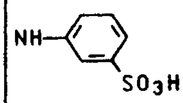 | orange |
| 77 | E-1 | 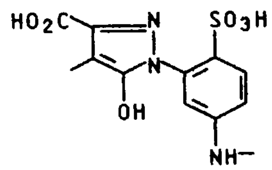 | Cl | 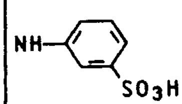 | gelb |
| 78 | E-2 | 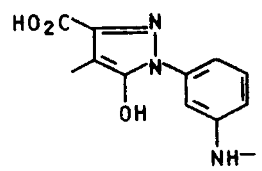 | Cl | 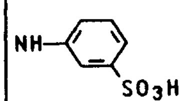 | gelb |
| 79 | E-1 | 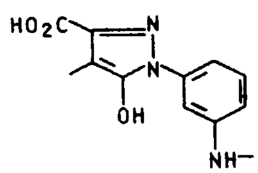 | F | 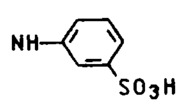 | gelb |
| 80 | E-1 | 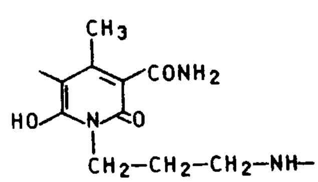 | Cl | 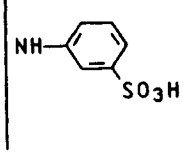 | grünstichig gelb |

Beispiel 81

Eine neutrale wäßrige Lösung von 24,2 g 4-(2'-Sulfatoethylsulfonyl)-3-(2''-sulfatoethylsulfonylmethyl)-anilin in 650 ml Wasser wurde mit einer Suspension von 9,5 g Cyanurchlorid in 150 ml Eiswasser versetzt und 1 Stunde bei 0 bis 5 °C gerührt, wobei durch Zugabe von Natriumbicarbonat ein pH-Wert von 5 bis 6 eingehalten wurde. Man filtrierte, gab die Mischung zu einer bei 40 °C und pH 5 bis 6 gerührten Lösung von 9,5 g 1,3-Diaminobenzol-4-sulfonsäure in 100 ml Wasser und hielt 2 Stunden bei 0 bis 5 °C und pH 5 bis 6.

Nach beendeter Umsetzung wurde bei 0 bis 5 °C durch Zugabe von 15 ml 3,33 N $NaNO_2$ und 30 ml 5 N Salzsäure diazotiert und auf 11,1 g 1,4-Dimethyl-3-sulfomethyl-6-hydroxypyridon-2 gekuppelt. Der erhaltene Farbstoff wurde mit Natriumchlorid ausgesalzen und schonend unter vermindertem Druck getrocknet. Er färbt Baumwolle in brillanten, echten, grünstichig gelben Tönen und entspricht der Formel

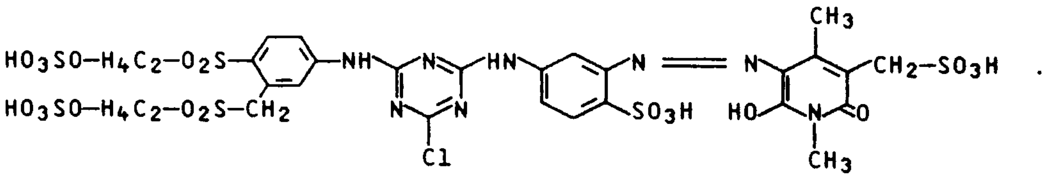

Beispiel 82

33 g des Farbstoffnatriumsalzes der Formel

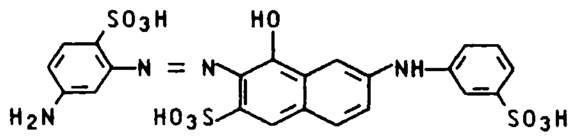

wurden bei pH 6 und 40 °C in Wasser gelöst und mit 31,6 g des in Beispiel 81 beschriebenen Kondensationsproduktes aus Cyanurchlorid und 4-(2'-Sulfatoethylsulfonyl)-3-(2'-sulfatoethylsulfonylmethyl)-anilin, gelöst in 800 ml Wasser, versetzt und weitere 2 Stunden bei 40 °C gerührt, wobei durch Einstreuen von Natriumbicarbonat ein pH-Wert von 5 bis 6 eingehalten wird. Der mit Kaliumchlorid ausgefällte und schonend getrocknete Farbstoff ergibt auf Baumwolle braune Färbungen und entspricht der Formel

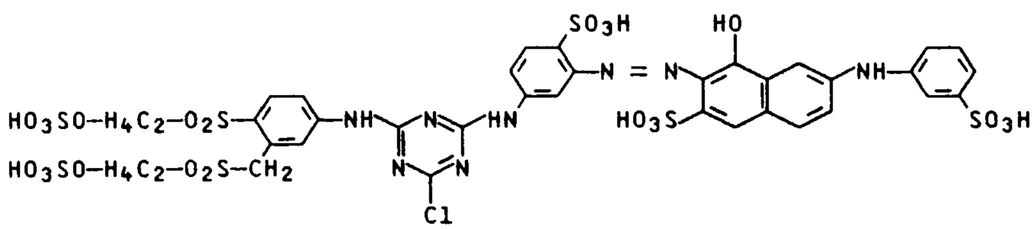

In ähnlicher Weise wurden die in Tabelle 5 beschriebenen Farbstoffe erhalten.

Tabelle 5

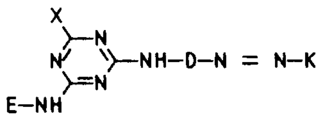

| Bsp.-Nr. | E | X | D | K | Farbton auf Baumwolle |
|---|---|---|---|---|---|
| 83 | E-1 | Cl | 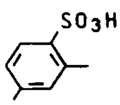 | 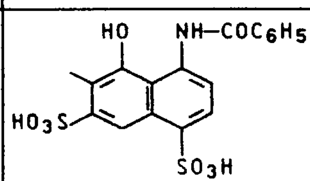 | rot |
| 84 | E-2 | F | 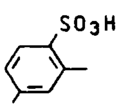 | 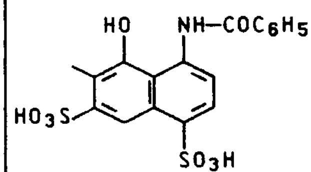 | rot |
| 85 | E-3 | F | 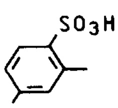 | 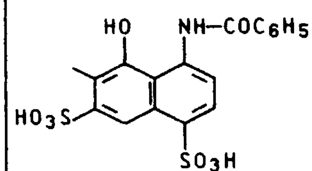 | rot |
| 86 | E-1 | Cl | 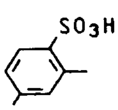 | 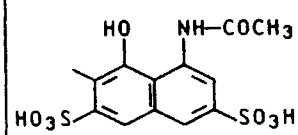 | rot |
| 87 | E-1 | Cl | 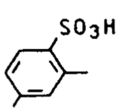 | 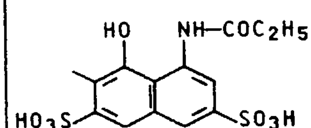 | rot |
| 88 | E-2 | Cl | 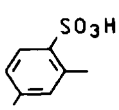 | 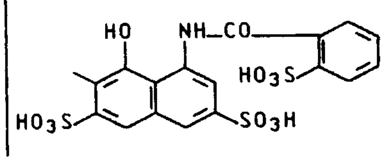 | rot |

Fortsetzung - Tabelle 5

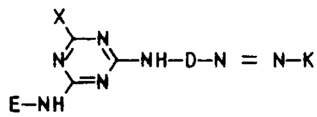

| Bsp.-Nr. | E | X | D | K | Farbton auf Baumwolle |
|---|---|---|---|---|---|
| 89 | E-1 | Cl | 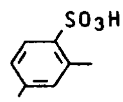 | 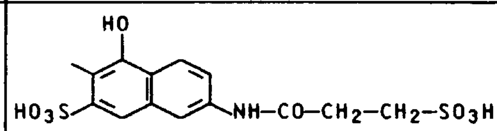 | orange |
| 90 | E-1 | Cl | 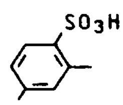 | 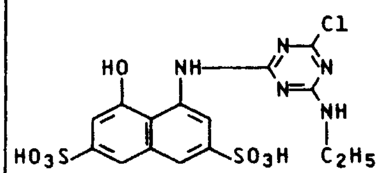 | rot |
| 91 | E-1 | Cl | 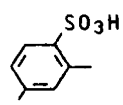 | 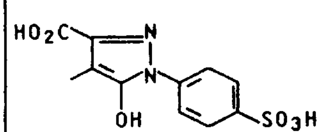 | gelb |
| 92 | E-1 | Cl | 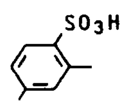 | 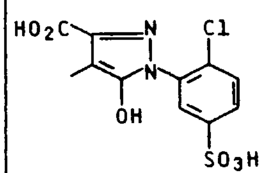 | gelb |
| 93 | E-1 | Cl | 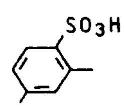 | 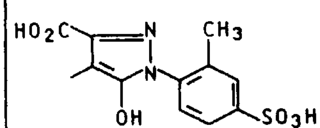 | gelb |
| 94 | E-1 | Cl | 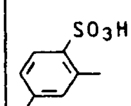 | 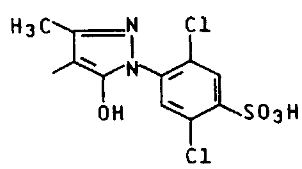 | gelb |

Fortsetzung - Tabelle 5

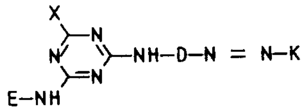

| Bsp.-Nr. | E | X | D | K | Farbton auf Baumwolle |
|---|---|---|---|---|---|
| 95 | E-5 | Cl | 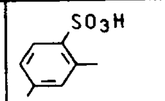 | 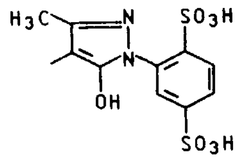 | gelb |
| 96 | E-1 | Cl | 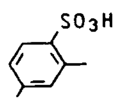 | 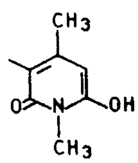 | grünstichig gelb |
| 97 | E-1 | Cl | 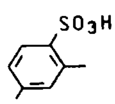 | 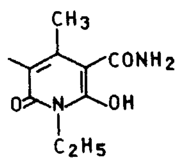 | grünstichig gelb |
| 98 | E-1 | Cl | 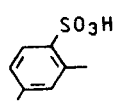 | 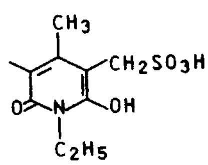 | grünstichig gelb |
| 99 | E-1 | Cl | 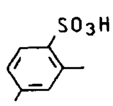 | 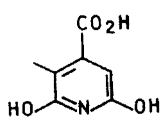 | grünstichig gelb |
| 100 | E-1 | Cl | 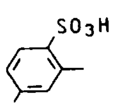 | 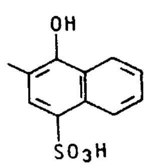 | orangerot |

Fortsetzung - Tabelle 5

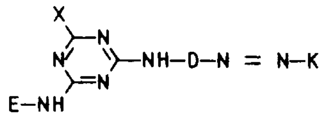

| Bsp.-Nr. | E | X | D | K | Farbton auf Baumwolle |
|---|---|---|---|---|---|
| 101 | E-1 | Cl | 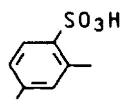 | 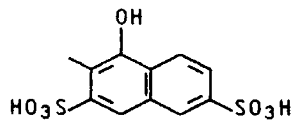 | orange |
| 102 | E-1 | Cl | 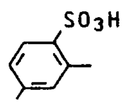 | 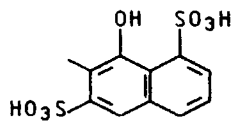 | orange |
| 103 | E-1 | Cl | 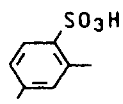 | 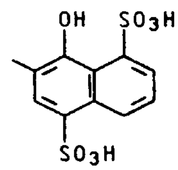 | orangerot |
| 104 | E-1 | Cl | 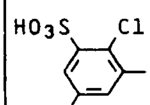 | 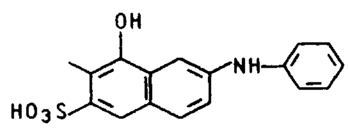 | braun |
| 105 | E-1 | Cl | 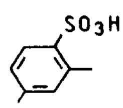 | 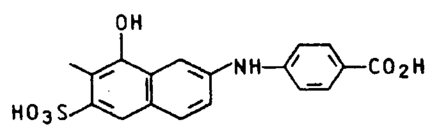 | braun |
| 106 | E-1 | Cl | 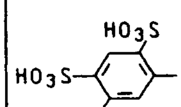 | 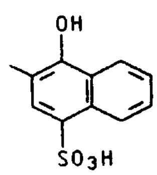 | orangerot |
| 107 | E-1 | Cl | 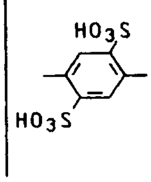 | 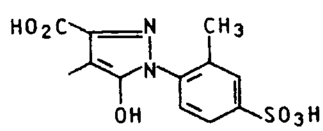 | goldgelb |

Fortsetzung - Tabelle 5

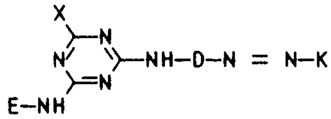

| Bsp.-Nr. | E | X | D | K | Farbton auf Baumwolle |
|---|---|---|---|---|---|
| 108 | E-1 | Cl | 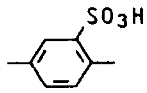 | 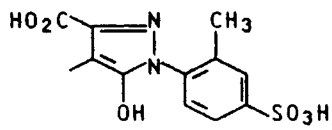 | goldgelb |
| 109 | E-1 | Cl | 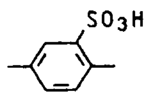 | 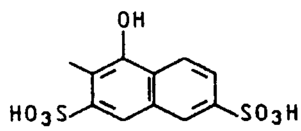 | rot |
| 110 | E-1 | Cl | 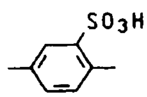 | 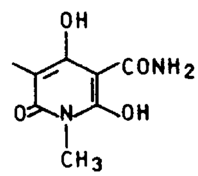 | goldgelb |
| 111 | E-1 | Cl | 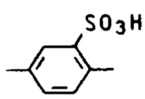 | 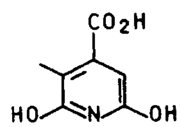 | goldgelb |
| 112 | E-1 | Cl | 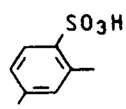 | 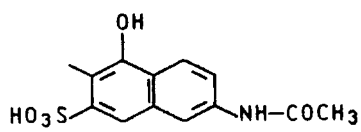 | orange |
| 113 | E-1 | F | 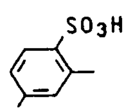 | 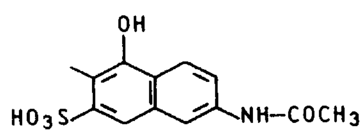 | orange |
| 114 | E-1 | Cl | 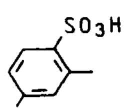 | 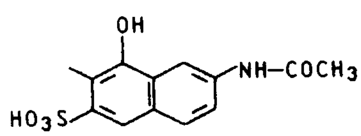 | orange |

Fortsetzung - Tabelle 5

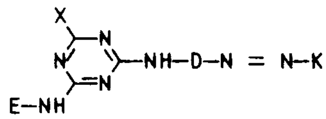

| Bsp.-Nr. | E | X | D | K | Farbton auf Baumwolle |
|---|---|---|---|---|---|
| 115 | E-1 | Cl | 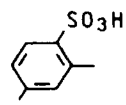 | 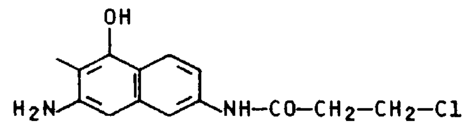 | orange |
| 116 | E-1 | Cl | 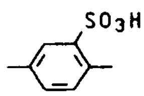 | 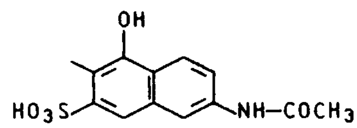 | rot |
| 117 | E-2 | Cl | 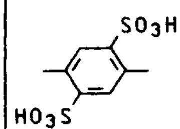 | 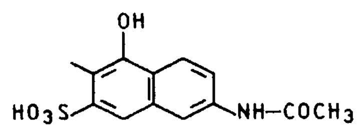 | rot |
| 118 | E-1 | Cl | 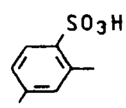 | 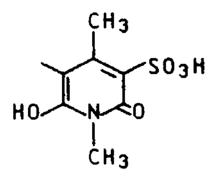 | grünstichig gelb |
| 119 | E-1 | Cl | 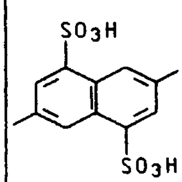 | 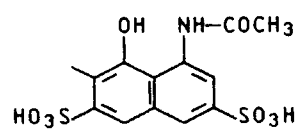 | blaustichig rot |
| 120 | E-1 | Cl | 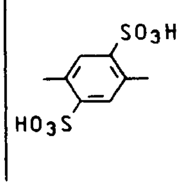 | 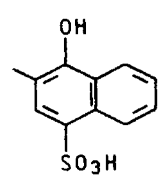 | rot |

Fortsetzung - Tabelle 5

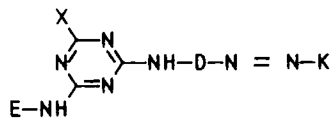

| Bsp.-Nr. | E | X | D | K | Farbton auf Baumwolle |
|---|---|---|---|---|---|
| 121 | E-1 | Cl | 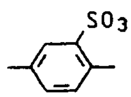 | 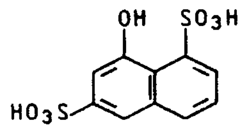 | rot |
| 122 | E-1 | Cl | 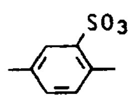 | 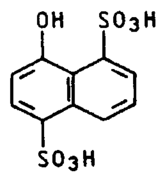 | rot |

Beispiel 123

27,7 g des durch Kupplung von diazotierter 2-Amino-naphthalin-3,6,8-trisulfonsäure auf 3-Aminophenylharnstoff erhaltenen Aminoazofarbstoffs wurden in 250 ml Wasser neutral gelöst und bei 40 °C mit einer wie in Bsp. 81 hergestellten neutralen wäßrigen Lösung von 31,6 g des Kondensationsproduktes aus Cyanurchlorid und 4-(2'-Sulfatoethylsulfonyl)-3-(2'-sulfatoethylsulfonylmethyl)anilin in 800 ml Wasser versetzt und 2 Stunden bei 40 °C gerührt. Dabei wurde durch Einstreuen von Natriumbicarbonat ein pH-Wert von 5 bis 6 eingehalten. Sobald dünnschichtchromatographisch keine freien Aminogruppen mehr nachweisbar waren, wurde mit Kaliumchlorid ausgesalzen und schonend unter vermindertem Druck getrocknet. Das Produkt entspricht der Formel

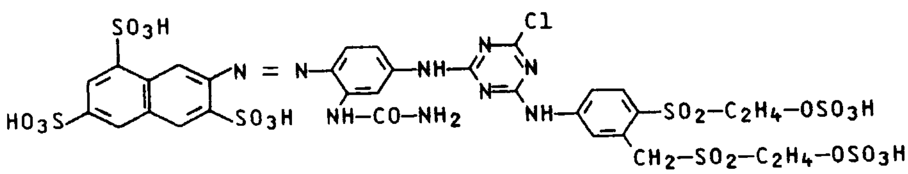

und färbt Baumwolle in echten, goldgelben Nuancen von guten Echtheiten.

Beispiel 124

Die neutrale wäßrige Lösung von 31,6 g des in Bsp. 81 beschriebenen primären Kondensationsproduktes aus Cyanurchlorid und 4-(2'-Sulfatoethylsulfonyl)-3-(2'-sulfatoethylsulfonylmethyl)anilin wurde mit 15 g 1-Amino-8-hydroxynaphthalin-3,6-disulfonsäure versetzt und 2 Stunden bei 40 °C gerührt, wobei durch Zugabe von Natriumbicarbonat ein pH-Wert von 5 bis 6 eingehalten wurde. Man kühlte mit Eis auf 0 bis 5 °C, gab 8,7 g diazotierter Anilin-2-sulfosäure zu und brachte die Kupplung durch Zusatz von Natriumbicarbonat bei pH 5 bis 6 zu Ende. Der entstandene Farbstoff besitzt die Strukturformel

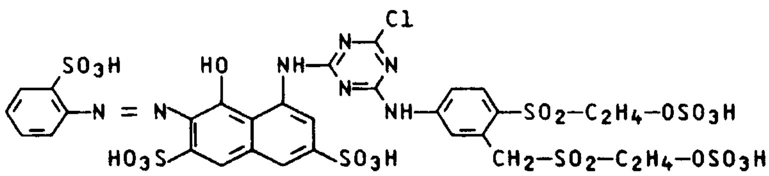

und färbt Baumwolle in echten, brillanten roten Nuancen.

In analoger Weise werden die in Tabelle 6 aufgeführten Farbstoffe erhalten.

Tabelle 6

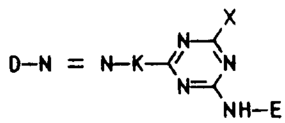

| Bsp.-Nr. | D | K | X | E | Farbton auf Baumwolle |
|---|---|---|---|---|---|
| 125 | 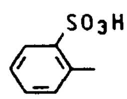 | 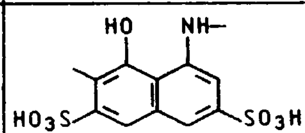 | Cl | E-1 | rot |
| 126 | 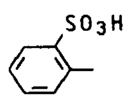 | 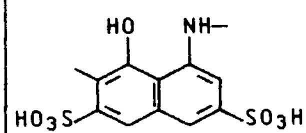 | F | E-2 | rot |
| 127 | 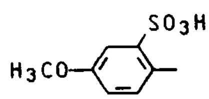 | 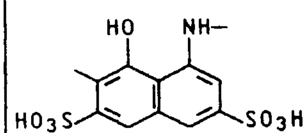 | Cl | E-1 | bordo-rot |
| 128 | 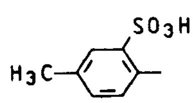 | 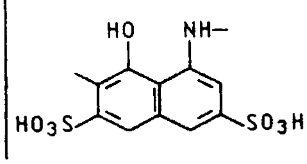 | Cl | E-1 | blaustichig rot |

Fortsetzung - Tabelle 6

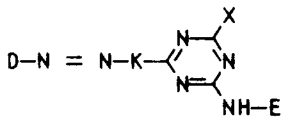

| Bsp.-Nr. | D | K | X | E | Farbton auf Baumwolle |
|---|---|---|---|---|---|
| 129 | 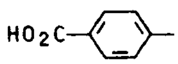 | 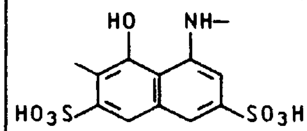 | Cl | E-1 | blaustichig rot |
| 130 | 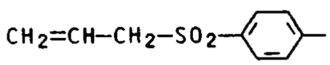 | 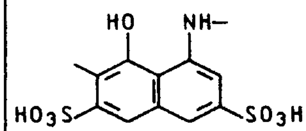 | Cl | E-1 | rot |
| 131 | 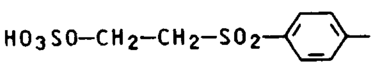 | 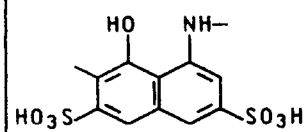 | Cl | E-1 | rot |
| 132 | 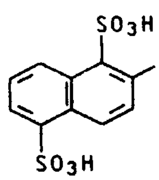 | 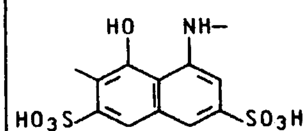 | Cl | E-1 | blaustichig rot |
| 133 | 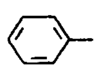 | 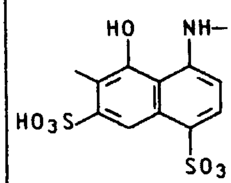 | Cl | E-1 | rot |
| 134 | 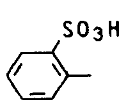 | 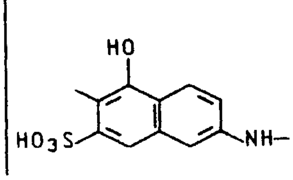 | Cl | E-1 | orange |

Fortsetzung - Tabelle 6

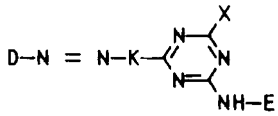

| Bsp.-Nr. | D | K | X | E | Farbton auf Baumwolle |
|---|---|---|---|---|---|
| 135 | 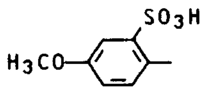 | 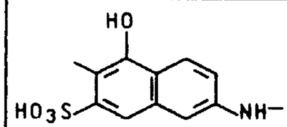 | Cl | E-1 | orange |
| 136 | 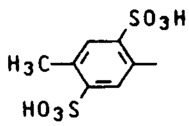 | 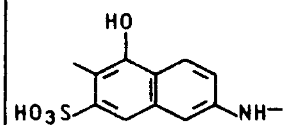 | Cl | E-1 | orange |
| 137 | 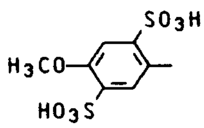 | 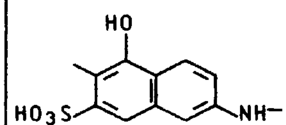 | Cl | E-1 | orange |
| 138 | 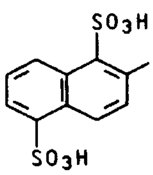 | 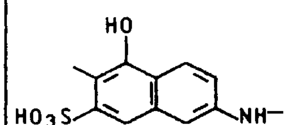 | Cl | E-2 | orange |
| 139 | 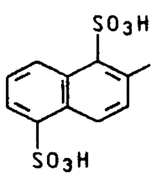 | 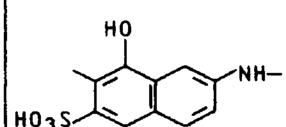 | Cl | E-1 | scharlach-rot |
| 140 | 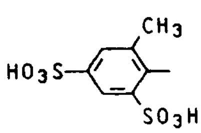 | 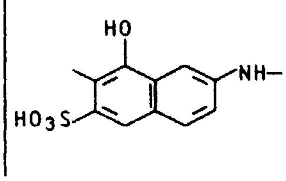 | Cl | E-1 | scharlach-rot |

Fortsetzung - Tabelle 6

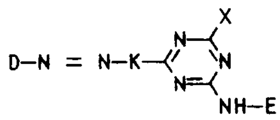

| Bsp.-Nr. | D | K | X | E | Farbton auf Baumwolle |
|---|---|---|---|---|---|
| 141 | 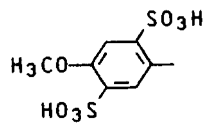 | 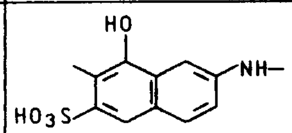 | Cl | E-1 | scharlach-rot |
| 142 | 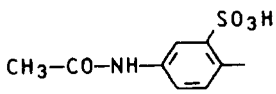 | 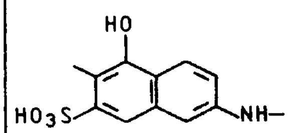 | Cl | E-1 | rot |
| 143 | 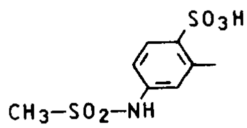 | 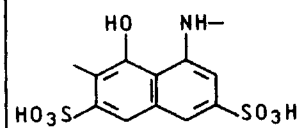 | Cl | E-1 | rot |
| 144 | 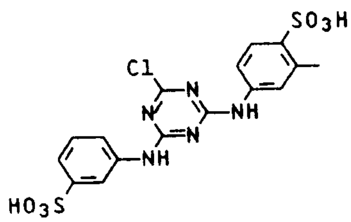 | 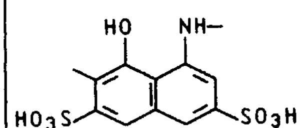 | Cl | E-1 | rot |
| 145 | 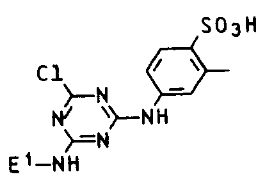 | 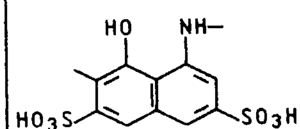 | Cl | E-1 | rot |
| 146 | 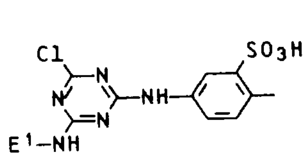 | 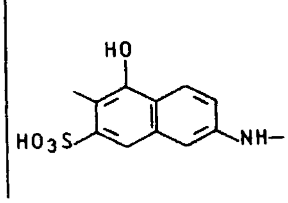 | Cl | E-1 | rot |

Fortsetzung - Tabelle 6

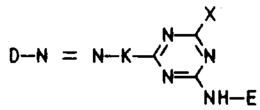

| Bsp.-Nr. | D | K | X | E | Farbton auf Baumwolle |
|---|---|---|---|---|---|
| 147 | 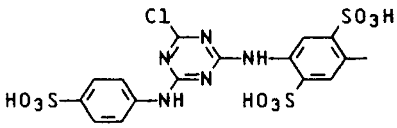 | 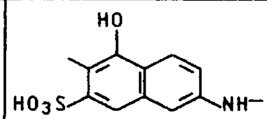 | Cl | E-1 | rot |
| 148 | 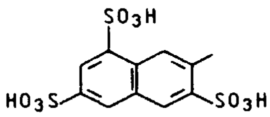 | 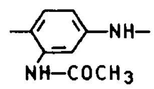 | Cl | E-1 | goldgelb |
| 149 | 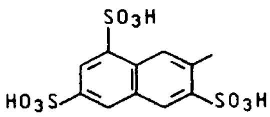 | 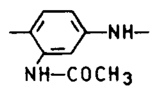 | F | E-1 | goldgelb |
| 150 | 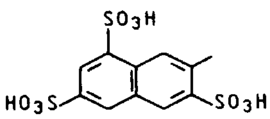 | 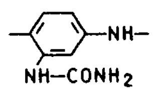 | F | E-1 | goldgelb |
| 151 | 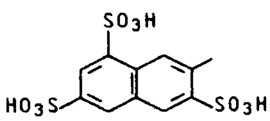 | 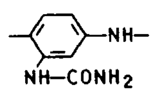 | Cl | E-6 | goldgelb |
| 152 | 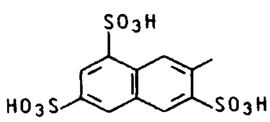 | 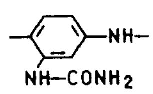 | Cl | E-2 | goldgelb |
| 153 | 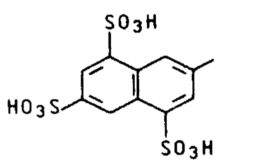 | 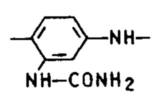 | F | E-3 | goldgelb |

Fortsetzung - Tabelle 6

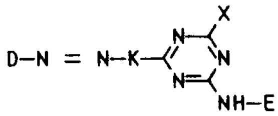

| Bsp.-Nr. | D | K | X | E | Farbton auf Baumwolle |
|---|---|---|---|---|---|
| 154 | 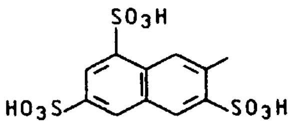 | 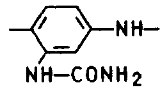 | Cl | E-5 | goldgelb |
| 155 | 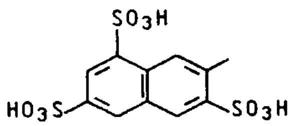 | 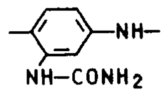 | Cl | E-7 | goldgelb |
| 156 | 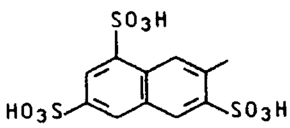 | 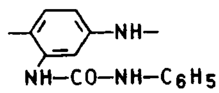 | Cl | E-1 | goldgelb |
| 157 | 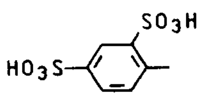 | 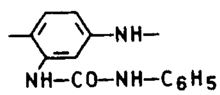 | Cl | E-1 | gelb |
| 158 | 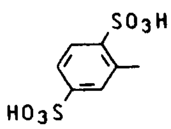 | 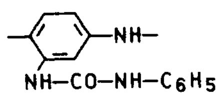 | Cl | E-1 | gelb |
| 159 | 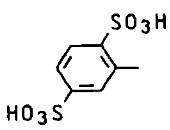 | 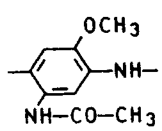 | Cl | E-1 | goldgelb |
| 160 | 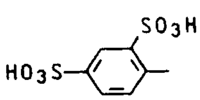 | 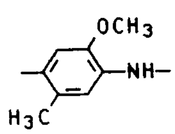 | Cl | E-1 | goldgelb |

Fortsetzung - Tabelle 6

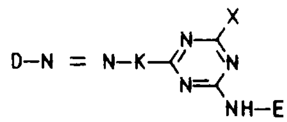

| Bsp.-Nr. | D | K | X | E | Farbton auf Baumwolle |
|---|---|---|---|---|---|
| 161 | 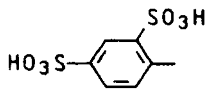 | 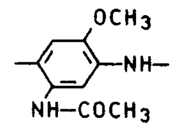 | Cl | E-1 | goldgelb |
| 162 | 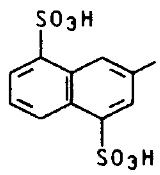 | 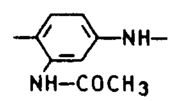 | Cl | E-1 | goldgelb |
| 163 | 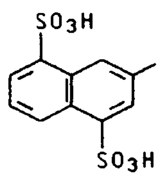 | 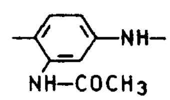 | F | E-1 | goldgelb |
| 164 | 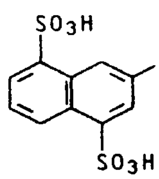 | 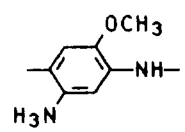 | Cl | E-1 | goldgelb |

Beispiel 165

7 g Sulfanilamid wurden in 200 ml Wasser gelöst und mit einer Mischung aus 8 ml 5 N $NaNO_2$-Lösung und 10 ml 10 N HCl bei 0 °C versetzt. Nach 1 Stunde bei 0 bis 5 °C tropfte man eine Suspension von 13,7 g 1-Amino-8-hydroxynaphthalin-3,6-disulfonsäure in 100 ml Wasser hinzu und rührte 12 Stunden bei Raumtemperatur. Man kühlte auf 10 °C ab und setzte folgende Diazokomponente zu: 500 ml wäßrige Lösung von 23,6 g 4-(2'-Sulfatoethylsulfonyl)-3-(2'-sulfatoethylsulfonylmethyl)anilin wurden bei 0 °C mit einer Suspension von 8,5 g Cyanurchlorid in 200 ml Eiswasser versetzt und 5 Stunden bei ca. 0 bis 5 °C gerührt. Dieses Reaktionsgemisch gab man langsam zu 8,2 g 1,3-Phenylendiamin-4-sulfonsäure - neutral in 150 ml $H_2O$ gelöst - bei 40 °C. Man hielt die Reaktionslösung 1 Stunde bei 40 °C und einem pH-Wert von 5 bis 5,5 und diazotierte dann 3 Stunden bei 0 °C mit 8 ml 5 N $NaNO_2$-Lösung, 5 ml Ameisensäure und 7 ml 10 N HCl. 3 Stunden wurde bei pH = 6 bis 7 (gehalten mit Natriumhydrogencarbonat) gekuppelt und dann bei pH = 6,5 über Nacht gerührt. Man salzte mit 200 g Natriumchlorid aus und erhielt nach dem Trocknen einen Farbstoff der Formel

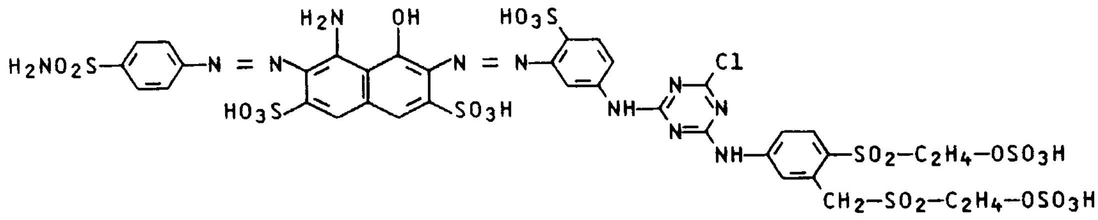

der Baumwolle in echten, marineblauen Tönen färbt.

Beispiel 166

Ersetzte man in Bsp. 165 1-Amino-8-hydroxynaphthalin-3,6-disulfonsäure durch 1-Amino-8-hydroxynaphthalin-4,6-disulfonsäure, so erhielt man einen Farbstoff mit ähnlichen Eigenschaften.

Tabelle 7 enthält weitere Farbstoffbeispiele die in ähnlicher Weise wie Beispiel 165 hergestellt wurden.

Tabelle 7

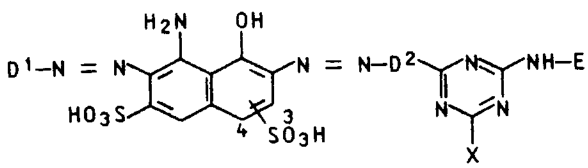

| Bsp.-Nr. | $D^1$ | Stellung 3/4 | $D^2$ | X | E | Farbton auf Baumwolle |
|---|---|---|---|---|---|---|
| 167 | 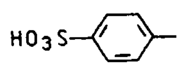 | 3 | 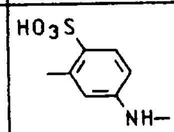 | Cl | E-1 | marineblau |
| 168 | 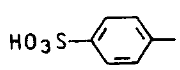 | 3 | 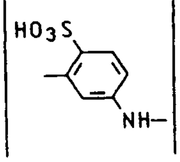 | F | E-2 | marineblau |

Fortsetzung - Tabelle 7

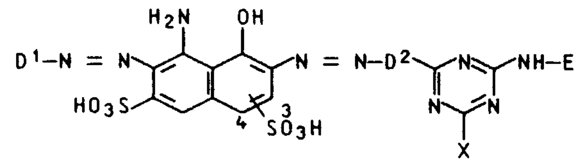

| Bsp.-Nr. | $D^1$ | Stellung 3/4 | $D^2$ | X | E | Farbton auf Baumwolle |
|---|---|---|---|---|---|---|
| 169 | 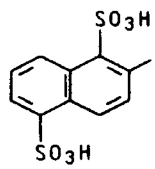 | 3 | 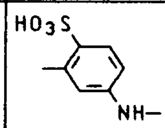 | Cl | E-1 | marineblau |
| 170 | 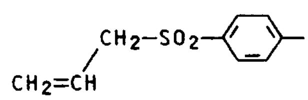 | 4 | 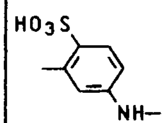 | Cl | E-1 | marineblau |
| 171 | 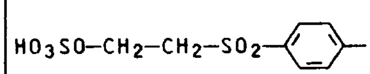 | 3 | 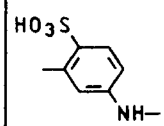 | Cl | E-1 | marineblau |
| 172 | 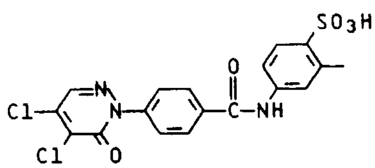 | 3 | 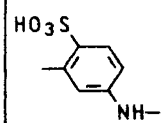 | Cl | E-1 | marineblau |
| 173 | 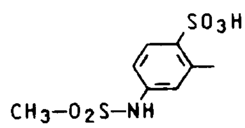 | 3 | 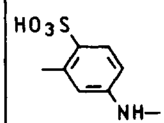 | Cl | E-1 | marineblau |
| 174 | 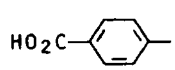 | 3 | 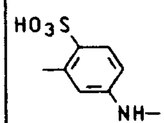 | Cl | E-1 | marineblau |
| 175 | 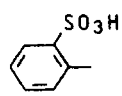 | 3 | 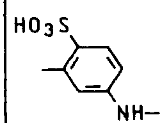 | Cl | E-1 | marineblau |
| 176 | 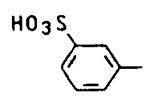 | 3 | 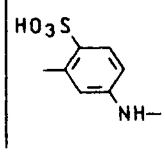 | Cl | E-1 | marineblau |

Beispiel 177

Zu der Lösung von 0,04 mol des analog Beispiel 160 hergestellten und diazotierten sekundären Kondensationsproduktes von 1,3-Phenylendiamin-4-sulfonsäure, Cyanurchlorid und 4-(2'-Sulfatoethylsulfonyl)-3-(2'-sulfatoethylsulfonylmethyl)-anilin gab man bei 0 bis 5 °C 13,7 g 1-Amino-8-hydroxynaphthalin-3,6-disulfonsäure (Na-Salz), suspendiert in 100 ml Wasser. Mit Natriumformiat wurde ein pH-Wert von 2,5 bis 3 gehalten. Man ließ 12 Stunden bei Raumtemperatur rühren, gab dann bei 10 °C 7,8 g salzsauer diazotierte Anilin-4-sulfonsäure, in 200 ml $H_2O$ gelöst, hinzu und hielt den pH-Wert mit Natriumbicarbonat bei 6 bis 6,5. Nach dem Aussalzen mit Natriumchlorid erhielt man einen Farbstoff der Formel

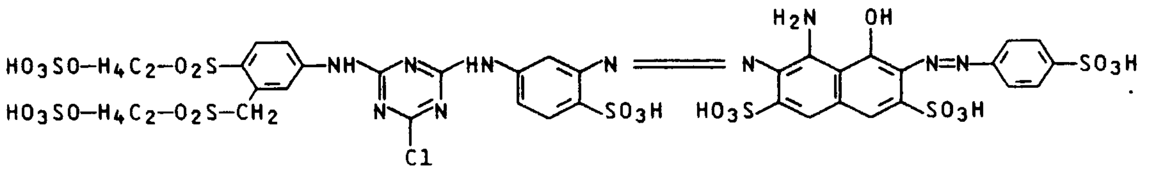

Die in Tabelle 8 aufgeführten Farbstoffe werden in analoger Weise erhalten.

Tabelle 8

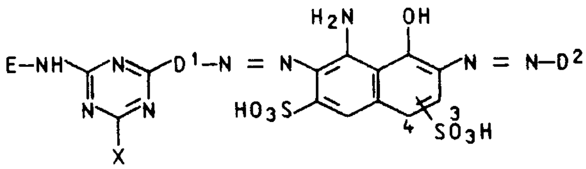

| Bsp.-Nr. | E | X | $D^1$ | Stellung 3/4 | $D^2$ | Farbton auf Baumwolle |
|---|---|---|---|---|---|---|
| 178 | E-1 | F | 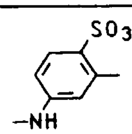 | 3 | 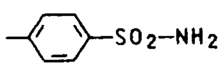 | marineblau |
| 179 | E-1 | Cl | 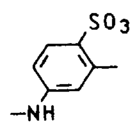 | 3 | 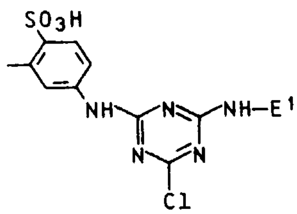 | marineblau |

Fortsetzung - Tabelle 8

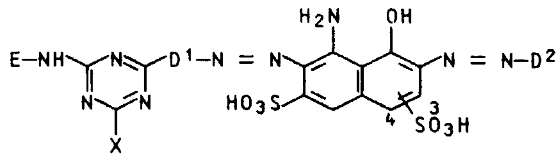

| Bsp.-Nr. | E | X | $D^1$ | Stellung 3/4 | $D^2$ | Farbton auf Baumwolle |
|---|---|---|---|---|---|---|
| 180 | E-1 | Cl | 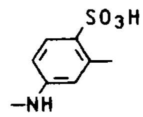 | 3 | 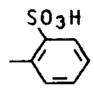 | marineblau |
| 181 | E-1 | Cl | 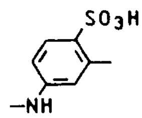 | 3 | 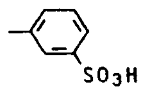 | marineblau |
| 182 | E-2 | Cl | 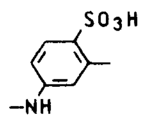 | 3 | 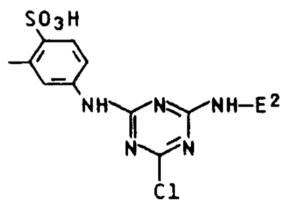 | marineblau |
| 183 | E-3 | Cl | 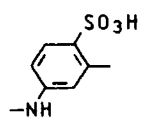 | 3 | 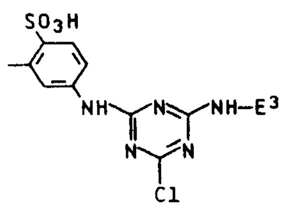 | marineblau |
| 184 | E-5 | Cl | 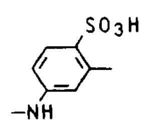 | 3 | 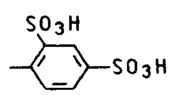 | marineblau |
| 185 | E-1 | Cl | 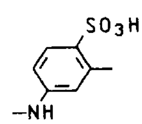 | 4 | 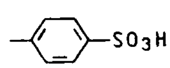 | marineblau |

Fortsetzung - Tabelle 8

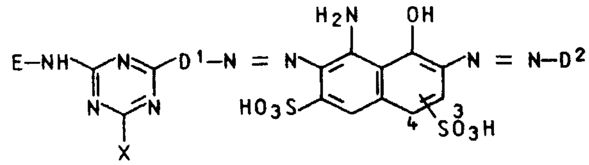

| Bsp.-Nr. | E | X | $D^1$ | Stellung 3/4 | $D^2$ | Farbton auf Baumwolle |
|---|---|---|---|---|---|---|
| 186 | E-1 | Cl | 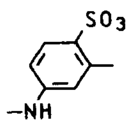 | 3 | 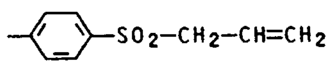 | marineblau |
| 187 | E-1 | Cl | 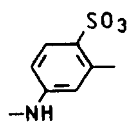 | 4 | 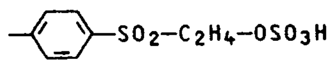 | marineblau |

Beispiel 188

38,6 g des bekannten Farbstoffs der Formel

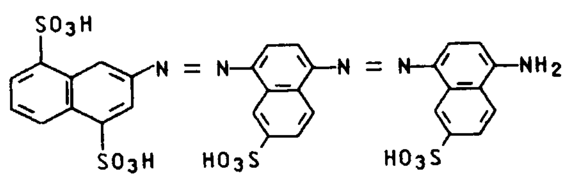

wurden bei neutralem pH in 400 ml Wasser bei 40 °C vorgelegt und mit 31,6 g des wie in Beispiel 81 hergestelten primären Kondensationsproduktes aus Cyanurchlorid und 4-(2'-Sulfatoethylsulfonyl)-3-(2'-sulfatoethylsulfonylmethylanilin in 500 ml Wasser versetzt. Man rührte bei 40 °C und pH = 5 bis 6 bis dünnschichtchromatographisch keine freien Aminogruppen mehr nachweisbar waren. Der nach Aussalzen mit Kaliumchlorid erhaltene Farbstoff entspricht der Formel

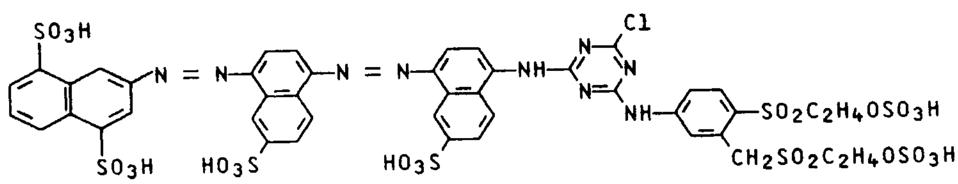

und färbt Baumwolle in rotbraunen Tönen.

Tabelle 9 enthält ähnlich hergestellte Farbstoffe.

Tabelle 9

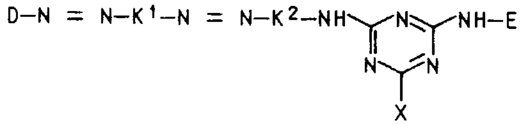

| Bsp.-Nr. | D | $K^1$ | $K^2$ | X | E | Farbton auf Baumwolle |
|---|---|---|---|---|---|---|
| 189 | 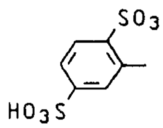 | 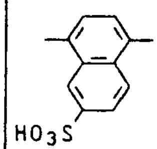 | 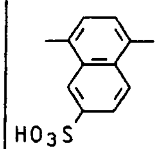 | Cl | E-1 | rotstichig braun |
| 190 | 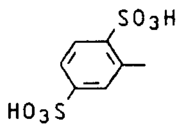 | 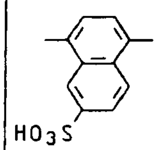 | 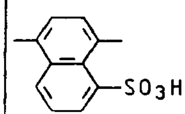 | Cl | E-1 | rotstichig braun |
| 191 | 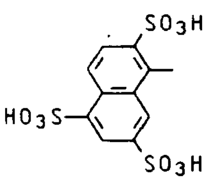 | 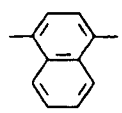 | 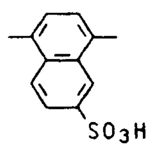 | Cl | E-1 | rotstichig braun |
| 192 | 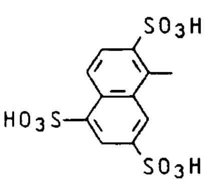 | 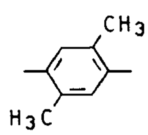 | 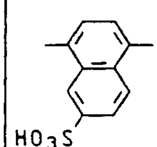 | Cl | E-1 | gelbstichig braun |
| 193 | 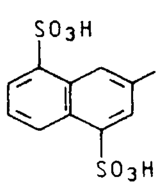 | 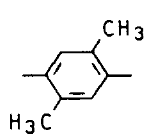 | 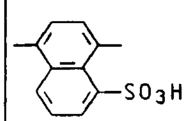 | Cl | E-2 | gelbstichig braun |

Fortsetzung - Tabelle 9

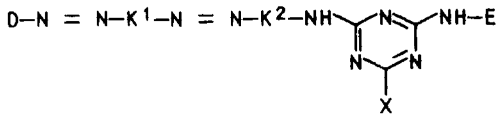

| Bsp.-Nr. | D | $K^1$ | $K^2$ | X | E | Farbton auf Baumwolle |
|---|---|---|---|---|---|---|
| 194 | 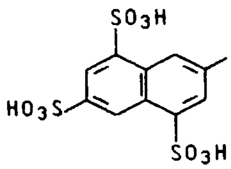 | 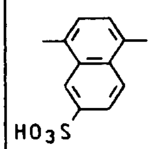 | 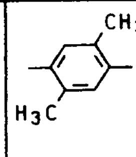 | F | E-1 | gelbstichig braun |
| 195 | 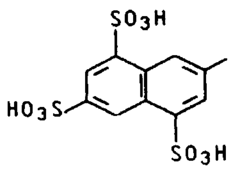 | 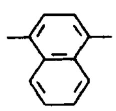 | 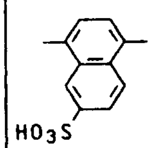 | Cl | E-1 | rotstichig braun |
| 196 | $E^1$ | 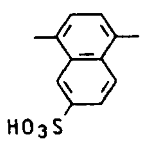 | 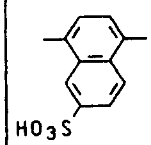 | Cl | 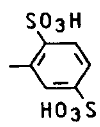 | rotbraun |
| 197 | 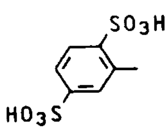 | 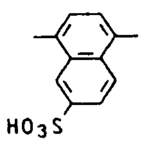 | 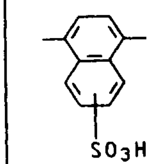 | Cl | E-1 | rotbraun |
| 198 | 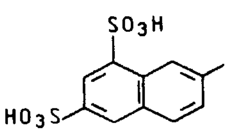 | 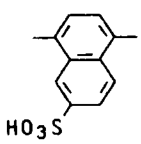 | 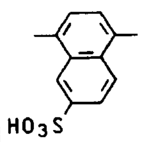 | Cl | E-1 | rotbraun |
| 199 | 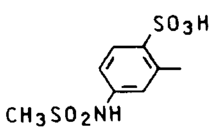 | 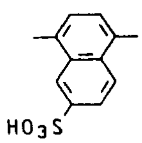 | 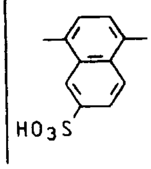 | Cl | E-1 | rotbraun |

Beispiel 200

64,8 g des bekannten Farbstoffs der Formel

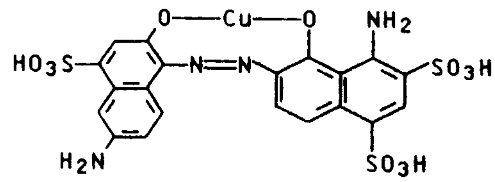

wurden in 700 ml Wasser angerührt. Bei einem pH-Wert von 5,5 - 6 und 40 °C setzte man über 2 Stunden 31,6 g des in Beispiel 81 beschriebenen primären Kondensationsproduktes aus Cyanurchlorid und 4-(2'-Sulfatoethylsulfonyl)-3-(2'-Sulfatoethylsulfonylmethylanilin), gelöst in 800 ml Wasser, hinzu und hielt weitere 30 min bei einem pH-Wert von 5,5 - 6 und 40 - 45 °C. Nach beendeter Umsetzung wurde mit Natriumchlorid ausgesalzen, abfiltriert und unter vermindertem Druck getrocknet. Der resultierende Farbstoff entspricht der Formel

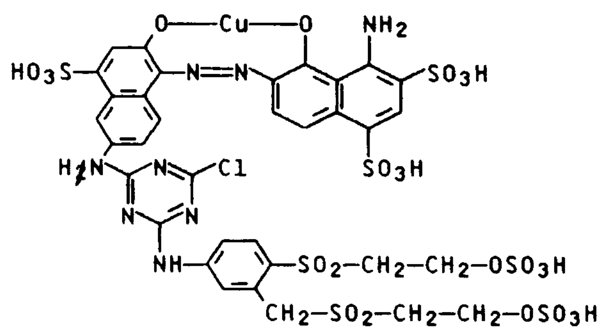

und färbt Baumwolle in echten blauen Tönen.

Beispiel 201

Zu einer Suspension von 75 g des bekannten Dichlortriazinfarbstoffs der Formel

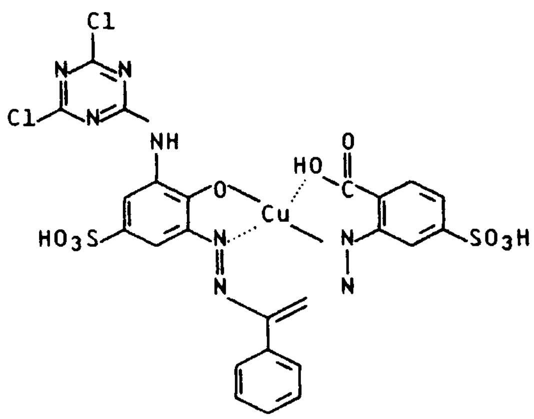

in 600 ml Wasser wurde bei pH = 7 eine Lösung von 49 g 4-(2'-Sulfatoethylsulfonyl-)-3-(2'-sulfatoethylsulfonylmethyl)anilin in 600 ml Wasser eingetragen. Die Suspension wurde auf 40 - 45 °C erwärmt und der pH wurde durch Zusatz von $NaHCO_3$ am Neutralpunkt gehalten. Nach 2,5 Stunden wurde der gebildete Farbstoff entsprechend der Formel

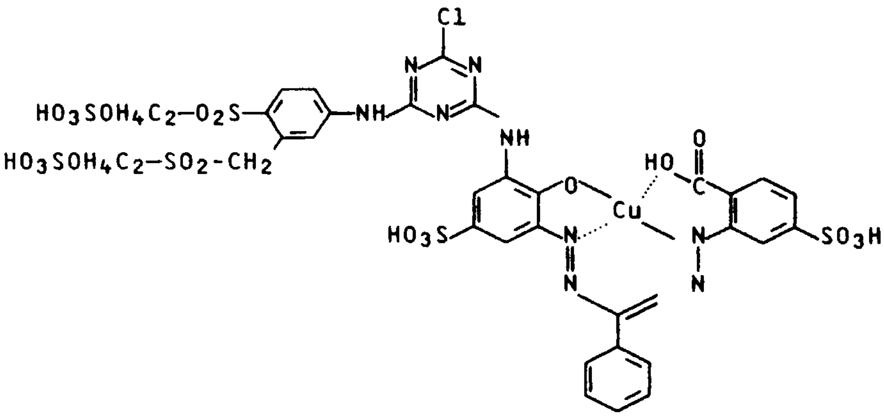

mit 250 g Natriumchlorid ausgesalzen, abfiltriert und getrocknet. Das erhaltene dunkelblaue Farbstoffpulver färbt Baumwolle in klaren blauen Tönen. Die Färbungen sind licht- und naßecht, sie weisen eine bemerkenswerte Stabilität gegenüber oxidativen Einflüssen auf.

Analog der in Beispiel 201 beschriebenen Methode können die in der Tabelle 10 aufgeführten Farbstoffe der Formel

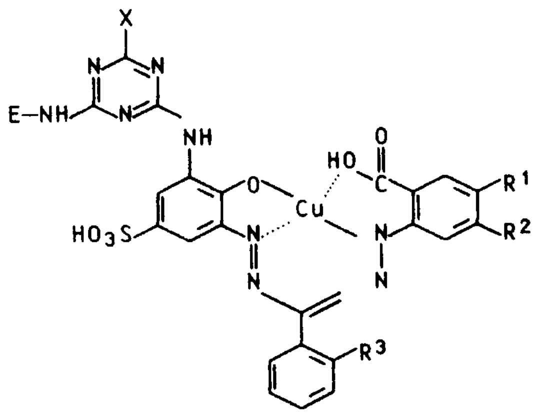

hergestellt werden.

Tabelle 10

| Bsp. | E | X | $R^1$ | $R^2$ | $R^3$ |
|---|---|---|---|---|---|
| 202 | E-2 | Cl | H | $SO_3H$ | H |
| 203 | E-3 | Cl | H | $SO_3H$ | H |
| 204 | E-1 | Cl | $SO_3H$ | H | H |
| 205 | E-2 | Cl | $SO_3H$ | H | H |
| 206 | E-3 | Cl | $SO_3H$ | H | H |
| 207 | E-1 | Cl | $SO_3H$ | H | $SO_3H$ |
| 208 | E-1 | Cl | H | $SO_3H$ | $SO_3H$ |

Beispiel 209

43,4 g 88 %ige 1-Amino-4-bromanthrachinon-2-sulfonsäure, 39 g des Amins der Formel

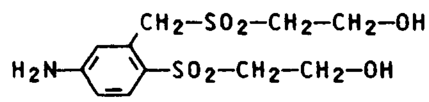

1,5 g Cu-Pulver, 0,75 g Cu-II-Sulfat und 50,4 g Natriumhydrogencarbonat wurden 120 Stunden auf 65 bis 70 °C erhitzt. Nach vollständigem Umsatz (Dünnschichtchromatographie) wurde heiß filtriert und das Filtrat mit conc. Salzsäure auf pH 1 gestellt. Der ölige Rückstand wurde bei 0 bis 5 °C durch Ausrühren mit 100 ml Ethanol kristallisiert, das kristalline Produkt isoliert, mit Ethanol gewaschen und getrocknet. Man erhielt 45 g einer Verbindung der Formel

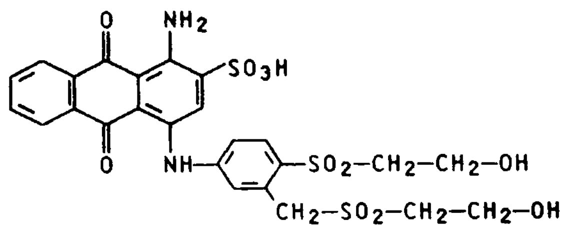

In analoger Weise werden die Verbindungen der Formel

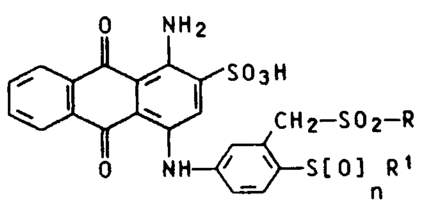

erhalten.

Tabelle 11

| Bsp.-Nr. | R | $R^1$ | n |
|---|---|---|---|
| 210 | $CH_2CH_2OH$ | $CH_2CH_2OH$ | 0 |
| 211 | $CH_2CH_2OH$ | $CH_2CH_2OH$ | 1 |
| 212 | $CH_2CH_2OH$ | $CH_2CH(CH_3)OH$ | 0 |
| 213 | $CH_2CH_2OH$ | $CH_2CH(CH_3)OH$ | 1 |
| 214 | $CH_2CH_2OH$ | $CH_2CH(CH_3)OH$ | 2 |
| 215 | $CH_2CH(CH_3)OH$ | $CH_2CH_2OH$ | 0 |
| 216 | $CH_2CH(CH_3)OH$ | $CH_2CH_2OH$ | 1 |
| 217 | $CH_2CH(CH_3)OH$ | $CH_2CH_2OH$ | 2 |
| 218 | $CH_2CH(CH_3)OH$ | $CH_2CH(CH_3)OH$ | 0 |
| 219 | $CH_2CH(CH_3)OH$ | $CH_2CH(CH_3)OH$ | 1 |
| 220 | $CH_2CH(CH_3)OH$ | $CH_2CH(CH_3)OH$ | 2 |

Beispiel 221

10 g der in Bsp. 209 erhaltenen Verbindung der Formel

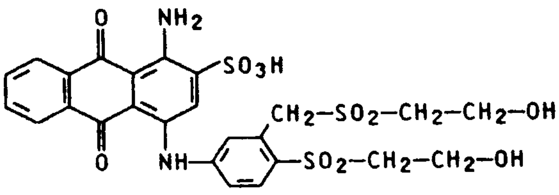

wurden in 40 g Chlorsulfonsäure 3 Stunden bei 20 bis 25 °C gerührt. Die Schmelze wurde auf 400 g Eis/Wasser gefällt und mit Natriumhydrogencarbonat auf pH = 5 gestellt. Nach Sprühtrocknung der Lösung wurde ein Produkt isoliert, das neben Salz den Farbstoff der Formel

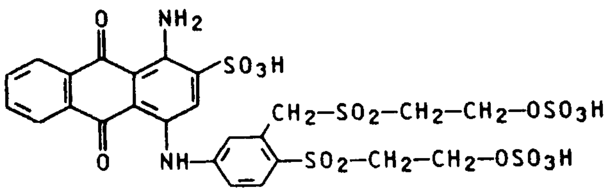

enthielt. Auf Baumwolle wurden brillante blaue Färbungen mit guten Echtheiten erhalten.

In analoger Weise werden die in Tabelle 12 aufgeführten Farbstoffe der Formel

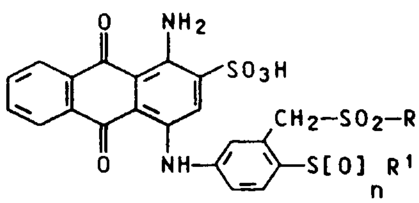

erhalten.

Tabelle 12

| Bsp.-Nr. | R | $R^1$ | n |
|---|---|---|---|
| 222 | $CH_2CH_2OSO_3H$ | $CH_2CH_2OSO_3H$ | 0 |
| 223 | $CH_2CH_2OSO_3H$ | $CH_2CH_2OSO_3H$ | 1 |
| 224 | $CH_2CH_2OSO_3H$ | $CH_2CH(CH_3)OSO_3H$ | 0 |
| 225 | $CH_2CH_2OSO_3H$ | $CH_2CH(CH_3)OSO_3H$ | 1 |
| 226 | $CH_2CH_2OSO_3H$ | $CH_2CH(CH_3)OSO_3H$ | 2 |
| 227 | $CH_2CH(CH_3)OSO_3H$ | $CH_2CH(CH_3)OSO_3H$ | 0 |
| 228 | $CH_2CH(CH_3)OSO_3H$ | $CH_2CH(CH_3)OSO_3H$ | 1 |
| 229 | $CH_2CH(CH_3)OSO_3H$ | $CH_2CH(CH_3)OSO_3H$ | 2 |
| 230 | $CH_2CH(CH_3)OSO_3H$ | $CH_2CH_2OSO_3H$ | 0 |
| 231 | $CH_2CH(CH_3)OSO_3H$ | $CH_2CH_2OSO_3H$ | 1 |
| 232 | $CH_2CH(CH_3)OSO_3H$ | $CH_2CH_2OSO_3H$ | 2 |

Beispiel 233

19,1 g der Verbindung der Formel

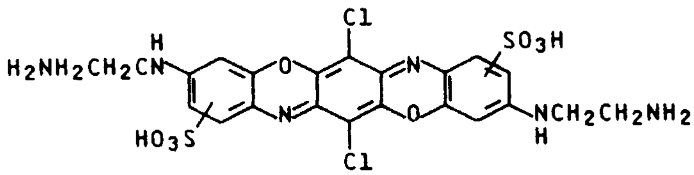

wurden in 1000 g Wasser eingerührt, wobei mit Natronlauge pH 10 eingestellt wurde. Diese Lösung wurde in eine 60 °C warme, auf pH 6 bis 8 gestellte Lösung des Kondensationsproduktes aus 13,5 g Cyanurchlorid mit 31,9 g 4-(2'-Sulfatoethylsulfonyl)-5-(2'-sulfatoethylsulfonylmethyl)anilin getropft. Unter Aufrechterhaltung von pH = 6,5 bis 7 wurde bei 60 °C gerührt bis die Umsetzung beendet war, was etwa 1 Stunde in Anspruch nahm (DC). Nach dem Abkühlen auf Raumtemperatur wurde mit 500 g NaCl ausgesalzen, der ausgefallene Farbstoff abgesaugt und getrocknet. Er färbt Baumwolle in brillanten blauen Tönen mit guten Echtheiten und entspricht der Formel

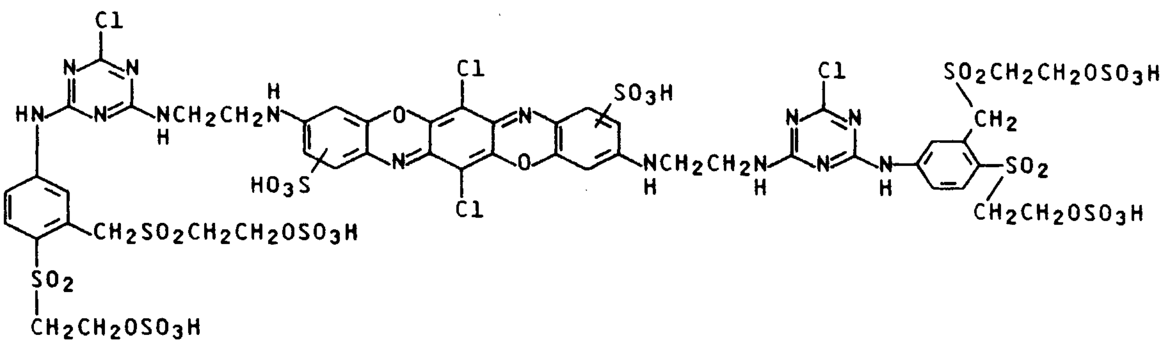

Weitere Farbstoffe, die in analoger Weise erhalten werden, sind in Tabelle 13 aufgeführt.

Tabelle 13

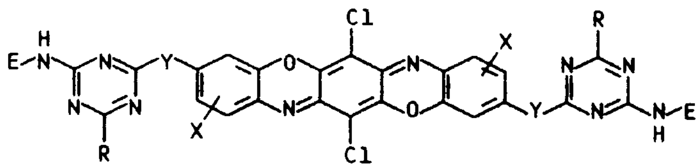

| Bsp.-Nr. | Y | X | R | E | Farbton auf Baumwolle |
|---|---|---|---|---|---|
| 234 | 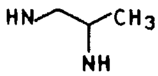 | $SO_3H$ | Cl | E-1 | blau |
| 235 | 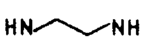 | $SO_3H$ | F | E-1 | blau |
| 236 | 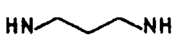 | $SO_3H$ | Cl | E-1 | blau |
| 237 | 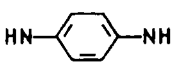 | $SO_3H$ | Cl | E-1 | blau |
| 238 | 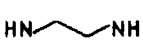 | $SO_2C_2H_4OSO_3H$ | Cl | E-1 | blau |
| 239 | 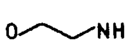 | $SO_3H$ | Cl | E-1 | rot |
| 240 | 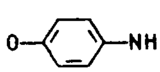 | $SO_3H$ | Cl | E-1 | rot |

Fortsetzung - Tabelle 13

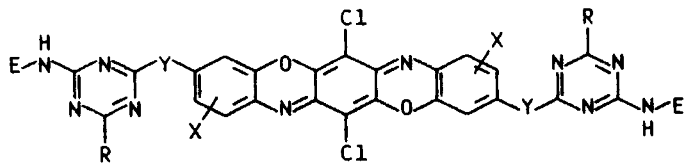

| Bsp.-Nr. | Y | X | R | E | Farbton auf Baumwolle |
|---|---|---|---|---|---|
| 241 | 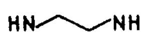 | $SO_3H$ | Cl | E-2 | blau |
| 242 | 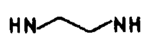 | $SO_3H$ | F | E-2 | blau |
| 243 | 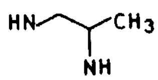 | $SO_3H$ | Cl | E-2 | blau |
| 244 | 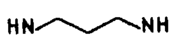 | $SO_3H$ | Cl | E-2 | blau |
| 245 | 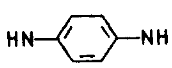 | $SO_3H$ | Cl | E-2 | blau |
| 246 | 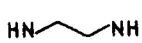 | $SO_2C_2H_4OSO_3H$ | Cl | E-2 | blau |
| 247 | 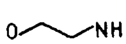 | $SO_3H$ | Cl | E-2 | rot |
| 248 | 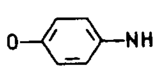 | $SO_3H$ | Cl | E-2 | rot |

Fortsetzung - Tabelle 13

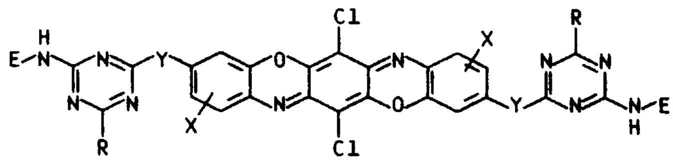

| Bsp.-Nr. | Y | X | R | E | Farbton auf Baumwolle |
|---|---|---|---|---|---|
| 249 | 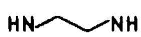 | $SO_3H$ | Cl | E-3 | blau |
| 250 | 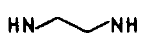 | $SO_3H$ | F | E-3 | blau |
| 251 | 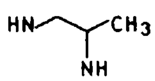 | $SO_3H$ | Cl | E-3 | blau |
| 252 | 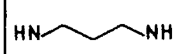 | $SO_3H$ | Cl | E-3 | blau |
| 253 | 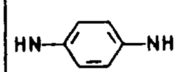 | $SO_3H$ | Cl | E-3 | blau |
| 254 | 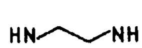 | $SO_2C_2H_4OSO_3H$ | Cl | E-3 | blau |
| 255 | 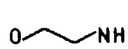 | $SO_3H$ | Cl | E-3 | rot |
| 256 | 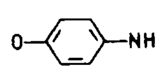 | $SO_3H$ | Cl | E-3 | rot |

Beispiel 257

Zu einer neutralen Lösung von 48 g 4-(2'-Sulfatoethylsulfonyl)-5-(2"-sulfatoethylsulfonylmethyl)anilin in 200 ml Wasser gab man bei 5 -10 °C 97 g Kupferphthalocyanin-tetrasulfochlorid als feuchten Preßkuchen.

Man rührte 12 Stunden bei 20 - 25 °C und hielt den PH-Wert durch Zugabe von 10 Ziger Sodalösung bei 6,5 - 7,3. Danach wurde abgesaugt und der Filterrückstand getrocknet. Man isolierte 47 g des Farbstoffs der Formel

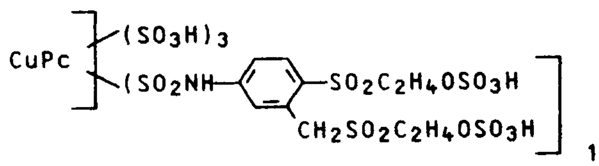

der 2,7 % NaCl enthielt. Der Farbstoff färbt Baumwolle in türkisen Farbtönen.

Beispiel 258

97 g Kupferphthalocyanin-tetrasulfochlorid wurden in 750 ml Wasser bei 0 -5 °C angerührt. Man gab 20,5 g Monoacetylethylendiamin zu und hielt den pH-Wert mit 10 %iger Sodalösung bei 7,3 bis 7,5. Nach 12 Stunden bei 20 -25 °C wurde durch Zugabe von Natronlauge der pH-Wert auf 10 erhöht und eine Stunde bei 95 °C gerührt. Mit konz. Salzsäure wurde ein Niederschlag ausgefällt. Man saugte ab, wusch mit ca. 2 %iger Salzsäure nach und rührte in 500 ml Wasser an. Mit Natronlauge stellte man den pH-Wert der Lösung auf 7,0 - 7,2 und gab 21,2 g Cyanurchlorid bei 0 - 5 °C zu. Der pH-Wert wurde durch Zutropfen von Sodalösung bei 6,5 - 7,0 gehalten. Nach 3 Stunden wurden 49 g 4-(2'-Sulfatoethylsulfonyl)-5-(2''-sulfatoethylsulfonylmethyl)-anilin zugegeben und die Temperatur auf 35 - 40 °C erhöht. Die Farbstofflösung wurde spühgetrocknet. Man isolierte 185 g des Farbstoffs der Formel

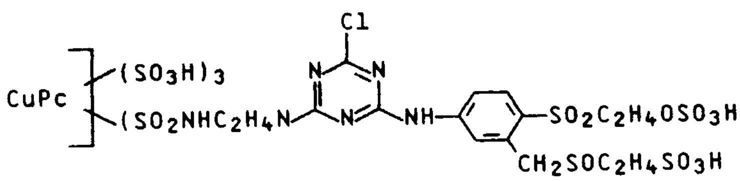

Der Farbstoff färbt Baumwolle in türkisen Farbtönen.

Beispiel 259

97 g Kupferphthalocyanin-tetrasulfochlorid wurden in 600 ml Wasser bei 0 - 50 °C angerührt. Man gab 41 g N-Monoacetyl-m-phenylendiamin zu und hielt den pH-Wert durch Zugabe von 10 %iger Sodalösung bei 6,8 bis 7,0. Dann gab man 12 g 25 %ige Ammoniaklösung und 16 g Natriumacetat zu und erhöhte die Temperatur innerhalb 3 Stunden auf 50 °C und hielt den pH-Wert bei 7,0. Man lieB 250 g HCl (konz.) zulaufen und erhöhte die Temperatur auf 90 - 95 °C. Nach 4 Stunden wurde abgekühlt, der resultierende Niederschlag abgesaugt und neutral gewaschen. Ohne Trocknung wurde der Niederschlag in 750 ml Wasser angerührt und bei pH 7,0 und 0 - 5 °C mit 19.4 g Cyanurchlorid versetzt. Der pH-Wert wurde durch Zugabe von Sodalösung bei 6,5 - 7.0 gehalten. Nach 3 Stunden gab man 49 g 4-(2'-Sulfatoethylsulfonyl)-5-(2''-sulfatoethylsulfonylmethyl)-anilin zu und erhöhte die Temperatur auf 35 -40 °C. Die Farbstofflösung wurde sprühgetrocknet. Man isolierte 260 g des Farbstoffs der Formel

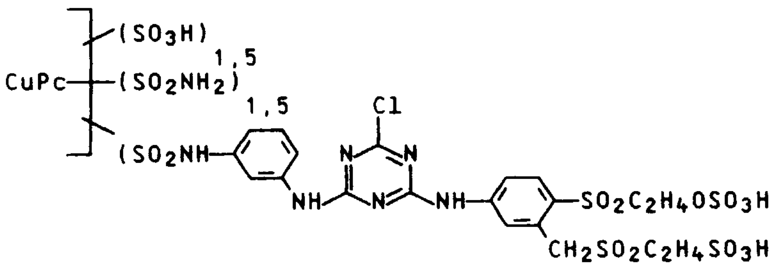

der Baumwolle in türkisen Farbtönen färbt.

Beispiel 260

350 g 5-Nitro-2-chlor-benzylchlorid, 136 g 2-Mercaptoethanol und 234,5 g Kaliumcarbonat wurden in 1700 ml Wasser 4 Stunden bei 40 °C und 1 Stunde bei 60 °C gerührt. Die Reaktionslösung wurde mit Essigsäure auf pH 6 gestellt und nach Zugabe von 10 g Wolframsäure mit 2060 g 15 %iger Wasserstoffperoxidlösung bei 55 °C versetzt. Nach Beendigung der exothermen Reaktion wurde 3 Stunden bei 85 °C und 12 Stunden bei 20 °C nachgerührt. Das ausgefallene Produkt wurde isoliert, mit Wasser gewaschen und unter vermindertem Druck bei 50 °C getrocknet. Man erhielt 395 g analysenreines Produkt folgender Konstitution:

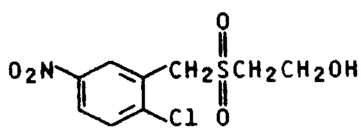

Analyse ber.: Chlor kovalent 12,7 %
gef.: 12,4 %

Schmp.: 127 - 128 °C

Wurde statt 2-Mercaptoethanol die gleiche molare Menge an 1-Mercaptopropan-2-ol eingesetzt, so wurde analysenreines Produkt der Formel (260a)

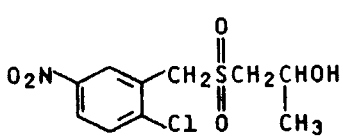

Schmp.: 121 - 122 °C

erhalten.

In analoger Weise wurden folgende Zwischenprodukte hergestellt:

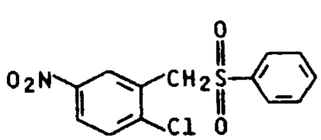

(260b) ,

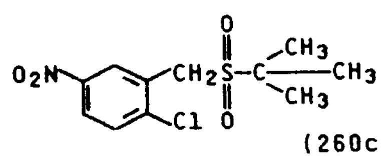

(260c)

Beispiel 261

29,8 g des in Bsp. 260 erhaltenen 4-Nitro-1-chlor-2-benzyl-β-hydroxyethylsulfons und 3,3 g Paraformaldehyd wurden in 100 ml Methanol auf 60 °C erhitzt. In 0,5 Stunden wurden bei dieser Temperatur 19,8 g einer 30 %igen Natriummethylatlösung zugetropft. Nach 3 Stunden Rühren bei 60 bis 65 °C wurde auf 20 bis 25 °C gekühlt, das ausgefallene Produkt isoliert, mit Wasser gewaschen und getrocknet. Es wurden 20 g analysenreines Produkt der Formel

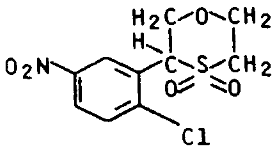

Analyse ber.: Chlor kovalent 12,2 %
gef.: 12,1 %

Schmp.: 224 °C

Beispiel 262

59,5 g des in Bsp. 260 erhaltenen 4-Nitro-1-chlor-2-benzyl-$\beta$-hydroxyethylsulfons wurden in 100 ml N-Methylpyrrolidinon gelöst. In die auf 0 °C gekühlte Lösung wurde eine frisch hergestellte Lösung aus 17,2 g Thioethanol, 12,3 g Kaliumhydroxid und 100 ml N-Methylpyrrolidinon getropft. Nach 12 Stunden Rühren bei 20 bis 25 °C wurde das Lösungsmittel unter vermindertem Druck abdestilliert, der Rückstand mit 200 ml Wasser versetzt und mit Essigsäure auf pH 6 gestellt. Nach 2 Stunden Rühren bei 0 bis 5 °C wurde das ausgefallene Produkt isoliert, mit Wasser gewaschen und getrocknet. Man erhielt 60 g eines Produktes, das überwiegend der Formel

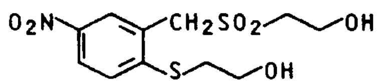

Schmp.: 126-128 °C

entsprach.

Folgende Verbindungen werden analog Bsp. 262 aus den in Bsp. 260 und 261 beschriebenen Zwischenprodukten erhalten.

Tabelle 14

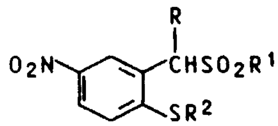

| Bsp.-Nr. | R | $R^1$ | $R^2$ |
|---|---|---|---|
| 263 | H | $-CH_2CH_2OH$ | $-CH_2CH_2OH$ |
| 264 | H | $-CH_2CH_2OH$ | $-CH_2CH(CH_3)OH$ |
| 265 | H | $-CH_2CH_2OH$ | $-C(CH_3)_3$ |
| 266 | H | $-CH_2CH_2OH$ | $-Ph$ |
| 267 | H | $-CH_2CH(CH_3)OH$ | $-CH_2CH_2OH$ |
| 268 | H | $-CH_2CH(CH_3)OH$ | $-CH_2CH(CH_3)OH$ |
| 269 | H | $-CH_2CH(CH_3)OH$ | $-C(CH_3)_3$ |
| 270 | H | $-CH_2CH(CH_3)OH$ | $-Ph$ |
| 271 | H | $-C(CH_3)_3$ | $-CH_2CH_2OH$ |
| 272 | H | $-C(CH_3)_3$ | $-CH_2CH(CH_3)OH$ |
| 273 | H | $-Ph$ | $-CH_2CH_2OH$ |
| 274 | H | $-Ph$ | $-CH_2CH(CH_3)OH$ |
| 275 | $-CH_2O-CH_2-CH_2-$ | | $-CH_2CH_2OH$ |
| 276 | $-CH_2O-CH_2-CH_2-$ | | $-CH_2CH(CH_3)OH$ |

Beispiel 277

32,1 g der in Bsp. 262 beschriebenen Verbindung der Formel

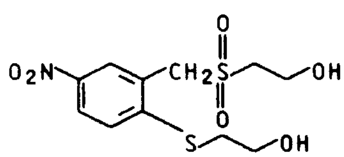

wurden in 200 ml Wasser bei 40 °C gelöst und nach Zugabe von 0,5 g Wolframsäure und molaren Mengen an Wasserstoffperoxid oxidiert. Nach 2 Stunden Rühren bei 40 °C wurde auf 0 bis 5 °C abgekühlt, das ausgefallene Produkt isoliert, mit Wasser gewaschen und unter vermindertem Druck bei 40 °C getrocknet. Es wurden 30,2 g eines Produktes erhalten, das überwiegend der Formel

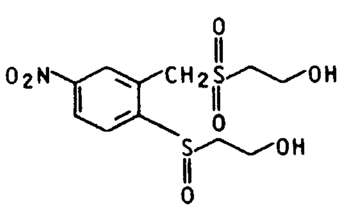

Schmp.: 58 - 61 °C

entsprach.

In analoger Weise werden die in Tabelle 15 beschriebenen Verbindungen erhalten.

Tabelle 15

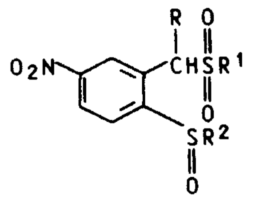

| Bsp.-Nr. | R | $R^1$ | $R^2$ |
|---|---|---|---|
| 278 | H | $-CH_2CH_2OH$ | $-CH_2CH_2OH$ |
| 279 | H | $-CH_2CH_2OH$ | $-CH_2CH(CH_3)OH$ |
| 280 | H | $-CH_2CH_2OH$ | $-C(CH_3)_3$ |
| 281 | H | $-CH_2CH_2OH$ | -Ph |
| 282 | H | $-CH_2CH(CH_3)OH$ | $-CH_2CH_2OH$ |
| 283 | H | $-CH_2CH(CH_3)OH$ | $-CH_2CH(CH_3)OH$ |
| 284 | H | $-CH_2CH(CH_3)OH$ | $-C(CH_3)_3$ |
| 285 | H | $-CH_2CH(CH_3)OH$ | -Ph |
| 286 | H | $-C(CH_3)_3$ | $-CH_2CH_2OH$ |
| 287 | H | $-C(CH_3)_3$ | $-CH_2CH(CH_3)OH$ |
| 288 | H | -Ph | $-CH_2CH_2OH$ |
| 289 | H | -Ph | $-CH_2CH(CH_3)OH$ |
| 290 | $-CH_2O-CH_2-CH_2-$ | | $-CH_2CH_2OH$ |
| 291 | $-CH_2O-CH_2-CH_2-$ | | $-CH_2CH(CH_3)OH$ |

Beispiel 292

59.5 g des in Bsp. 260 erhaltenen 4-Nitro-1-chlor-2-benzyl-β-hydroxyethylsulfons wurden in 100 ml N-Methylpyrrolidinon gelöst. In die auf 0 °C gekühlte Lösung wurde eine frisch hergestellte Lösung aus 17,2 g Thioethanol, 12,3 g Kaliumhydroxid und 100 ml N-Methylpyrrolidinon zugetropft. Nach 12 Stunden Rühren bei 20 bis 25 °C wurde das Lösungsmittel unter vermindertem Druck abdestilliert, der Rückstand mit 200 ml Wasser versetzt und auf 40 °C erhitzt. Die Lösung wurde mit Essigsäure auf pH = 6 gestellt und nach Zugabe von 0,8 g Wolframsäure mit der molaren Menge an Wasserstoffperoxid oxidiert. Nach 2 Stunden Rühren bei 40 °C wurde auf 0 bis 5 °C abgekühlt, das ausgefallene Produkt isoliert, mit Wasser gewaschen und unter vermindertem Druck bei 40 °C getrocknet. Es wurden 60,9 g eines Produktes erhalten, das der in Bsp. 277 beschriebene Verbindung der Formel

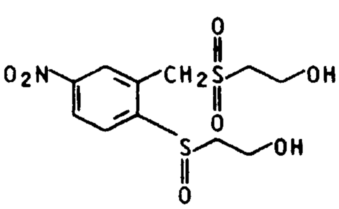

Schmp.: 58 - 61 °C

entsprach.
Die in Tabelle 15 beschriebenen Verbindungen können auch nach der oben beschriebenen Methode erhalten werden.

Beispiel 293

963 g des in Bsp. 260 erhaltenen 4-Nitro-1-chlor-2-benzyl-ß-hydroxyethylsulfons wurden in 1500 ml N-Methylpyrrolidinon gelöst. In die auf 0 °C gekühlte Lösung wurde eine frisch hergestellte Lösung aus 297 g Thioethanol, 212 g Kaliumhydroxid und 1500 ml N-Methylpyrrolidinon in 3 Stunden zugetropft. Nach 12 Stunden Rühren bei 20 bis 25 °C wurde das Lösungsmittel unter vermindertem Druck abdestilliert, der Rückstand mit 3000 ml Wasser versetzt, auf 40 °C erhitzt und nach Zugabe von 10 g Wolframsäure mit 2100 g einer 30 %igen Wasserstoffperoxidlösung oxidiert. Nach Beendigung der exothermen Reaktion wurde 2 Stunden bei 60 °C, 2 Stunden bei 80 °C und 1 Stunde bei 95 °C nachgerührt und auf 20 bis 25 °C abgekühlt. Das ausgefallene Produkt wurde isoliert, mit Wasser gewaschen und unter vermindertem Druck bei 40 °C getrocknet. Es wurden 1005 g eines Produktes erhalten, das der Formel

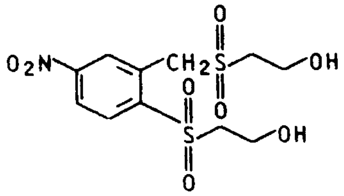

Schmp.: 139 - 142 °C

entsprach.

Die in Bsp. 260 und 261 beschriebenen Zwischenprodukte können analog Bsp. 293 zu den in Tabelle 16 aufgeführten Verbindungen umgesetzt werden.

Tabelle 16

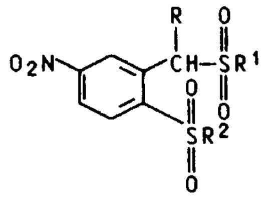

| Bsp.-Nr. | R | $R^1$ | $R^2$ |
|---|---|---|---|
| 294 | H | $-CH_2CH_2OH$ | $-CH_2CH(CH_3)OH$ |
| 295 | H | $-CH_2CH_2OH$ | $-C(CH_3)_3$ |
| 296 | H | $-CH_2CH_2OH$ | $-Ph$ |
| 297 | H | $-CH_2CH(CH_3)OH$ | $-CH_2CH_2OH$ |
| 298 | H | $-CH_2CH(CH_3)OH$ | $-CH_2CH(CH_3)OH$ |
| 299 | H | $-CH_2CH(CH_3)OH$ | $-C(CH_3)_3$ |
| 300 | H | $-CH_2CH(CH_3)OH$ | $-Ph$ |
| 301 | H | $-C(CH_3)_3$ | $-CH_2CH_2OH$ |
| 302 | H | $-C(CH_3)_3$ | $-CH_2CH(CH_3)OH$ |
| 303 | H | $-Ph$ | $-CH_2CH_2OH$ |
| 304 | H | $-Ph$ | $-CH_2CH(CH_3)-OH$ |
| 305 | $-CH_2O-CH_2-CH_2-$ | | $-CH_2CH_2OH$ |
| 306 | $-CH_2O-CH_2-CH_2-$ | | $-CH_2CH(CH_3)-OH$ |

Beispiel 307

32,1 g der in Bsp. 262 beschriebenen Verbindung wurden in 100 ml Wasser bei 40 °C angelöst und nach Zugabe von 0,5 g Wolframsäure mit 70 g 30 %iger Wasserstoffperoxidlösung oxidiert. Nach Beendigung der exothermen Reaktion wurde 2 Stunden bei 60 °C und 2 Stunden bei 80 °C nachgerührt. Nach Abkühlen auf 20 bis 25 °C wurde das ausgefallene Produkt isoliert, mit Wasser gewaschen und unter vermindertem Druck bei 40 °C getrocknet. Es wurden 34 g eines Produktes erhalten, das der in Bsp. 293 beschriebenen Verbindung der Formel

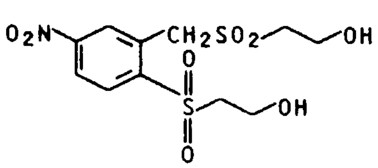

Schmp.: 139 - 142 °C

entsprach

Die in Tabelle 14 beschriebenen Verbindungen können analog Beispiel 307 zu den in Tabelle 15 aufgeführten Verbindungen umgesetzt werden.

Beispiel 308

50 g der in Bsp. 307 beschriebenen Verbindung, 142 ml Thionylchlorid, 500 ml Toluol und 2 ml Dimethylformamid wurden 1 Stunde bei 65 °C und 2 Stunden bei 85 °C gerührt. Nach Abkühlen auf 5 bis

10 °C wurde ausgefallenes Produkt isoliert, mit Wasser gewaschen und unter vermindertem Druck bei 60 °C getrocknet. Es wurden 54 g eines Produktes erhalten, das der Formel

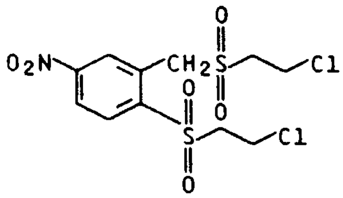

Schmp.: 158 °C

entsprach.

Die in den Tabellen 14, 15 und 16 aufgeführten Produkte können analog Bsp. 308 zu Verbindungen der Formel

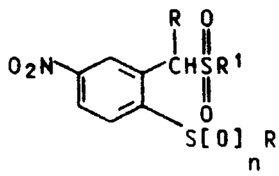

umgesetzt werden.

Tabelle 17

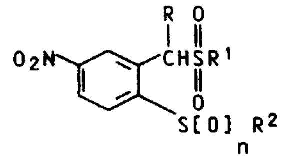

| Bsp.-Nr. | R | $R^1$ | $R^2$ | n |
|---|---|---|---|---|
| 309 | H | $-CH_2CH_2Cl$ | $-CH_2CH_2Cl$ | 0 |
| 310 | H | $-CH_2CH_2Cl$ | $-CH_2CH_2Cl$ | 1 |
| 311 | H | $-CH_2CH_2Cl$ | $-CH_2CH(CH_3)Cl$ | 0 |
| 312 | H | $-CH_2CH_2Cl$ | $-CH_2CH(CH_3)Cl$ | 1 |
| 313 | H | $-CH_2CH_2Cl$ | $-CH_2CH(CH_3)Cl$ | 2 |
| 314 | H | $-CH_2CH_2Cl$ | -Ph | 0 |
| 315 | H | $-CH_2CH_2Cl$ | -Ph | 1 |
| 316 | H | $-CH_2CH_2Cl$ | -Ph | 2 |
| 317 | H | $-CH_2CH_2Cl$ | $-C(CH_3)_3$ | 0 |
| 318 | H | $-CH_2CH_2Cl$ | $-C(CH_3)_3$ | 1 |
| 319 | H | $-CH_2CH_2Cl$ | $-C(CH_3)_3$ | 2 |
| 320 | H | $-CH_2CH(CH_3)Cl$ | $-CH_2CH_2Cl$ | 0 |
| 321 | H | $-CH_2CH(CH_3)Cl$ | $-CH_2CH_2Cl$ | 1 |
| 322 | H | $-CH_2CH(CH_3)Cl$ | $-CH_2CH_2Cl$ | 2 |
| 323 | H | $-CH_2CH(CH_3)Cl$ | $-CH_2CH(CH_3)Cl$ | 0 |
| 324 | H | $-CH_2CH(CH_3)Cl$ | $-CH_2CH(CH_3)Cl$ | 1 |
| 325 | H | $-CH_2CH(CH_3)Cl$ | $-CH_2CH(CH_3)Cl$ | 2 |
| 326 | H | $-CH_2CH(CH_3)Cl$ | -Ph | 0 |
| 327 | H | $-CH_2CH(CH_3)Cl$ | -Ph | 1 |
| 328 | H | $-CH_2CH(CH_3)Cl$ | -Ph | 2 |
| 329 | H | $-CH_2CH(CH_3)Cl$ | $-C(CH_3)_3$ | 0 |
| 330 | H | $-CH_2CH(CH_3)Cl$ | $-C(CH_3)_3$ | 1 |
| 331 | H | $-CH_2CH(CH_3)Cl$ | $-C(CH_3)_3$ | 2 |
| 332 | H | $-C(CH_3)_3$ | $-CH_2CH_2Cl$ | 0 |
| 333 | H | $-C(CH_3)_3$ | $-CH_2CH_2Cl$ | 1 |
| 334 | H | $-C(CH_3)_3$ | $-CH_2CH_2Cl$ | 2 |
| 335 | H | $-C(CH_3)_3$ | $-CH_2CH(CH_3)Cl$ | 0 |
| 336 | H | $-C(CH_3)_3$ | $-CH_2CH(CH_3)Cl$ | 1 |
| 337 | H | $-C(CH_3)_3$ | $-CH_2CH(CH_3)Cl$ | 2 |

Fortsetzung - Tabelle 17

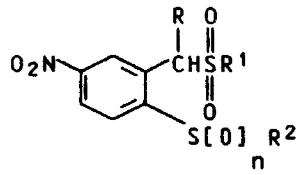

| Bsp.-Nr. | R | $R^1$ | $R^2$ | n |
|---|---|---|---|---|
| 338 | H | $-Ph$ | $-CH_2CH_2Cl$ | 0 |
| 339 | H | $-Ph$ | $-CH_2CH_2Cl$ | 1 |
| 340 | H | $-Ph$ | $-CH_2CH_2Cl$ | 2 |
| 341 | H | $-Ph$ | $-CH_2CH(CH_3)Cl$ | 0 |
| 342 | H | $-Ph$ | $-CH_2CH(CH_3)Cl$ | 1 |
| 343 | H | $-Ph$ | $-CH_2CH(CH_3)Cl$ | 2 |
| 344 | | $-CH_2O-CH_2CH_2-$ | $-CH_2CH_2Cl$ | 0 |
| 345 | | $-CH_2O-CH_2CH_2-$ | $-CH_2CH_2Cl$ | 1 |
| 346 | | $-CH_2O-CH_2CH_2-$ | $-CH_2CH_2Cl$ | 2 |
| 347 | | $-CH_2O-CH_2CH_2-$ | $-CH_2CH(CH_3)Cl$ | 0 |
| 348 | | $-CH_2O-CH_2CH_2-$ | $-CH_2CH(CH_3)Cl$ | 1 |
| 349 | | $-CH_2O-CH_2CH_2-$ | $-CH_2CH(CH_3)Cl$ | 2 |

Beispiel 350

160 g der in Bsp. 293 beschriebenen Verbindung wurden in 1000 ml Methanol mit Raney-Nickel katalytisch bei 25 bis 30 °C reduziert. Nach Beendigung der Reduktion wurde vom Raney-Nickel abgetrennt und Methanol abdestilliert. Man erhielt das Amin der Formel

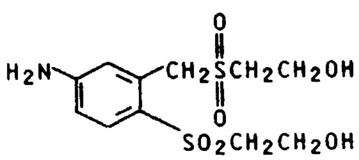

Schmp.: 112 - 113 °C

als Öl, das innerhalb einer Stunde bei 0 bis 5 °C kristallisierte.

In analoger Weise werden die folgenden Amine mit der Formel

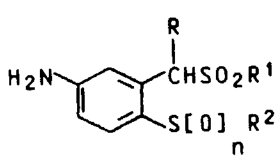

erhalten.

Tabelle 18

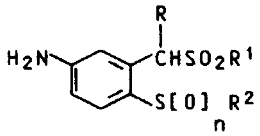

| Bsp.-Nr. | R | $R^1$ | $R^2$ | n |
|---|---|---|---|---|
| 351 | H | $-CH_2CH_2OH$ | $-CH_2CH_2OH$ | 0 |
| 352 | H | $-CH_2CH_2OH$ | $-CH_2CH_2OH$ | 1 |
| 353 | H | $-CH_2CH_2OH$ | $-CH_2CH(CH_3)OH$ | 0 |
| 354 | H | $-CH_2CH_2OH$ | $-CH_2CH(CH_3)OH$ | 1 |
| 355 | H | $-CH_2CH_2OH$ | $-CH_2CH(CH_3)OH$ | 2 |
| 356 | H | $-CH_2CH_2OH$ | -Ph | 0 |
| 357 | H | $-CH_2CH_2OH$ | -Ph | 1 |
| 358 | H | $-CH_2CH_2OH$ | -Ph | 2 |
| 359 | H | $-CH_2CH_2OH$ | $-C(CH_3)_3$ | 0 |
| 360 | H | $-CH_2CH_2OH$ | $-C(CH_3)_3$ | 1 |
| 361 | H | $-CH_2CH_2OH$ | $-C(CH_3)_3$ | 2 |
| 362 | H | $-CH_2CH(CH_3)OH$ | $-CH_2CH_2OH$ | 0 |
| 363 | H | $-CH_2CH(CH_3)OH$ | $-CH_2CH_2OH$ | 1 |
| 364 | H | $-CH_2CH(CH_3)OH$ | $-CH_2CH_2OH$ | 2 |
| 365 | H | $-CH_2CH(CH_3)OH$ | $-CH_2CH(CH_3)OH$ | 0 |
| 366 | H | $-CH_2CH(CH_3)OH$ | $-CH_2CH(CH_3)OH$ | 1 |
| 367 | H | $-CH_2CH(CH_3)OH$ | $-CH_2CH(CH_3)OH$ | 2 |
| 368 | H | $-CH_2CH(CH_3)OH$ | -Ph | 0 |
| 369 | H | $-CH_2CH(CH_3)OH$ | -Ph | 1 |
| 370 | H | $-CH_2CH(CH_3)OH$ | -Ph | 2 |
| 371 | H | $-CH_2CH(CH_3)OH$ | $-C(CH_3)_3$ | 0 |
| 372 | H | $-CH_2CH(CH_3)OH$ | $-C(CH_3)_3$ | 1 |
| 373 | H | $-CH_2CH(CH_3)OH$ | $-C(CH_3)_3$ | 2 |
| 374 | H | $-C(CH_3)_3$ | $-CH_2CH_2OH$ | 0 |
| 375 | H | $-C(CH_3)_3$ | $-CH_2CH_2OH$ | 1 |
| 376 | H | $-C(CH_3)_3$ | $-CH_2CH_2OH$ | 2 |
| 377 | H | $-C(CH_3)_3$ | $-CH_2CH(CH_3)OH$ | 0 |
| 378 | H | $-C(CH_3)_3$ | $-CH_2CH(CH_3)OH$ | 1 |
| 379 | H | $-C(CH_3)_3$ | $-CH_2CH(CH_3)OH$ | 2 |

Fortsetzung - Tabelle 18

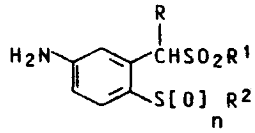

| Bsp.-Nr. | R | $R^1$ | $R^2$ | n |
|---|---|---|---|---|
| 380 | H | $-Ph$ | $-CH_2CH_2OH$ | 0 |
| 381 | H | $-Ph$ | $-CH_2CH_2OH$ | 1 |
| 382 | H | $-Ph$ | $-CH_2CH_2OH$ | 2 |
| 383 | H | $-Ph$ | $-CH_2CH(CH_3)OH$ | 0 |
| 384 | H | $-Ph$ | $-CH_2CH(CH_3)OH$ | 1 |
| 385 | H | $-Ph$ | $-CH_2CH(CH_3)OH$ | 2 |
| 386 | H | $-CH_2CH_2Cl$ | $-CH_2CH_2Cl$ | 0 |
| 387 | H | $-CH_2CH_2Cl$ | $-CH_2CH_2Cl$ | 1 |
| 388 | H | $-CH_2CH_2Cl$ | $-CH_2CH_2Cl$ | 2 |
| 389 | H | $-CH_2CH_2Cl$ | $-CH_2CH(CH_3)Cl$ | 0 |
| 390 | H | $-CH_2CH_2Cl$ | $-CH_2CH(CH_3)Cl$ | 1 |
| 391 | H | $-CH_2CH_2Cl$ | $-CH_2CH(CH_3)Cl$ | 2 |
| 392 | H | $-CH_2CH_2Cl$ | $-Ph$ | 0 |
| 393 | H | $-CH_2CH_2Cl$ | $-Ph$ | 1 |
| 394 | H | $-CH_2CH_2Cl$ | $-Ph$ | 2 |
| 395 | H | $-CH_2CH_2Cl$ | $-C(CH_3)_3$ | 0 |
| 396 | H | $-CH_2CH_2Cl$ | $-C(CH_3)_3$ | 1 |
| 397 | H | $-CH_2CH_2Cl$ | $-C(CH_3)_3$ | 2 |
| 398 | H | $-CH_2CH(CH_3)Cl$ | $-CH_2CH_2Cl$ | 0 |
| 399 | H | $-CH_2CH(CH_3)Cl$ | $-CH_2CH_2Cl$ | 1 |
| 400 | H | $-CH_2CH(CH_3)Cl$ | $-CH_2CH_2Cl$ | 2 |
| 401 | H | $-CH_2CH(CH_3)Cl$ | $-CH_2CH(CH_3)Cl$ | 0 |
| 402 | H | $-CH_2CH(CH_3)Cl$ | $-CH_2CH(CH_3)Cl$ | 1 |
| 403 | H | $-CH_2CH(CH_3)Cl$ | $-CH_2CH(CH_3)Cl$ | 2 |
| 404 | H | $-CH_2CH(CH_3)Cl$ | $-Ph$ | 0 |
| 405 | H | $-CH_2CH(CH_3)Cl$ | $-Ph$ | 1 |
| 406 | H | $-CH_2CH(CH_3)Cl$ | $-Ph$ | 2 |
| 407 | H | $-CH_2CH(CH_3)Cl$ | $-C(CH_3)_3$ | 0 |
| 408 | H | $-CH_2CH(CH_3)Cl$ | $-C(CH_3)_3$ | 1 |
| 409 | H | $-CH_2CH(CH_3)Cl$ | $-C(CH_3)_3$ | 2 |

Fortsetzung - Tabelle 18

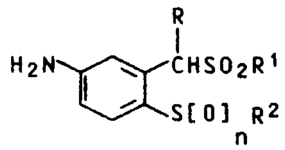

| Bsp.-Nr. | R | $R^1$ | $R^2$ | n |
|---|---|---|---|---|
| 410 | H | $-C(CH_3)_3$ | $-CH_2CH_2Cl$ | 0 |
| 411 | H | $-C(CH_3)_3$ | $-CH_2CH_2Cl$ | 1 |
| 412 | H | $-C(CH_3)_3$ | $-CH_2CH_2Cl$ | 2 |
| 413 | H | $-C(CH_3)_3$ | $-CH_2CH(CH_3)Cl$ | 0 |
| 414 | H | $-C(CH_3)_3$ | $-CH_2CH(CH_3)Cl$ | 1 |
| 415 | H | $-C(CH_3)_3$ | $-CH_2CH(CH_3)Cl$ | 2 |
| 416 | H | -Ph | $-CH_2CH_2Cl$ | 0 |
| 417 | H | -Ph | $-CH_2CH_2Cl$ | 1 |
| 418 | H | -Ph | $-CH_2CH_2Cl$ | 2 |
| 419 | H | -Ph | $-CH_2CH(CH_3)Cl$ | 0 |
| 420 | H | -Ph | $-CH_2CH(CH_3)Cl$ | 1 |
| 421 | H | -Ph | $-CH_2CH(CH_3)Cl$ | 2 |
| 422 | | $-CH_2O-CH_2CH_2-$ | $-CH_2CH_2OH$ | 0 |
| 423 | | $-CH_2O-CH_2CH_2-$ | $-CH_2CH_2OH$ | 1 |
| 424 | | $-CH_2O-CH_2CH_2-$ | $-CH_2CH_2OH$ | 2 |
| 425 | | $-CH_2O-CH_2CH_2-$ | $-CH_2CH(CH_3)OH$ | 0 |
| 426 | | $-CH_2O-CH_2CH_2-$ | $-CH_2CH(CH_3)OH$ | 1 |
| 427 | | $-CH_2O-CH_2CH_2-$ | $-CH_2CH(CH_3)OH$ | 2 |
| 428 | | $-CH_2O-CH_2CH_2-$ | $-CH_2CH_2Cl$ | 0 |
| 429 | | $-CH_2O-CH_2CH_2-$ | $-CH_2CH_2Cl$ | 1 |
| 430 | | $-CH_2O-CH_2CH_2-$ | $-CH_2CH_2Cl$ | 2 |
| 431 | | $-CH_2O-CH_2CH_2-$ | $-CH_2CH(CH_3)Cl$ | 0 |
| 432 | | $-CH_2O-CH_2CH_2-$ | $-CH_2CH(CH_3)Cl$ | 1 |
| 433 | | $-CH_2O-CH_2CH_2-$ | $-CH_2CH(CH_3)Cl$ | 2 |

Beispiel 434

90 g der Verbindung der Formel

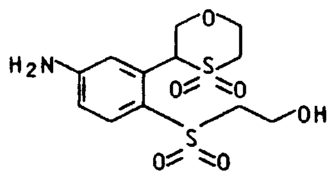

wurden bei 0 bis 10 °C in 360 g Chlorsulfonsäure eingetragen und 5 Stunden bei 20 bis 25 °C gerührt. Nach Austragen auf 900 ml Eiswasser erfolgte spontan die Kristallisation. Die Fällung wurde filtriert, mit Aceton schwefelsäurefrei gewaschen und getrocknet. Die Ausbeute war nahezu quantitativ. Das Produkt entsprach der Formel

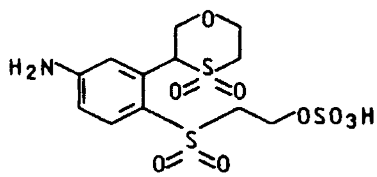

In analoger Weise werden die in Tabelle 19 aufgeführten Verbindungen erhalten.

Tabelle 19

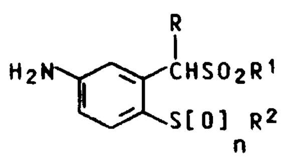

| Bsp.-Nr. | R | $R^1$ | $R^2$ | n |
|---|---|---|---|---|
| 435 | H | $-CH_2CH_2OSO_3H$ | $-C(CH_3)_3$ | 0 |
| 436 | H | $-CH_2CH_2OSO_3H$ | $-C(CH_3)_3$ | 1 |
| 437 | H | $-CH_2CH_2OSO_3H$ | $-C(CH_3)_3$ | 2 |
| 438 | H | $-CH_2CH_2OSO_3H$ | -Ph | 0 |
| 439 | H | $-CH_2CH_2OSO_3H$ | -Ph | 1 |
| 440 | H | $-CH_2CH_2OSO_3H$ | -Ph | 2 |

Fortsetzung - Tabelle 19

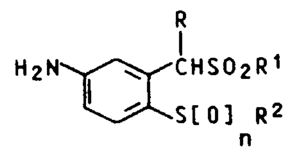

| Bsp.-Nr. | R | $R^1$ | $R^2$ | n |
|---|---|---|---|---|
| 441 | H | $-CH_2CH(CH_3)OSO_3H$ | $-C(CH_3)_3$ | 0 |
| 442 | H | $-CH_2CH(CH_3)OSO_3H$ | $-C(CH_3)_3$ | 1 |
| 443 | H | $-CH_2CH(CH_3)OSO_3H$ | $-C(CH_3)_3$ | 2 |
| 444 | H | $-CH_2CH(CH_3)OSO_3H$ | $-Ph$ | 0 |
| 445 | H | $-CH_2CH(CH_3)OSO_3H$ | $-Ph$ | 1 |
| 446 | H | $-CH_2CH(CH_3)OSO_3H$ | $-Ph$ | 2 |
| 447 | H | $-C(CH_3)_3$ | $-CH_2CH_2OSO_3H$ | 0 |
| 448 | H | $-C(CH_3)_3$ | $-CH_2CH_2OSO_3H$ | 1 |
| 449 | H | $-C(CH_3)_3$ | $-CH_2CH_2OSO_3H$ | 2 |
| 450 | H | $-C(CH_3)_3$ | $-CH_2CH(CH_3)OSO_3H$ | 0 |
| 451 | H | $-C(CH_3)_3$ | $-CH_2CH(CH_3)OSO_3H$ | 1 |
| 452 | H | $-C(CH_3)_3$ | $-CH_2CH(CH_3)OSO_3H$ | 2 |
| 453 | H | $-Ph$ | $-CH_2CH_2OSO_3H$ | 0 |
| 454 | H | $-Ph$ | $-CH_2CH_2OSO_3H$ | 1 |
| 455 | H | $-Ph$ | $-CH_2CH_2OSO_3H$ | 2 |
| 456 | H | $-Ph$ | $-CH_2CH(CH_3)OSO_3H$ | 0 |
| 457 | H | $-Ph$ | $-CH_2CH(CH_3)OSO_3H$ | 1 |
| 458 | H | $-Ph$ | $-CH_2CH(CH_3)OSO_3H$ | 2 |
| 459 | $-CH_2O-CH_2CH_2-$ | | $-CH_2CH(CH_3)OSO_3H$ | 0 |
| 460 | $-CH_2O-CH_2CH_2-$ | | $-CH_2CH(CH_3)OSO_3H$ | 1 |
| 461 | $-CH_2O-CH_2CH_2-$ | | $-CH_2CH(CH_3)OSO_3H$ | 2 |
| 462 | $-CH_2OCH_2CH_2-$ | | $-CH_2CH_2OSO_3H$ | 0 |
| 463 | $-CH_2OCH_2CH_2-$ | | $-CH_2CH_2OSO_3H$ | 1 |

Beispiel 464

32 g der in Bsp. 350 beschriebenen Verbindung der Formel

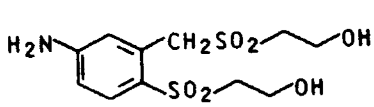

wurden bei 0 bis 10 °C in 120 g Chlorsulfonsäure eingetragen und 2 Stunden bei 20 bis 25 °C gerührt. Nach Austragen auf 300 g Eiswasser blieb das Produkt der Formel

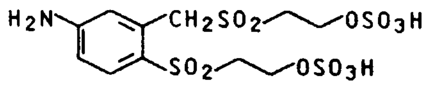

in Lösung. Wurde der Schwefelsäureester für Diazotierungsreaktionen eingesetzt, so erfolgte die Diazotierung aus schwefelsaurer Lösung. Zur Kondensation mit heterocyclischen Ankerkomponenten wurde die schwefelsaure Lösung mit Natriumhydrogencarbonat neutralisiert und als Lösung weiter umgesetzt.

In analoger Weise werden folgende Verbindungen der allgemeinen Formel

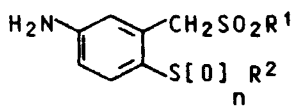

erhalten.

Tabelle 20

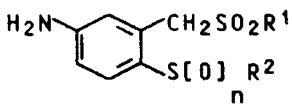

| Bsp.-Nr. | $R^1$ | $R^2$ | n |
|---|---|---|---|
| 465 | $-CH_2CH_2OSO_3H$ | $-CH_2CH_2OSO_3H$ | 0 |
| 466 | $-CH_2CH_2OSO_3H$ | $-CH_2CH_2OSO_3H$ | 1 |
| 467 | $-CH_2CH_2OSO_3H$ | $-CH_2CH(CH_3)OSO_3H$ | 0 |
| 468 | $-CH_2CH_2OSO_3H$ | $-CH_2CH(CH_3)OSO_3H$ | 1 |
| 469 | $-CH_2CH_2OSO_3H$ | $-CH_2CH(CH_3)OSO_3H$ | 2 |
| 470 | $-CH_2CH(CH_3)OSO_3H$ | $-CH_2CH(CH_3)OSO_3H$ | 0 |
| 471 | $-CH_2CH(CH_3)OSO_3H$ | $-CH_2CH(CH_3)OSO_3H$ | 1 |
| 472 | $-CH_2CH(CH_3)OSO_3H$ | $-CH_2CH(CH_3)OSO_3H$ | 2 |
| 473 | $-CH_2CH(CH_3)OSO_3H$ | $-CH_2CH_2OSO_3H$ | 0 |
| 474 | $-CH_2CH(CH_3)OSO_3H$ | $-CH_2CH_2OSO_3H$ | 1 |
| 475 | $-CH_2CH(CH_3)OSO_3H$ | $-CH_2CH_2OSO_3H$ | 2 |

## Patentansprüche

1. Doppelankerreaktivfarbstoffe der Formel I

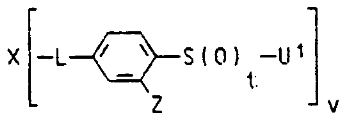

(I),

in der

U[1] $C_1$-$C_4$-Alkyl, Phenyl, $C_2$-$C_{10}$-Alkenyl oder den Rest

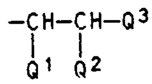

,

wobei $Q^1$ und $Q^2$ gleich oder verschieden sind und unabhängig voneinander jeweils für Wasserstoff oder $C_1$-$C_4$-Alkyl und $Q^3$ für eine unter alkalischen Reaktionsbedingungen abspaltbare Gruppe stehen,

Z den Rest

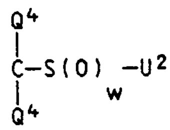

,

oder

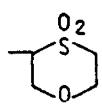

wobei

$Q^4$ unabhängig voneinander für Wasserstoff oder $C_1$-$C_4$-Alkyl, das gegebenenfalls durch Cyano substituiert ist, $U^2$ für $C_1$-$C_4$-Alkyl, Phen, $C_2$-$C_{10}$-Alkenyl oder den Rest

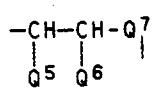

,

in dem $Q^5$ und $Q^6$ gleich oder verschieden sind und unabhängig voneinander jeweils die Bedeutung von Wasserstoff oder $C_1$-$C_4$-Alkyl und $Q^7$ die Bedeutung einer unter alkalischen Reaktionsbedingungen abspaltbaren Gruppe besitzen und w für 0, 1 oder 2 stehen,

t 0, 1 oder 2,

v 1 oder 2,

X a) den Rest eines Chromophors, der gegebenenfalls eine weitere reaktive Gruppe aufweist und der sich von einem Mono- oder Disazofarbstoff, einem Triphendioxazin, einem Anthrachinon, einem Kupfer-Formazan oder einem metallisierten Phthalocyanin ableitet, oder

b) den Rest einer Kupplungskomponente, an den gegebenenfalls zusätzlich der Rest einer Diazokomponente über eine Azobrücke gebunden ist und der gegebenenfalls eine zusätzlich reaktive Gruppe aufweist, und

L im Fall a) ein Brückenglied der Formel

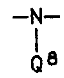

oder

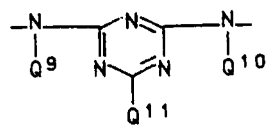,

wobei $Q^8$ für Wasserstoff oder $C_1$-$C_4$-Alkyl, $Q^9$ und $Q^{10}$ gleich oder verschieden sind und unabhängig voneinander jeweils für Wasserstoff oder $C_1$-$C_4$-Alkyl und $Q^{11}$ für Fluor, Chlor oder Brom stehen, oder
im Fall b) eine Azobrücke bedeuten,
mit der Maßgabe, dass mindestens einer der beiden Reste $S(O)_t$-$U^1$ und Z eine faserreaktive Gruppe darstellt.

**2.** Doppelankerreaktivfarbstoffe gemäß Anspruch 1, die der Formel Ia

$X[-L-E^1]_v$ (Ia)

entsprechen, in der X, L und v jeweils die in Anspruch 1 genannte Bedeutung besitzen und $E^1$ für einen Rest der Formel IIa

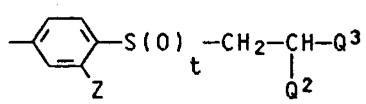 (IIa)

steht, in der

$Q^2$ Wasserstoff oder Methyl,
$Q^3$ $OSO_3H$,
Z den Rest

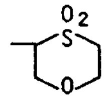

oder

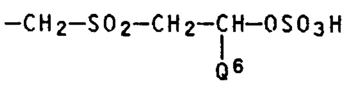,

wobei $Q^6$ für Wasserstoff oder Methyl stehen, und
t 0 oder 2 bedeuten.

**3.** Benzylverbindungen der Formel III

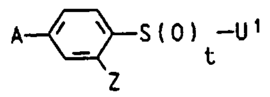 (III),

in der

A Nitro oder Amino,

$U^1$ $C_1$-$C_4$-Alkyl, Phenyl, $C_2$-$C_{10}$-Alkenyl oder den Rest

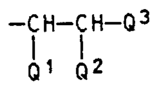 ,

wobei $Q^1$ und $Q^2$ gleich oder verschieden sind und unabhängig voneinander jeweils für Wasserstoff oder $C_1$-$C_4$-Alkyl und $Q^3$ für Hydroxy oder eine unter alkalischen Reaktionsbedingungen abspaltbare Gruppe stehen,

Z den Rest

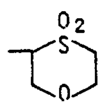

oder

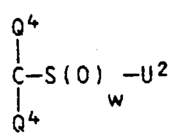 ,

wobei

$Q^4$ unabhängig voneinander für Wasserstoff oder $C_1$-$C_4$-Alkyl, das gegebenenfalls durch Cyano substituiert ist, $U^2$ für $C_1$-$C_4$-Alkyl, Phenyl, $C_2$-$C_{10}$-Alkenyl oder den Rest

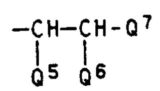 ,

in dem $Q^5$ und $Q^6$ gleich oder verschieden sind und unabhängig voneinander jeweils die Bedeutung von Wasserstoff oder $C_1$-$C_4$-Alkyl und $Q^7$ die Bedeutung von Hydroxy oder einer unter alkalischen Reaktionsbedingungen abspaltbaren Gruppe besitzen und w für 0, 1 oder 2 stehen, und

t 0, 1 oder 2 bedeuten,

mit der Maßgabe, daß mindestens einer der beiden Reste $S(O)_t$-$U^1$ und Z eine faserreaktive Gruppe darstellt.

**Claims**

**1.** A double attachment reactive dye of the formula I

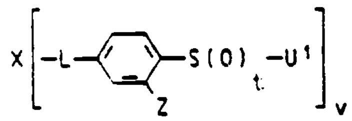

(I),

where

U¹ is $C_1$-$C_4$-alkyl, phenyl, $C_2$-$C_{10}$-alkenyl or the radical

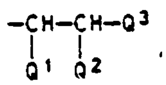

where $Q^1$ and $Q^2$ are identical or different and each is independently of the other hydrogen or $C_1$-$C_4$-alkyl and $Q^3$ is a group which is detachable under alkaline reaction conditions,

Z is the radical

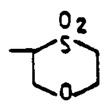

or

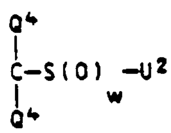

,

where each $Q^4$ is independently of the other hydrogen or $C_1$-$C_4$-alkyl which may be substituted by cyano, $U^2$ is $C_1$-$C_4$-alkyl, phenyl, $C_2$-$C_{10}$-alkenyl or the radical

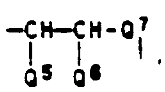

where $Q^5$ and $Q^6$ are identical or different and each is independently of the other hydrogen or $C_1$-$C_4$-alkyl and $Q^7$ is a group which is detachable under alkaline reaction conditions, and w is 0, 1 or 2,

t is 0, 1 or 2,

v is 1 or 2,

X is a) the radical of a chromophore which may have a further reactive group and which is derived from a mono- or disazo dye, a triphendioxazine, an anthraquinone, a copper formazan or a metallized phthalocyanine, or

b) the radical of a coupling component which may additionally be linked to the radical of a diazo component via an azo bridge and which may additionally have a reactive group, and

L is a) a bridge member of the formula

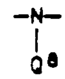

or

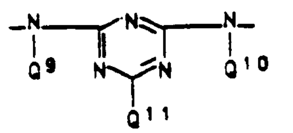,

where $Q^8$ is hydrogen or $C_1$-$C_4$-alkyl, $Q^9$ and $Q^{10}$ are identical or different and each is independently of the other hydrogen or $C_1$-$C_4$-alkyl and $Q^{11}$ is fluorine, chlorine or bromine, or

b) an azo bridge,

with the proviso that at least one of the two radicals $S(O)_t$-$U^1$ and Z is a fiber-reactive group.

**2.** A double attachment reactive dye as claimed in claim 1 of the formula Ia

$X[-L-E^1]_v$ (Ia),

where X, L and v are each as defined in claim 1 and $E^1$ is a radical of the formula IIa

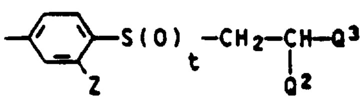

(IIa),

where

$Q^2$ is hydrogen or methyl,

$Q^3$ is $OSO_3H$,

Z is the radical

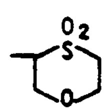

or

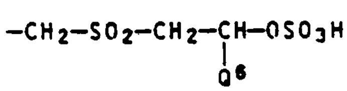

,

where $Q^6$ is hydrogen or methyl, and

t is 0 or 2.

**3.** A benzyl compound of the formula III

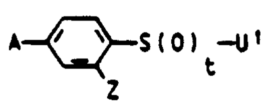

(III),

where

A is nitro or amino,

$U^1$ is $C_1$-$C_4$-alkyl, phenyl, $C_2$-$C_{10}$-alkenyl or the radical

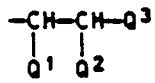,

where $Q^1$ and $Q^2$ are identical or different and each is independently of the other hydrogen or $C_1$-$C_4$-alkyl and $Q^3$ is hydroxyl or a group which is detachable under alkaline reaction conditions,

Z is the radical

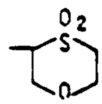

or

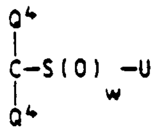,

where each $Q^4$ is independently of the other hydrogen or $C_1$-$C_4$-alkyl which may be substituted by cyano, $U^2$ is $C_1$-$C_4$-alkyl, phenyl, $C_2$-$C_{10}$-alkenyl or the radical

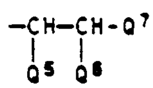,

where $Q^5$ and $Q^6$ are identical or different and each is independently of the other hydrogen or $C_1$-$C_4$-alkyl and $Q^7$ is hydroxyl or a group which is detachable under alkaline reaction conditions, and

w is 0, 1 or 2, and

t is 0, 1 or 2,

with the proviso that at least one of the two radicals $S(O)_t$-$U^1$ and Z is a fiber-reactive group.

## Revendications

**1.** Colorants à deux groupements réactifs de formule I

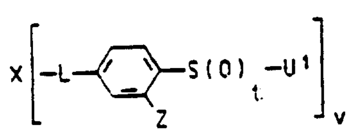

(I),

dans laquelle

$U^1$ est un groupement alkyle en $C_1$-$C_4$, phényle, alcényle en $C_2$-$c_{10}$ ou le reste

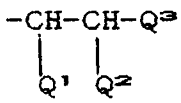

où $Q^1$ et $Q^2$ sont identiques ou différents et représentent indépendamment l'un de l'autre un atome d'hydrogène ou un groupement alkyle en $C_1$-$C_4$ et $Q^3$ est mis pour un groupement qui peut s'éliminer dans des conditions de réaction alcalines,

Z représente le reste

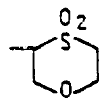

ou

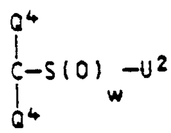

où les substituants $Q^4$ représentent indépendamment l'un de l'autre un atome d'hydrogène ou un groupement alkyle en $C_1$-$C_4$ qui est éventuellement substitué par un groupement cyano, $U^2$ représente un groupement alkyle en $C_1$-$C_4$, phényle, alcényle en $C_2$-$C_{10}$ ou le reste

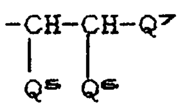

où $Q^5$ et $Q^6$ sont identiques ou différents et représentent indépendamment l'un de l'autre un atome d'hydrogène ou un groupement alkyle en $C_1$-$C_4$ et $Q^7$ représente un groupement pouvant être éliminé dans des conditions de réaction alcalines, et w est mis pour 0, 1 ou 2,

t est mis pour 0, 1 ou 2,

v est mis pour 1 ou 2,

X représente a) le reste d'un chromophore qui présente éventuellement un autre groupement réactif et qui provient d'un colorant monoazoïque ou disazoïque, d'une triphènedioxazine, d'une anthraquinone, d'un formazane-cuivre ou d'une phtalocyanine métallisée, ou

b) le reste d'un composant de copulation auquel est éventuellement également lié le reste d'un composant diazoïque par un pont azo et qui présente éventuellement un groupement réactif supplémentaire, et

L représente dans le cas a), un chaînon de pontage de formules

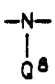

ou

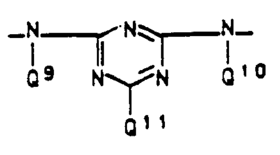

où $Q^8$ est mis pour un atome d'hydrogène ou un groupement alkyle en $C_1$-$C_4$, $Q^9$ et $Q^{10}$ sont identiques ou différents et représentent indépendamment l'un de l'autre un atome d'hydrogène ou un groupement alkyle en $C_1$-$C_4$ et $Q^{11}$ est mis pour le fluor, le chlore ou le brome, ou dans le cas b), un pont azo,

avec la condition qu'au moins l'un des deux restes $S(O)_t$-$U^1$ et Z représente un groupement réactif sur les fibres.

**2.** Colorants à deux groupements réactifs selon la revendication 1, qui correspondent à la formule Ia

$X[-L-E^1]_v$ (Ia)

dans laquelle X, L et v ont chacun les significations données dans la revendication 1 et $E^1$ est mis pour un reste de formule IIa

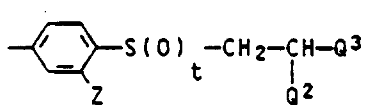 (IIa)

dans laquelle

- $Q^2$ est un atome d'hydrogène ou un groupement méthyle,
- $Q^3$ est un groupement $OSO_3H$,
- Z est le reste

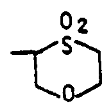

ou

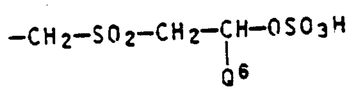 ,

où $Q^6$ est mis pour un atome d'hydrogène ou un groupement méthyle, et

- t est mis pour 0 ou 2.

**3.** Dérivés benzyliques de formule III

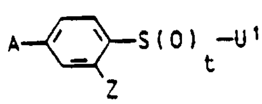 (III),

dans laquelle

- A est mis pour un groupement nitro ou amino,
- $U^1$ représente un groupement alkyle en $C_1$-$C_4$, phényle, alcényle en $C_2$-$C_{10}$ ou le reste

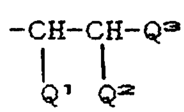

où $Q^1$ et $Q^2$ sont identiques ou différents et représentent indépendamment l'un de l'autre un atome d'hydrogène ou un groupement alkyle en $C_1$-$C_4$ et $Q^3$ est mis pour un groupement

hydroxy ou un groupement pouvant être éliminé dans des conditions de réaction alcalines,

Z représente le reste

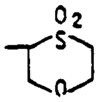

ou

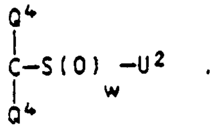

où les substituants $Q^4$ représentent indépendamment l'un de l'autre un atome d'hydrogène ou un groupement alkyle en $C_1$-$C_4$ qui est éventuellement substitué par un groupement cyano, $U^2$ représente un groupement alkyle en $C_1$-$C_4$, phényle, alcényle en $C_2$-$C_{10}$ ou le reste

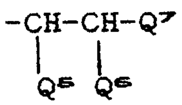

où $Q^5$ et $Q^6$ sont identiques ou différents et représentent indépendamment l'un de l'autre un atome d'hydrogène ou un groupement alkyle en $C_1$-$C_4$ et $Q^7$ représente un groupement hydroxy ou un groupement pouvant être éliminé dans des conditions de réaction alcalines et w est mis pour 0, 1 ou 2, et

t représente 0, 1 ou 2,

étant spécifié qu'au moins l'un des deux restes $S(O)_t$-$U^1$ et Z représente un groupement réactif vis-à-vis des fibres.